# EUROPEAN PATENT APPLICATION

(11) **EP 4 368 722 A1**
(43) Date of publication of application: **15.05.2024**
(21) Application number: 22837729.7
(22) Date of filing: 07.07.2022
(51) Int. Cl.: C12P 21/08, C12P 21/02, C07K 14/31, C07K 16/00, C07K 19/00, C12N 1/21, C12N 15/10, C12N 15/11, C12N 15/31, C12N 15/52, C12N 15/62, C12N 15/77

(54) **METHOD FOR SECRETORY PRODUCTION OF UNNATURAL-AMINO-ACID-CONTAINING PROTEIN**

(30) Priority: 07.07.2021 JP 2021112526
(71) Applicant: AJINOMOTO CO., INC., Chuo-ku Tokyo 104-8315 (JP)
(72) Inventor: HARUNA, Kenichi, Kawasaki-shi, Kanagawa 210-8681 (JP); SATO, Haruna, Kawasaki-shi, Kanagawa 210-8681 (JP); MATSUDA, Yoshihiko, Kawasaki-shi, Kanagawa 210-8681 (JP); ITO, Kenichiro, Kawasaki-shi, Kanagawa 210-8681 (JP)
(74) Representative: Hoffmann Eitle
(86) International application number: PCT/JP2022/026916
(87) International publication number: WO 2023/282315

(57) **Abstract**

A method for secretory production of a protein containing a noncanonical amino acid is provided. Secretory production of a noncanonical amino acid-containing protein is carried out by culturing a coryneform bacterium having a genetic construct for secretory expression of a protein containing a noncanonical amino acid, which is modified to express an orthogonal pair of tRNA corresponding to the noncanonical amino acid and aminoacyl tRNA synthetase.

## Description

### BACKGROUND

### Technical Field

The present invention relates to a method for secretory production of a noncanonical amino acid (ncAA)-containing protein.

### Brief Description of Related Art

For functional modification of proteins, technology has been developed to introduce noncanonical amino acids (ncAAs) as an alternative to the 20 types of natural amino acids, into the amino acid sequences of proteins. For example, technology to express a protein including ncAA in the amino acid sequence (ncAA-containing protein) utilizing an orthogonal pair of tRNA corresponding to ncAA and aminoacyl tRNA synthetase (aaRS) in a host such as Escherichia coli, *Scharomyces cereviciae,* or a mammalian cell has been reported (Non Patent Literature 1 to 3).

However, no technology is known for producing a ncAA-containing protein utilizing a coryneform bacterium.

### Citation List

### Non Patent Literature

[Non Patent Literature 1] Liu CC, Schultz PG. Adding new chemistries to the genetic code. Annu Rev Biochem. 2010; 79:413-44
[Non Patent Literature 2] Jason W Chin et. al., Addition of p-azido-L-phenylalanine to the genetic code of Escherichia coli, J Am Chem Soc. 2002 Aug 7;124(31):9026-7
[Non Patent Literature 3] Wei Wan et. al., Pyrrolysyl-tRNA synthetase: an ordinary enzyme but an outstanding genetic code expansion tool, Biochim Biophys Acta. 2014 Jun;1844(6):1059-70

### SUMMARY

### Technical Problem

An aspect of the present invention is to provide a method for secretory production of a noncanonical amino acid (ncAA)-containing protein.

### Solution to Problem

As a result of intensive studies to solve the problem described above, the present inventors found that secretory production of a ncAA-containing protein can be carried out by utilizing a coryneform bacterium that has been modified to express an orthogonal pair of tRNA corresponding to a noncanonical amino acid (ncAA) and aminoacyl tRNA synthetase (aaRS). This has led to the completion of the present invention.

In other words, the present invention can be exemplified as follows.
[1] A method for producing a protein containing a noncanonical amino acid, comprising:
   culturing a coryneform bacterium having a genetic construct for secretory expression of the protein containing a noncanonical amino acid in a medium containing the noncanonical amino acid; and
   collecting the protein containing a noncanonical amino acid produced by secretory production,
   wherein the coryneform bacterium is modified to express an orthogonal pair of a tRNA corresponding to the noncanonical amino acid and an aminoacyl tRNA synthetase.
[2] The method described above, wherein the genetic construct comprises, in the direction from 5' to 3', a promoter sequence that functions in a coryneform bacterium, a nucleic acid sequence encoding a signal peptide that functions in a coryneform bacterium, and a nucleic acid sequence encoding the protein containing a noncanonical amino acid, and
   wherein the protein containing a noncanonical amino acid is expressed as a fusion protein with the signal peptide.
[3] The method described above, wherein the noncanonical amino acid is encoded by a stop codon or a four-residue codon.
[4] The method described above, wherein the stop codon is UAG or UGA.
[5] The method described above, wherein the noncanonical amino acid is a tyrosine derivative or a lysine derivative.
[6] The method described above, wherein the noncanonical amino acid is selected from the group consisting of p-azido-L-phenylalanine, 3-azido-L-tyrosine, 3-chloro-L-tyrosine, 3-nitro-L-tyrosine, O-sulfo-L-tyrosine, L-pyrrolidine, and N_{δ}-alloc-L-lysine.
[7] The method described above, wherein the tRNA is tRNA(Tyr) or tRNA(Pyl).
[8] The method described above, wherein the tRNA(Tyr) is an RNA in (a), (b), or (c) described below:
   (a) RNA comprising the nucleotide sequence of SEQ ID NO: 42 or 44;
   (b) RNA comprising a nucleotide sequence with a modified anticodon in the nucleotide sequence of SEQ ID NO: 40, 42, or 44; or
   (c) RNA comprising a nucleotide sequence having an identity of 90% or higher to the nucleotide sequence of the RNA in (a) or (b), wherein the RNA has a function as a tRNA corresponding to the noncanonical amino acid.
[9] The method described above, wherein the tRNA(Pyl) is an RNA in (a), (b), or (c) described below:
   (a) RNA comprising the nucleotide sequence of SEQ ID NO: 46, 119, or 121;
   (b) RNA comprising a nucleotide sequence with a modified anticodon in the nucleotide sequence of SEQ ID NO: 46, 119, or 121; or
   (c) RNA comprising a nucleotide sequence having an identity of 90% or higher to the nucleotide sequence of the RNA in (a) or (b), wherein the RNA has a function as a tRNA corresponding to the noncanonical amino acid.
[10] The method described above, wherein the aminoacyl tRNA synthetase is tyrosyl tRNA synthetase or pyrrolidyl tRNA synthetase.
[11] The method described above, wherein the tyrosyl tRNA synthetase is a protein in (a), (b), (c) or (d) described below:
   (a) a protein comprising the amino acid sequence of SEQ ID NO: 50 or 52;
   (b) a protein comprising an amino acid sequence having a mutation that modifies the substrate specificity of the amino acid sequence of SEQ ID NO: 48, 50, or 52, wherein the protein has an aminoacyl tRNA synthetase activity for the noncanonical amino acid;
   (c) a protein comprising the amino acid sequence described in (a) or (b), but which includes substitution, deletion, insertion, and/or addition of 1 to 10 amino acid residues; or
   (d) a protein comprising an amino acid sequence having an identity of 90% or higher to the amino acid sequence of the protein described in (a) or (b).
[12] The method described above, wherein the mutation that modifies the substrate specificity includes a mutation in one or more amino acid residues selected from the group consisting of: Y32, H70, E107, D158, 1159, L162, and D286.
[13] The method described above, wherein the pyrrolidyl tRNA synthetase is a protein in (a), (b), (c), or (d) mentioned below:
   (a) a protein comprising the amino acid sequence of SEQ ID NO: 54 or 115;
   (b) a protein comprising an amino acid sequence having a mutation that modifies the substrate specificity of the amino acid sequence of SEQ ID NO: 54 or 115, wherein the protein has an aminoacyl tRNA synthetase activity for the noncanonical amino acid;
   (c) a protein comprising the amino acid sequence described in (a) or (b), but which includes substitution, deletion, insertion, and/or addition of 1 to 10 amino acid residues, wherein the protein has an aminoacyl tRNA synthetase activity for the noncanonical amino acid; or
   (d) a protein comprising an amino acid sequence having an identity of 90% or higher to the amino acid sequence of the protein described in (a) or (b), wherein the protein has an aminoacyl tRNA synthetase activity for the noncanonical amino acid.
[14] The method described above, wherein the mutation that modifies the substrate specificity includes a mutation in one or more amino acid residues selected from the group consisting of: M241, L266, A267, L270, Y271, L274, N311, C313, M315, Y349, V367, and W383.
[15] The method described above, wherein the coryneform bacterium has been further modified so as to harbor a phoS gene encoding a mutant PhoS protein.
[16] The method described above, wherein the mutation is a mutation of replacing an amino acid residue corresponding to the tryptophan residue at position 302 in SEQ ID NO: 2 with an amino acid residue other than aromatic amino acid and histidine residues.
[17] The method described above, wherein the amino acid residue other than aromatic amino acid and a histidine residue is selected from the group consisting of a lysine residue, alanine residue, valine residue, serine residue, cysteine residue, methionine residue, aspartic acid residue, and asparagine residue in the wildtype PhoS protein.
[18] The method described above, wherein the wildtype PhoS protein is a protein in (a), (b), or (c) described below:
   (a) a protein comprising any of the amino acid sequences of SEQ ID NOS: 2 to 7;
   (b) a protein comprising any of the amino acid sequences of SEQ ID NOS: 2 to 7, but which includes substitution, deletion, insertion, and/or addition of 1 to 10 amino acid residues, wherein the protein has a function as a sensor kinase of a PhoRS system; or
   (c) a protein comprising an amino acid sequence having an identity of 90% or higher to any of the amino acid sequences of SEQ ID NOS: 2 to 7, wherein the protein has a function as a sensor kinase of a PhoRS system.

[19] The method described above, wherein the signal peptide is a Tat-dependent signal peptide.

[20] The method described above, wherein the Tat-dependent signal peptide is selected from the group consisting of TorA signal peptide, SufI signal peptide, PhoD signal peptide, LipA signal peptide, and IMD signal peptide.

[21] The method described above, wherein the coryneform bacterium has been further modified so that the expression of one or more genes encoding a Tat secretion system is increased as compared with a non-modified strain.

[22] The method described above, wherein the genes encoding a Tat secretion system consists of a tatA gene, tatB gene, tatC gene, and tatE gene.

[23] The method described above, wherein the signal peptide is a Sec-dependent signal peptide.

[24] The method described above, wherein the Sec-dependent signal peptide is selected from the group consisting of PS1 signal peptide, PS2 signal peptide, and SlpA signal peptide.

[25] The method described above, wherein the genetic construct further comprises a nucleic acid sequence encoding an amino acid sequence including Gln-Glu-Thr between the nucleic acid sequence encoding a signal peptide that functions in a coryneform bacterium and the nucleic acid sequence encoding the protein containing a noncanonical amino acid.

[26] The method described above, wherein the genetic construct further comprises a nucleic acid sequence encoding an amino acid sequence used for enzymatic cleavage between the nucleic acid sequence encoding an amino acid sequence including Gln-Glu-Thr and the nucleic acid sequence encoding the protein containing a noncanonical amino acid.

[27] The method described above, wherein the coryneform bacterium belongs to the genus *Corynebacterium.*

[28] The method described above, wherein the coryneform bacterium is *Corynebacterium glutamicum.*

[29] The method described above, wherein the coryneform bacterium is a modified strain derived from *Corynebacterium glutamicum* AJ12036 (FERM BP-734) or *Corynebacterium glutamicum* ATCC 13869.

[30] The method described above, wherein the coryneform bacterium is a coryneform bacterium with the number of molecules of a cell surface layer protein per cell reduced as compared with a non-modified coryneform bacterium.

[31] The method described above, wherein the coryneform bacterium has a first expression vector carrying the genetic construct and a second expression vector carrying a gene encoding the tRNA and a gene encoding the aminoacyl tRNA synthetase.

[32] The method described above, wherein the first expression vector further carries a gene encoding the tRNA and/or a gene encoding the aminoacyl tRNA synthetase.

[33] The method described above, wherein the first expression vector is a pPK vector and the second expression vector is a pVC vector.

[34] The method described above, wherein the first expression vector is pPK4 or pPK5 and the second expression vector is pVC7 or pVC7N.

[35] The method described above, wherein the coryneform bacterium has a single expression vector carrying the genetic construct, a gene encoding the tRNA, and a gene encoding the aminoacyl tRNA synthetase.

[36] The method described above, wherein the expression vector is a pPK vector.

[37] The method described above, wherein the expression vector is pPK4 or pPK5.

[38] The method described above, wherein the noncanonical amino acid-containing protein is an antibody-related molecule, an antibody mimetic, or a physiologically active protein.

[39] The method described above, wherein the noncanonical amino acid-containing protein is a VHH fragment, a Z domain of protein A, a fluorescent protein, or a growth factor.

### Brief Description of Drawings

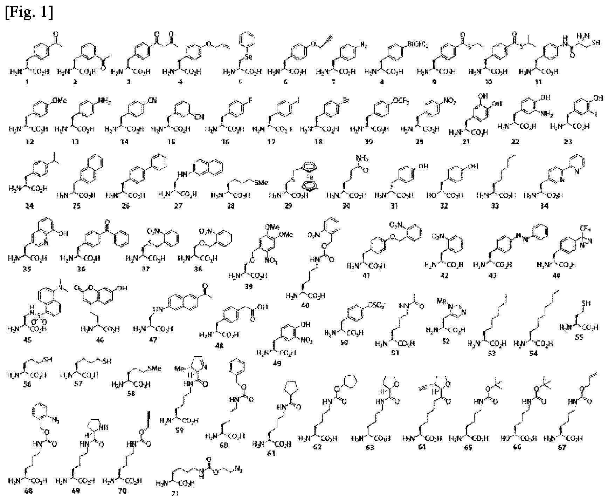

FIG. 1 is a diagram showing an example of ncAA.

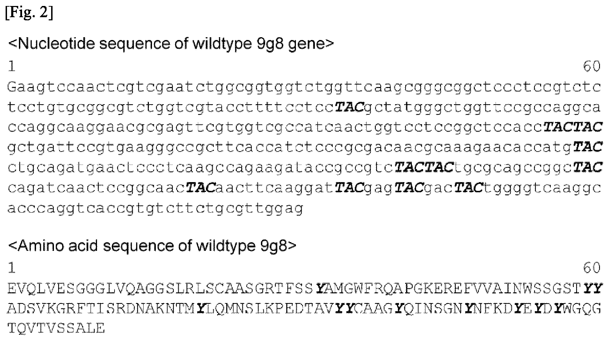

FIG. 2 is a diagram showing the nucleotide sequence of a gene encoding the anti-epidermal-growth-factor receptor (epidermal-growth-factor receptor: EGFR) VHH antibody 9g8 (SEQ ID NO: 57) and the amino acid sequence of 9g8 (SEQ ID NO: 58). The ncAA introduction site and its corresponding triplet are shown in bold italics.

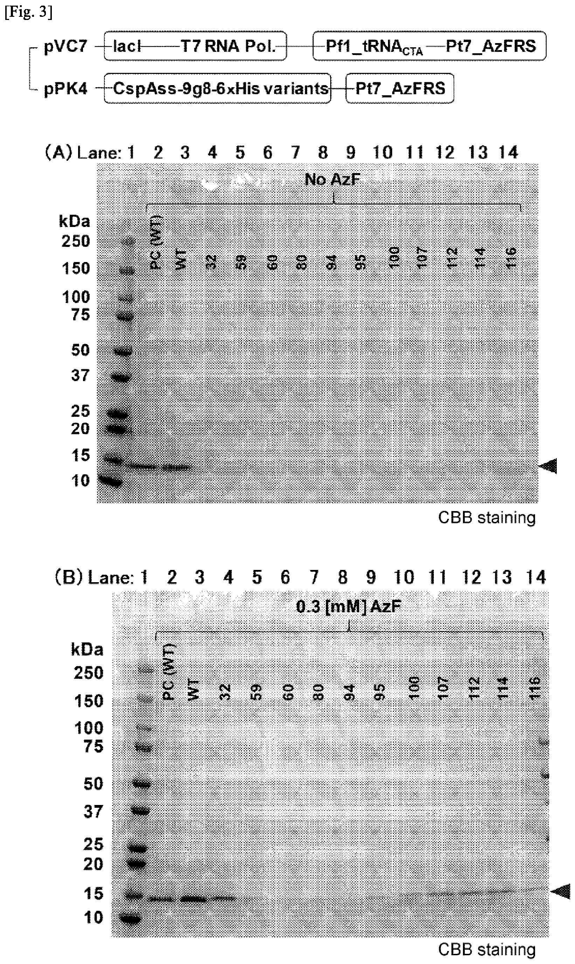

FIG. 3 is a diagram showing the structure of the expression construct of AzF-introduced 9g8 mutant using C. *glutamicum* as an expression host and SDS PAGE results of the culture supernatant (photographs). Panel (A) shows the results obtained by culturing in the absence of AzF, and panel (B) shows the results obtained by culturing in the presence of 0.3 mM AzF. Lane 1, molecular weight marker; Lane 2, control strain PC (strain introduced with the wildtype (WT) 9g8 vector); Lane 3, control strain WT (strain introduced with the AzFN3 vector and the wildtype 9g8+AzFRS vector); Lanes 4-14, mutant 9g8-expressing strain (the numbers (32 to 116) indicate modified sites). Each arrow indicates the location corresponding to the full length of 9g8.

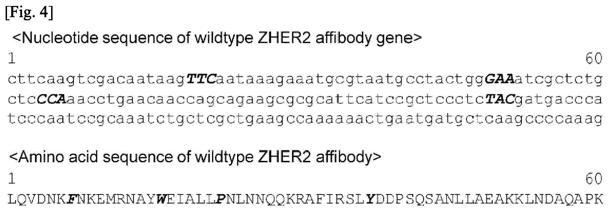

FIG. 4 is a diagram showing the nucleotide sequence of a gene encoding the anti-epidermal-growth-factor receptor (human epidermal-growth-factor receptor 2: HER2) antibody ZHER2 affibody (SEQ ID NO: 83) and the amino acid sequence of the ZHER2 affibody (SEQ ID NO: 84). The ncAA introduction site and its corresponding triplet are shown in bold italics.

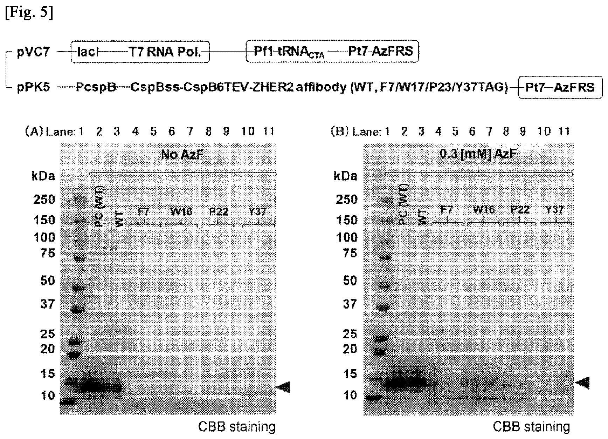

FIG. 5 is a diagram showing the structure of the expression construct of AzF-introduced ZHER2 affibody mutant using *C*. *glutamicum* as an expression host and SDS PAGE results of the culture supernatant (photographs). Panel (A) shows the results obtained by culturing in the absence of AzF, and panel (B) shows the results obtained by culturing in the presence of 0.3 mM AzF. Lane 1, molecular weight marker; Lane 2, control strain PC (strain introduced with the wildtype ZHER2 affibody vector); Lane 3, control strain WT (strain introduced with the AzFN3 vector and the wildtype ZHER2 affibody+AzFRS vector); Lanes 4-11, mutant ZHER2 affibody-expressing strain (F7, W16, P22, and Y37 indicate modified sites). Each arrow indicates the location corresponding to the full length of the ZHER2 affibody.

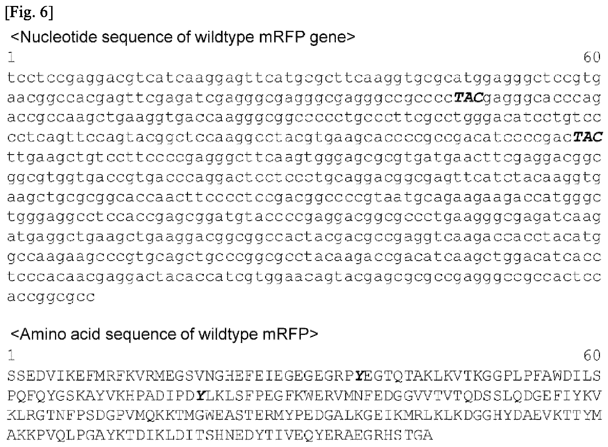

FIG. 6 is a diagram showing the nucleotide sequence of a gene encoding the monomeric red fluorescent protein (mRFP) (SEQ ID NO: 94) and the amino acid sequence of mRFP (SEQ ID NO: 95). The ncAA introduction site and its corresponding triplet are shown in bold italics.

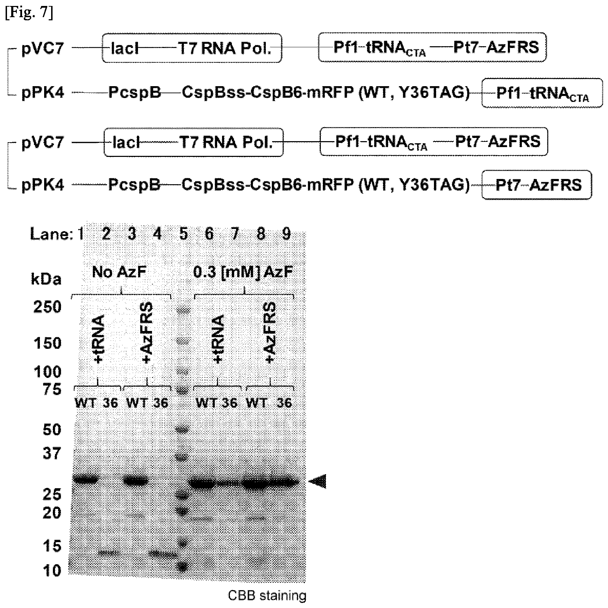

FIG. 7 is a diagram showing the structure of the expression construct of AzF-introduced mRFP mutant using *C*. *glutamicum* as an expression host and SDS PAGE results of the culture supernatant (photograph). "No AzF" indicates the results obtained by culturing in the absence of AzF, and "0.3 mM AzF" indicates the results obtained by culturing in the presence of 0.3 mM AzF. Lanes 1 and 6, control strain WT+tRNA (strain introduced with the AzFN3 vector and the wildtype mRFP+tRNA_{CTA} vector); Lanes 2 and 7, strain introduced with the AzFN3 vector and the mutant mRFP+tRNA_{CTA} vector; Lanes 3 and 8, control strain WT+AzFRS (strain introduced with the AzFN3 vector and the wildtype mRFP+AzFRS vector); Lanes 4 and 9, strain introduced with the AzFN3 vector and the mutant mRFP+AzFRS vector; Lane 5, molecular weight marker. The arrow indicates the location corresponding to the full length of mRFP.

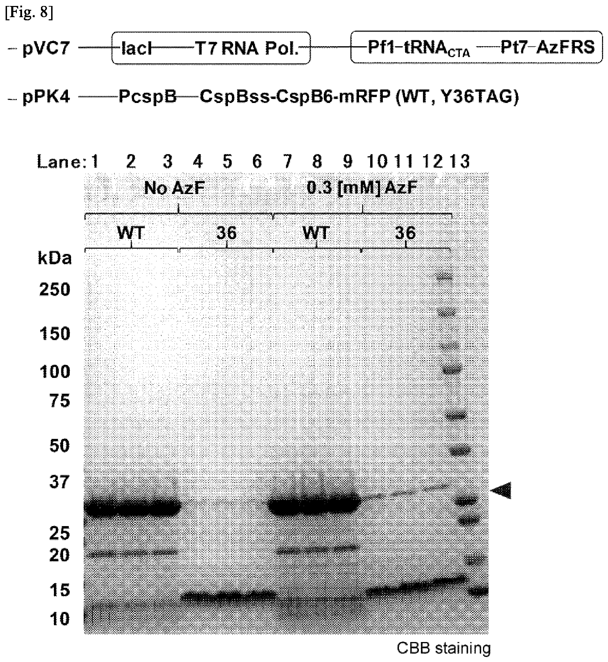

FIG. 8 is a diagram showing the structure of the expression construct of AzF-introduced mRFP mutant using *C*. *glutamicum* as an expression host and SDS PAGE results of the culture supernatant (photograph). "No AzF" indicates the results obtained by culturing in the absence of AzF, and "0.3 mM AzF" indicates the results obtained by culturing in the presence of 0.3 mM AzF. Lanes 1-3 and 7-9, control strain WT (strain introduced with the AzFN3 vector and the wildtype mRFP vector); Lanes 4-6 and 10-12, mutant mRFP-expressing strain; Lane 13, molecular weight marker. The arrow indicates the location corresponding to the full length of mRFP.

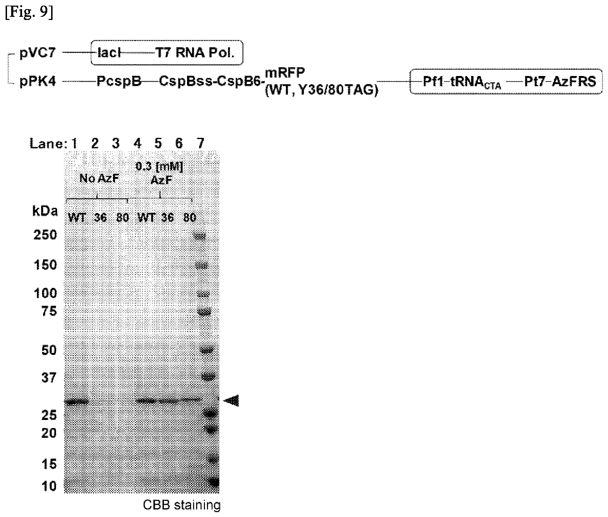

FIG. 9 is a diagram showing the structure of the expression construct of AzF-introduced mRFP mutant using *C*. *glutamicum* as an expression host and SDS PAGE results of the culture supernatant (photograph). "No AzF" indicates the results obtained by culturing in the absence of AzF, and "0.3 mM AzF" indicates the results obtained by culturing in the presence of 0.3 mM AzF. Lanes 1 and 4, control strain WT (strain introduced with the pVC7T7pol 1 vector and the wildtype mRFP+AzFN3 vector); Lanes 2-3 and 5-6, mutant mRFP-expressing strain (the numbers (36 and 80) indicate modified sites); Lane 7, molecular weight marker. The arrow indicates the location corresponding to the full length of mRFP.

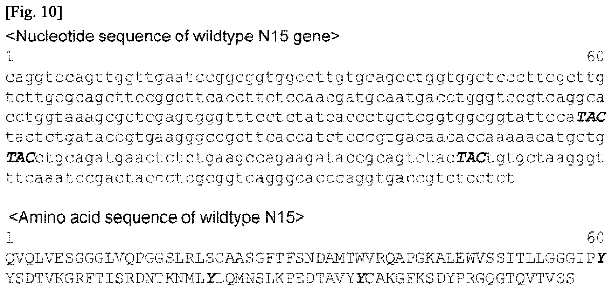

FIG. 10 is a diagram showing the nucleotide sequence of a gene encoding the anti-Izumo protein 1 N-terminal extracellular domain (NDOM) VHH antibody N15 (SEQ ID NO: 103) and the amino acid sequence of N15 (SEQ ID NO: 104). The ncAA introduction site and its corresponding triplet are shown in bold italics.

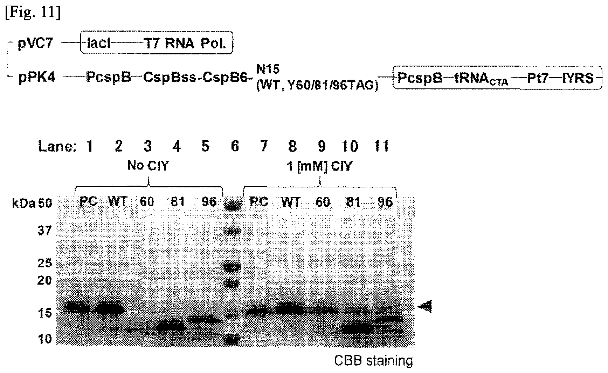

FIG. 11 is a diagram showing the structure of the expression construct of ClY-introduced N15 mutant using *C*. *glutamicum* as an expression host and SDS PAGE results of the culture supernatant (photograph). "No ClY" indicates the results obtained by culturing in the absence of ClY, and "1 mM ClY" indicates the results obtained by culturing in the presence of 1 mM ClY. Lanes 1 and 7, control strain PC (strain introduced with the wildtype N15 vector); Lanes 2 and 8, control strain WT (strain introduced with the pVC7T7pol1 vector and the wildtype N15+IYN3 vector); Lanes 3-5 and 9-11, mutant N15-expressing strain (the numbers (60, 81 and 96) indicate modified sites); Lane 6, molecular weight marker. The arrow indicates the location corresponding to the full length of N15.

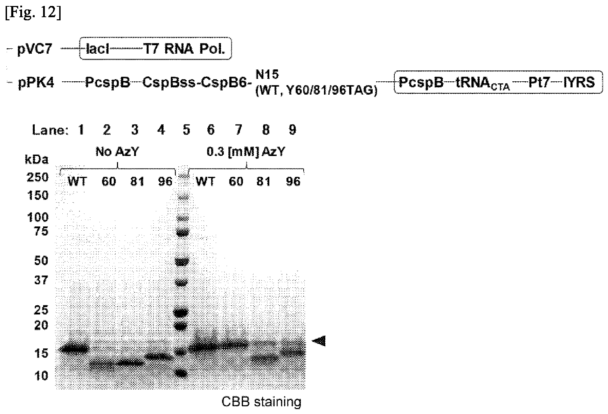

FIG. 12 is a diagram showing the structure of the expression construct of AzY-introduced N15 mutant using *C*. *glutamicum* as an expression host and SDS PAGE results of the culture supernatant (photograph). "No AzY" indicates the results obtained by culturing in the absence of AzY, and "0.3 mM AzY" indicates the results obtained by culturing in the presence of 0.3 mM AzY. Lanes 1 and 7, control strain WT (strain introduced with the pVC7T7pol1 vector and the wildtype N15+IYN3 vector); Lanes 2-4 and 7-9, mutant N15-expressing strain (the numbers (60, 81 and 96) indicate modified sites); Lane 5, molecular weight marker. The arrow indicates the location corresponding to the full length of N15.

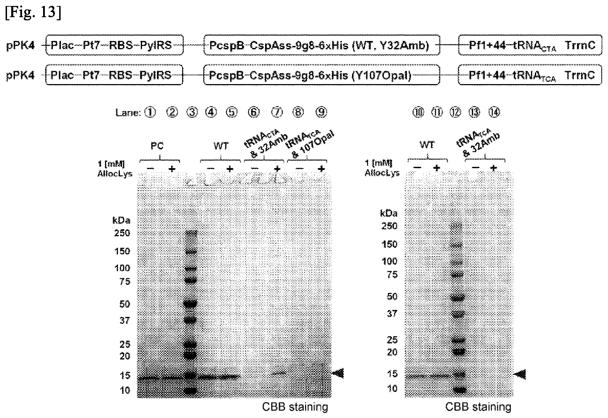

FIG. 13 is a diagram showing the structure of the expression construct of AllocLys-introduced 9g8 mutant using *C*. *glutamicum* as an expression host and SDS PAGE results of the culture supernatant (photographs). "-" indicates the results obtained by culturing in the absence of AllocLys, and "+" indicates the results obtained by culturing in the presence of 1 mM AllocLys. Lanes 1 and 2, control strain PC (strain introduced with the wildtype 9g8 vector); Lanes 3 and 12, molecular weight marker; Lane 4, 5, 10, and 11, control strain WT (strain introduced with the PylRS+WT 9g8+tRNA(Pyl)CTA vector); Lanes 6 and 7, mutant 9g8-expressing strain (combination of 9g8 (Y32TAG) and tRNA_{CTA}); Lanes 8 and 9, mutant 9g8-expressing strain (combination of 9g8 (Y107TGA) and tRNA_{TCA}); Lanes 13 and 14, mutant 9g8-expressing strain (combination of 9g8 (Y32TAG) and tRNA_{TCA}). Each arrow indicates the location corresponding to the full length of 9g8.

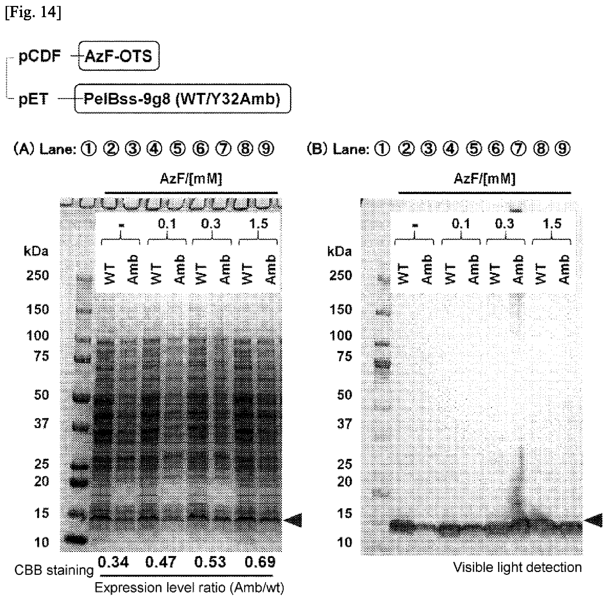

FIG. 14 is a diagram showing the structure of the expression construct of AzF-introduced 9g8 mutant using *E. coli* as an expression host and the results of SDS PAGE and Western blotting of the culture supernatant (photographs). Panel (A) shows the results of SDS PAGE, and panel (B) shows the results of Western blotting. Lane 1, molecular weight marker; Lanes 2, 4, 6, and 8, control strain WT (strain introduced with the AzFN3 vector for *E. coli* and the wildtype 9g8 vector for *E*. *coli*); Lanes 3, 5, 7, and 9, mutant 9g8-expressing strain (strain introduced with the AzFN3 vector for *E. coli* and the mutant 9g8 vector for *E. coli*). Each arrow indicates the location corresponding to the full length of 9g8.

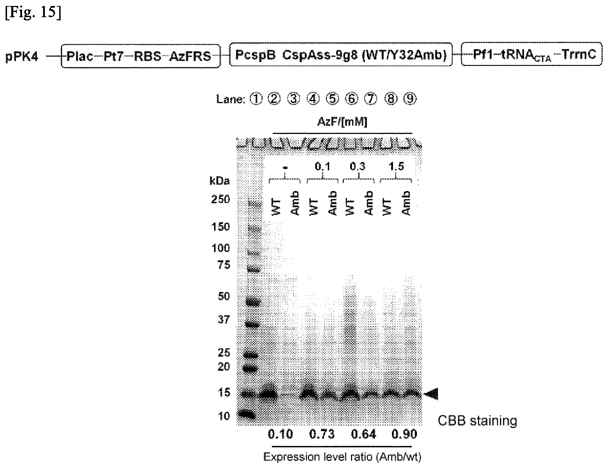

FIG. 15 is a diagram showing the structure of the expression construct of AzF-introduced 9g8 mutant using *C*. *glutamicum* as an expression host and SDS PAGE results of the culture supernatant (photographs). Lane 1, molecular weight marker; Lanes 2, 4, 6, and 8, control strain WT (strain introduced with the AzFRS+WT 9g8+tRNA_{CTA} vector); Lanes 3, 5, 7, and 9, mutant 9g8-expressing strain (strain introduced with the AzFRS+mutant 9g8+tRNA_{CTA} vector). Each arrow indicates the position corresponding to the total length of 9g8.

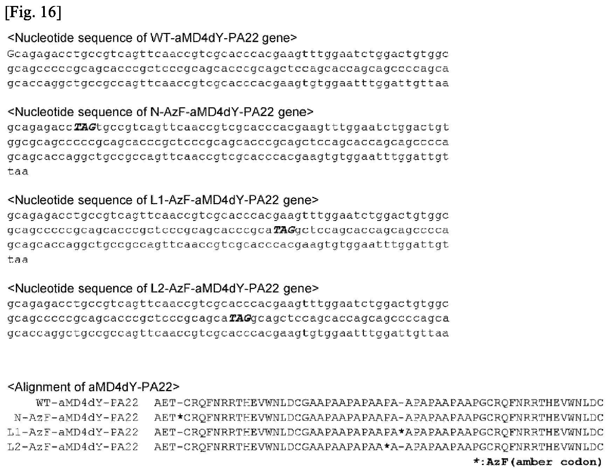

FIG. 16 is a diagram showing the nucleotide sequences of the wildtype and mutant aMD4dY-PA22 genes and the alignment of wildtype aMD4dY-PA22 and mutant aMD4dY-PA22. The triplet encoding AzF is shown in bold italics.

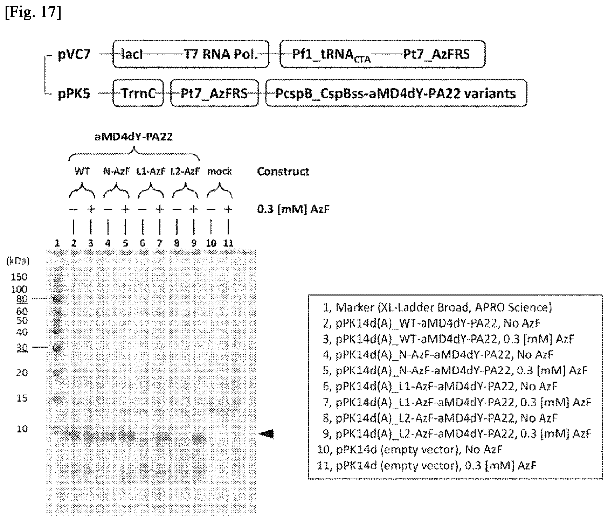

FIG. 17 is a diagram showing the structure of the expression construct of AzF-introduced aMD4dY-PA22 mutant using *C*. *glutamicum* as an expression host and SDS PAGE results of the culture supernatant (photograph). The arrow indicates the location corresponding to the full length of aMD4dY-PA22.

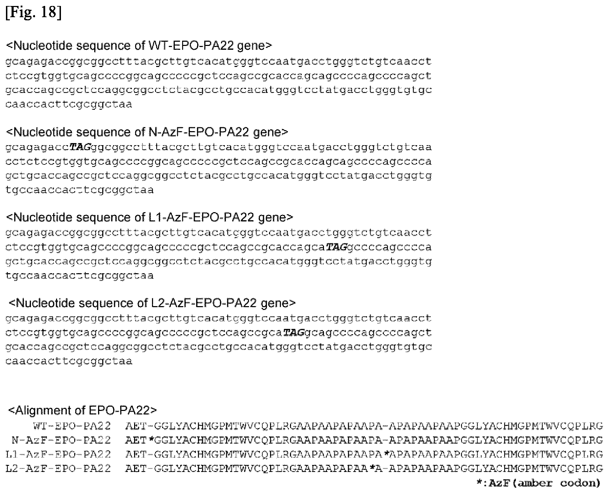

FIG. 18 is a diagram showing the nucleotide sequences of the wildtype and mutant EPO-PA22 genes and the alignment of wildtype EPO-PA22 and mutant EPO-PA22. The triplet encoding AzF is shown in bold italics.

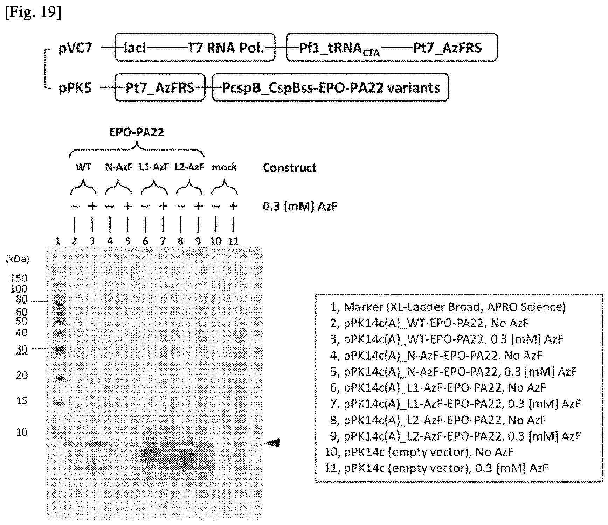

FIG. 19 is a diagram showing the structure of the expression construct of AzF-introduced EPO-PA22 mutant using *C*. *glutamicum* as an expression host and SDS PAGE results of the culture supernatant (photographs). Each arrow indicates the location corresponding to the full length of EPO-PA22.

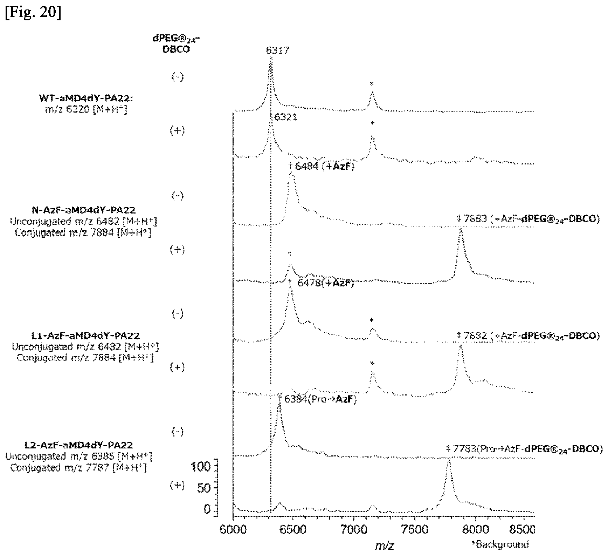

FIG. 20 is a diagram showing the results of PEG modification of wildtype aMD4dY-PA22 and mutant aMD4dY-PA22 by a strain-promoted alkyne azide cycloaddition (SPAAC) reaction.

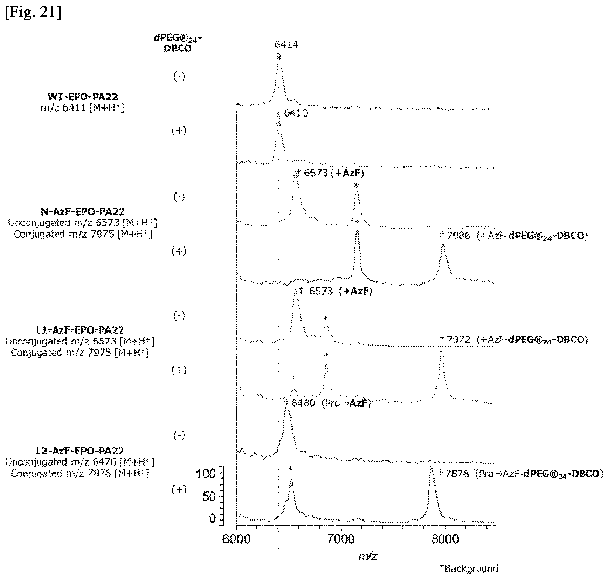

FIG. 21 is a diagram showing the results of PEG modification of wildtype EPO-PA22 and mutant EPO-PA22 by a strain-promoted alkyne azide cycloaddition (SPAAC) reaction.

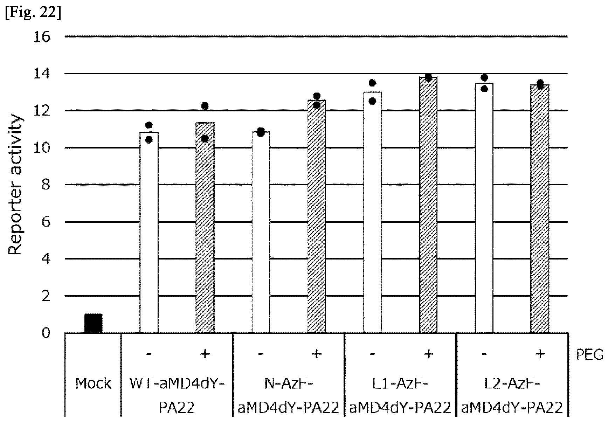

FIG. 22 is a diagram showing the results of reporter assay of wildtype aMD4dY-PA22 and mutant aMD4dY-PA22 modified with or without PEG.

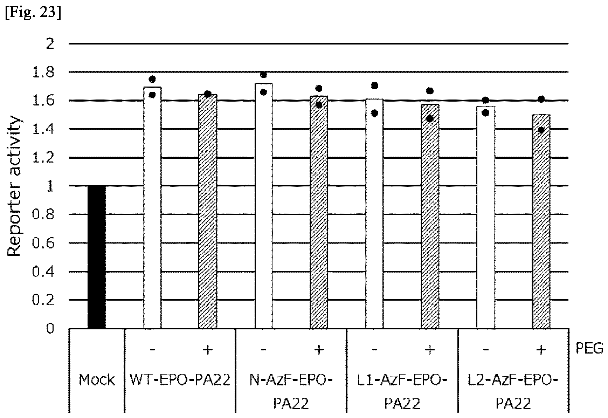

FIG. 23 is a diagram showing the results of reporter assay of wildtype EPO-PA22 and mutant EPO-PA22 modified with or without PEG.

### Description of Embodiments

Hereinafter, the present invention will be explained in detail.

The method of the present invention is a method for secretory production of a noncanonical amino acid (ncAA)-containing protein using a coryneform bacterium.

Specifically, the method of the present invention may be a method for producing a protein containing a noncanonical amino acid, comprising:
culturing a coryneform bacterium having a genetic construct for secretory expression of the protein containing a noncanonical amino acid; and
collecting the protein containing a noncanonical amino acid produced by secretory production,
wherein the coryneform bacterium is modified to express an orthogonal pair of a tRNA corresponding to the noncanonical amino acid and an aminoacyl tRNA synthetase.

The coryneform bacterium (i.e., the coryneform bacterium used for the method of the present invention) is also referred to as "bacterium as described herein" or "coryneform bacterium as described herein". Furthermore, the bacterium as described herein or a parent strain used for constructing the same is also referred to as "host".

The aforementioned genetic construct (i.e., the genetic construct for secretory expression of a ncAA-containing protein) is also referred to as "genetic construct for secretory expression".

The aforementioned pair (i.e., the orthogonal pair of a tRNA corresponding to ncAA and an aminoacyl tRNA synthetase (aaRS)) is also referred to as "orthogonal tRNA (ncAA)/ncAA-aaRS pair".

### <1> Coryneform Bacterium of the Invention

The coryneform bacterium as described herein is a coryneform bacterium having a genetic construct for secretory expression of a ncAA-containing protein (i.e., genetic construct for secretory expression), which has been modified to express an orthogonal pair of tRNA corresponding to ncAA and aaRS (i.e., orthogonal tRNA (ncAA)/ncAA-aaRS pair).

### <1-1> Coryneform Bacterium Having Ability of Secretory Production of ncAA-containing Protein

The coryneform bacterium as described herein has an ability of secretory production of a ncAA-containing protein. The coryneform bacterium as described herein has an ability of secretory production of a ncAA-containing protein, relying at least on a combination of having the genetic construct for secretory expression and expressing the orthogonal tRNA (ncAA)/ncAA-aaRS pair. Specifically, the coryneform bacterium may have an ability of secretory production of a ncAA-containing protein, relying on a combination of having the genetic construct for secretory expression and expressing the orthogonal tRNA (ncAA)/ncAA-aaRS pair, or relying on a combination of having the genetic construct for secretory expression, expressing the orthogonal tRNA (ncAA)/ncAA-aaRS pair, and other characteri stic(s).

The expression that a protein is "secreted" means that the protein is transported out of a bacterial cell (extracellularly transported). Examples of a position outside of a bacterial cell (outside of a cell) include a medium and a cell surface layer. That is, molecules of the secreted protein may be present, for example, in the medium, in the cell surface layer, or in both of the medium and the cell surface layer. That is, the expression that a protein is "secreted" is not limited when all the molecules of the protein eventually are present in the medium in completely free forms, but also includes, for example, when all the molecules of the protein are present in the cell surface layer, and also when some of the molecules of the protein are present in the medium and the remaining molecules of the protein are present in the cell surface layer.

That is, the phrase "ability to produce a ncAA-containing protein by secretory production" refers to an ability of the bacterium as described herein to secrete the ncAA-containing protein into a medium and/or a cell surface layer, and accumulate it there to such an extent that the ncAA-containing can be collected from the medium and/or the cell surface layer, when the bacterium is cultured in the medium. The accumulation amount may be, for example, in terms of the accumulation amount in the medium, 10 µg/L or more, 1 mg/L or more, 100 mg/L or more, or 1 g/L or more. Also, the accumulation amount may be, for example, in terms of the accumulation amount in the cell surface layer, such an amount that if the ncAA-containing protein in the cell surface layer is collected and suspended in a liquid of the same volume as the medium, the concentration of the ncAA-containing protein in the suspension is 10 µg/L or more, 1 mg/L or more, or 100 mg/L or more.

Coryneform bacteria are aerobic gram-positive bacilli. Examples of the coryneform bacteria include *Corynebacterium* bacteria, *Brevibacterium* bacteria, *Microbacterium* bacteria, and so forth. Advantages of use of the coryneform bacteria include that they inherently secrete an extremely small amount of proteins out of cells compared with fungi, yeasts, *Bacillus* bacteria, etc., which are conventionally used for secretory production of proteins, and therefore the purification process of a protein produced by secretory production is expected to be simplified or eliminated, that they can grow well in a simple medium containing a saccharide, ammonia, mineral salts, and so forth, and therefore they are excellent in view of cost of medium, culture method, and culture productivity, and so forth.

Specific examples of coryneform bacteria include the following species:
*Corynebacterium acetoacidophilum*
*Corynebacterium acetoglutamicum*
*Corynebacterium alkanolyticum*
*Corynebacterium callunae*
*Corynebacterium crenatum*
*Corynebacterium glutamicum*
*Corynebacterium lilium*
*Corynebacterium melassecola*
*Corynebacterium thermoaminogenes* (*Corynebacterium efficiens*)
*Corynebacterium herculis*
*Brevibacterium divaricatum (Corynebacterium glutamicum)*
*Brevibacterium flavum (Corynebacterium glutamicum)*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Corynebacterium ammoniagenes (Corynebacterium stationis)*
*Brevibacterium album*
*Brevibacterium cerinum*
*Microbacterium ammoniaphilum*

Specific examples of coryneform bacteria include the following strains:
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium crenatum* AS1.542
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium thermoaminogenes (Corynebacterium efficiens*) AJ12340 (FERM BP-1539)
*Corynebacterium herculis* ATCC 13868
*Brevibacterium divaricatum* (*Corynebacterium glutamicum*) ATCC 14020
*Brevibacterium flavum* (*Corynebacterium glutamicum*) ATCC 13826, ATCC 14067, AJ12418 (FERM BP-2205)
*Brevibacterium immariophilum* ATCC 14068
*Brevibacterium lactofermentum* (*Corynebacterium glutamicum*) ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes* (*Corynebacterium stationis*) ATCC 6871, ATCC 6872
*Brevibacterium album* ATCC 15111
*Brevibacterium cerinum* ATCC 15112
*Microbacterium ammoniaphilum* ATCC 15354

The *Corynebacterium* bacteria include bacteria that had previously been classified into the genus *Brevibacterium* but have been united into the genus *Corynebacterium* (Int. J. Syst. Bacteriol., 41, 255 (1991)). Moreover, *Corynebacterium stationis* includes bacteria that had previously been classified as *Corynebacterium ammoniagenes,* but are presently re-classified into *Corynebacterium stationis* on the basis of nucleotide sequence analysis of 16S rRNA, etc. (Int. J. Syst. Evol. Microbiol., 60, 874-879 (2010)).

These strains are available from, for example, the American Type Culture Collection (Address: 10801 University Boulevard, Manassas, Va. 20110, United States of America). That is, registration numbers are assigned to the respective strains, and the strains can be ordered by using these registration numbers (refer to atcc.org). The registration numbers of the strains are listed in the catalogue of the American Type Culture Collection. These strains can also be obtained from, for example, the depositories at which the strains were deposited.

In particular, the *Corynebacterium glutamicum* (*C. glutamicum*) AJ12036 strain (FERM BP-734), which was isolated as a streptomycin (Sm) resistant mutant strain from a wildtype strain *C*. *glutamicum* ATCC 13869 is predicted to have a mutation in a gene responsible for a function involved in secretion of proteins, and shows an extremely high secretory production ability for proteins as high as about 2 to 3 times in terms of accumulation amount of proteins under optimum culture conditions, compared with the parent strain (wildtype strain), and therefore it is preferred as a host bacterium (WO2002/081694). The AJ12036 strain was originally deposited at the Fermentation Research Institute, Agency of Industrial Science and Technology (currently, independent administrative agency, National Institute of Technology and Evaluation, International Patent Organism Depositary, #120, 2- 5- 8 Kazusakamatari, Kisarazu-shi, Chiba-ken, 292- 0818, Japan) on Mar. 26, 1984 as an international deposit, and assigned an accession number of FERM BP-734.

*Corynebacterium thermoaminogenes* AJ12340 (FERM BP-1539) was originally deposited at the Fermentation Research Institute, Agency of Industrial Science and Technology (currently, independent administrative agency, National Institute of Technology and Evaluation, International Patent Organism Depositary, #120, 2- 5- 8 Kazusakamatari, Kisarazu-shi, Chiba-ken, 292- 0818, Japan) on Mar. 13, 1987 as an international deposit, and assigned an accession number of FERM BP-1539. *Brevibacterium flavum* AJ12418 (FERM BP-2205) was originally deposited at the Fermentation Research Institute, Agency of Industrial Science and Technology (currently, independent administrative agency, National Institute of Technology and Evaluation, International Patent Organism Depositary, #120, 2- 5- 8 Kazusakamatari, Kisarazu-shi, Chiba-ken, 292- 0818, Japan) on Dec. 24, 1988 as an international deposit, and assigned an accession number of FERM BP-2205.

Moreover, a strain having an enhanced ability to produce a protein by secretory production may be a coryneform bacterium as mentioned above as a parent strain by using a mutagenesis method or a genetic recombination method, and used as a host. For example, after a parent strain is treated with ultraviolet irradiation or a chemical mutation agent such as N-methyl-N'-nitrosoguanidine, a strain having an enhanced ability to produce a protein by secretory production can be selected.

Furthermore, if a strain obtained by modifying such a strain as mentioned above so that it does not produce a cell surface layer protein is used as a host, purification of the ncAA-containing protein secreted in the medium or on the cell surface layer becomes easy, and therefore it is particularly preferred. Such modification can be carried out by introducing a mutation into the coding region of the cell surface layer protein or an expression control region thereof, on the chromosome by mutagenesis or genetic recombination. Examples of coryneform bacterium modified so that it does not produce a cell surface layer protein include the *C. glutamicum* YDK010 strain (WO2002/081694), which is a cell surface layer protein PS2-deficient strain of the *C. glutamicum* AJ12036 strain (FERM BP-734).

The coryneform bacterium as described herein can be obtained by appropriately modifying a coryneform bacterium as mentioned above (e.g., introducing the genetic construct for secretory expression, a gene encoding the orthogonal tRNA (ncAA)/ncAA-aaRS pair, and (optionally) other modifications). That is, the bacterium may be, for example, a modified strain derived from such a coryneform bacterium as described above. The bacterium may be, specifically, for example, a modified strain derived from *C. glutamicum* AJ12036 (FERM BP-734) or a modified strain derived from *C. glutamicum* ATCC 13869. A modified strain derived from *C. glutamicum* AJ12036 (FERM BP-734) falls also within a modified strain derived from *C. glutamicum* ATCC 13869. Modifications for constructing the bacterium can be performed in any order.

### <1-2> Genetic Construct for Secretory Expression of ncAA-containing Protein and Introduction of the Same

The coryneform bacterium as described herein has the genetic construct for secretory expression of a ncAA-containing protein (i.e., genetic construct for secretory expression).

It is known that a secretory protein is generally translated as a preprotein (also referred to as prepeptide) or a preproprotein (also referred to as prepropeptide), and then becomes a mature protein through processing. Specifically, a secretory protein is generally translated as a preprotein or preproprotein, then a signal peptide as the pre-moiety is cleaved with a protease (generally called signal peptidase), and the secretory protein is converted into a mature protein or proprotein. As for the proprotein, the pro-moiety thereof is further cleaved by a protease, and the proprotein becomes a mature protein. Therefore, a signal peptide is used for the secretory production of a ncAA-containing protein in the method as described herein. A preprotein and a preproprotein of a secretory protein may be collectively referred to as "secretory protein precursor". The "signal peptide" (also referred to as "signal sequence") refers to an amino acid sequence that is present at the N-terminus of a secretory protein precursor and that is not usually present in the natural mature protein.

The genetic construct for secretory expression includes, in the direction from 5' to 3', a promoter sequence that functions in a coryneform bacterium, a nucleic acid sequence encoding a signal peptide, and a nucleic acid sequence encoding the ncAA-containing protein. The nucleic acid sequence encoding the signal peptide may be linked downstream of the promoter sequence so that the signal peptide is expressed under the control of the promoter. The nucleic acid sequence encoding the ncAA-containing protein may be linked downstream of the nucleic acid sequence encoding the signal peptide so that the ncAA-containing protein is expressed as a fusion protein with the signal peptide. This fusion protein is also referred to as "fusion protein of the present invention". The signal peptide and the ncAA-containing protein may or may not be adjacent to each other in the fusion protein of the present invention. That is, the expression "a ncAA-containing protein is expressed as a fusion protein with a signal peptide" encompasses not only a case in which a ncAA-containing protein is adjacent to a signal peptide so as to be expressed as a fusion protein with the signal peptide but also a case in which a ncAA-containing protein forms a fusion protein with a signal peptide via a different amino acid sequence so as to be expressed. For example, as described later, the fusion protein of the present invention may include an insertion sequence, such as an amino acid sequence including Gln-Glu-Thr or an amino acid sequence used for enzymatic cleavage, between the signal peptide and the ncAA-containing protein. In addition, as described later, the ncAA-containing protein eventually obtained may not have a signal peptide. That is, the expression "a ncAA-containing protein is expressed as a fusion protein with a signal peptide" may simply mean that the ncAA-containing protein constitutes a fusion protein with the signal peptide at the time of expression, and it may not necessarily mean that the eventually-obtained ncAA-containing protein constitutes a fusion protein with the signal peptide. A nucleic acid sequence may also be read as "gene". For example, a nucleic acid sequence encoding a ncAA-containing protein is also referred to as "gene encoding a ncAA-containing protein" or "ncAA-containing protein gene". Examples of the nucleic acid sequence include DNA. The genetic construct for secretory expression may also include a control sequence (operator, SD sequence, terminator, etc.) effective for expression of the fusion protein in a coryneform bacterium at such an appropriate position that it can function.

The promoter is not particularly limited so long as a promoter that functions in the chosen coryneform bacterium. The "promoter that functions in a coryneform bacterium" refers to a promoter that possesses promoter activity (i.e., gene transcription activity) in a coryneform bacterium. Examples of the promoter that functions in a coryneform bacterium include those described later in "Methods for Increasing Activity of Protein".

The signal peptide is not particularly limited so long as a signal peptide that functions in the chosen coryneform bacterium. The signal peptide may be a signal peptide derived from a coryneform bacterium, such as one derived from the host, or it may be a heterologous signal peptide. The signal peptide may be the native signal peptide of the ncAA-containing protein, or a signal peptide of another protein. The expression "signal peptide that functions in a coryneform bacterium" refers to a signal peptide that, upon being linked at the N-terminus of an objective protein, allows a coryneform bacterium to secrete the protein. Whether or not a signal peptide can function in a coryneform bacterium can be confirmed by, for example, allowing an objective protein to be fused with the signal peptide so as to be expressed and observing the secretion of the protein.

Examples of the signal peptide include a Tat-dependent signal peptide and a Sec-dependent signal peptide.

The term "Tat-dependent signal peptide" refers to a signal peptide recognized by the Tat system. The term "Tat-dependent signal peptide" may specifically refer to a signal peptide that, upon being linked at the N-terminus of an objective protein, results in secretion of the protein by the Tat secretion system.

Examples of a Tat-dependent signal peptide include, for example, a signal peptide of the TorA protein (trimethylamine-N-oxidoreductase) of *E. coli,* a signal peptide of the SufI protein (ftsI suppressor) of *E. coli,* a signal peptide of the PhoD protein (phosphodiesterase) of *Bacillus subtilis,* a signal peptide of the LipA protein (lipoic acid synthase) of *Bacillus subtilis,* a signal peptide of the IMD protein (isomaltodextranase) of *Arthrobacter globiformis.* The amino acid sequences of these signal peptides are as follows:
TorA signal peptide: MNNNDLFQASRRRFLAQLGGLTVAGMLGPSLLTPRRATA (SEQ ID NO: 18);
SufI signal peptide: MSLSRRQFIQASGIALCAGAVPLKASA (SEQ ID NO: 19);
PhoD signal peptide: MAYDSRFDEWVQKLKEESFQNNTFDRRKFIQGAGKIAGLSLGLTIAQS (SEQ ID NO: 20);
LipA signal peptide: MKFVKRRTTALVTTLMLSVTSLFALQPSAKAAEH (SEQ ID NO: 21); and
IMD signal peptide: MMNLSRRTLLTTGSAATLAYALGMAGSAQA (SEQ ID NO: 22).

The Tat-dependent signal peptide has a twin-arginine motif. Examples of the twin-arginine motif include, for example, S/T-R-R-X-F-L-K (SEQ ID NO: 23) and R-R-X-#-# (X: naturally occurring amino acid residue; #: hydrophobic amino acid residue).

The term "Sec-dependent signal peptide" refers to a signal peptide recognized by the Sec system. The term "Sec-dependent signal peptide" may specifically refer to a signal peptide that, upon being linked at the N-terminus of an objective protein, results in secretion of the protein by the Sec secretion system.

Examples of the Sec-dependent signal peptide include, for example, signal peptides of cell surface layer proteins of coryneform bacteria. Cell surface layer proteins of coryneform bacteria are as described above. Examples of cell surface layer proteins of coryneform bacteria include *C*. *glutamicum-derived* PS1 and PS2 (CspB) (Japanese Patent Laid-open (Kohyo) No. 6-502548) and *C*. *stationis-derived* SlpA (CspA) (Japanese Patent Laid-open (Kokai) No. 10-108675). The amino acid sequence of the PS1 signal peptide of *C*. *glutamicum* is shown in SEQ ID NO: 25, the amino acid sequence of the PS2 (CspB) signal peptide (PS2 signal peptide) of C. *glutamicum* is shown in SEQ ID NO: 26, and the amino acid sequence of the SlpA (CspA) signal peptide (SlpA signal peptide) of *C*. *stationis* is shown in SEQ ID NO: 27.

The Tat-dependent signal peptide may be a variant of any of the Tat-dependent signal peptides exemplified above, so long as it contains a twin-arginine motif and the original function thereof is maintained. The Sec-dependent signal peptide may be a variant of any of the Sec-dependent signal peptides exemplified above, so long as the original function thereof is maintained. The aforementioned descriptions concerning conservative variants of ncAA-aaRS and the ncAA-aaRS gene can be similarly applied to variants of the signal peptide and the gene encoding it. For example, the signal peptide may be a peptide having any of the amino acid sequences of the signal peptides exemplified above, but including substitution, deletion, insertion, and/or addition of one or several amino acid residues at one or several positions. The number meant by the term "one or several" used for a variant of the signal peptide is specifically, 1 to 7, 1 to 5, 1 to 3, or 1 to 2. The terms "TorAsignal peptide", "SufI signal peptide", "PhoD signal peptide", "LipA signal peptide", "IMD signal peptide", "PS1 signal peptide", "PS2 signal peptide", and "SlpA signal peptide" include not only the peptides of SEQ ID NOS: 18 to 22 and 25 to 27, respectively, but also include conservative variants thereof.

The expression "the original function is maintained" for a Tat-dependent signal peptide means that the peptide is recognized by the Tat system, and specifically, may mean that the peptide has a function of, upon being linked at the N-terminus of an objective protein, resulting in secretion of the protein by the Tat secretion system. Whether a peptide functions as the Tat-dependent signal peptide can be confirmed by, for example, confirming an increase in the secretory production amount of a protein linked with the peptide at the N-terminus due to enhancement of the Tat secretion system, or confirming a reduction in the secretory production amount of a protein linked with the peptide at the N-terminus due to deletion of the Tat secretion system.

The expression "the original function is maintained" for a Sec-dependent signal peptide means that the peptide is recognized by the Sec system, and specifically, may mean that the peptide has a function of, upon being linked at the N-terminus of an objective protein, resulting in secretion of the protein by the Sec secretion system. Whether a peptide functions as the Sec-dependent signal peptide can be confirmed by, for example, confirming an increase in the secretory production amount of a protein linked with the peptide at the N-terminus due to enhancement of the Sec secretion system, or confirming a reduction in the secretory production amount of a protein linked with the peptide at the N-terminus due to deletion of the Sec secretion system.

Signal peptides are generally cleaved by a signal peptidase when a translation product is secreted out of cells. That is, the ncAA-containing protein eventually obtained may not have a signal peptide. A gene encoding a signal peptide may be used in the natural form thereof but may be modified so as to have optimal codons according to the codon usage frequency of the chosen host.

A nucleic acid sequence encoding an amino acid sequence including Gln-Glu-Thr may be inserted between a nucleic acid sequence encoding a signal peptide and a nucleic acid sequence encoding a ncAA-containing protein in the genetic construct for secretory expression (WO2013/062029). The "amino acid sequence including Gln-Glu-Thr" is also referred to as an "insertion sequence used in the present invention". Examples of an insertion sequence used in the present invention include the amino acid sequence including Gln-Glu-Thr described in WO2013/062029. An insertion sequence used in the present invention can be preferably used in combination with, in particular, a Sec-dependent signal peptide.

The insertion sequence used in the present invention is preferably a sequence consisting of three or more amino acid residues from the N-terminus of the mature protein (hereinafter also referred to as "mature CspB" or "CspB mature protein") of a cell surface layer protein CspB of a coryneform bacterium. The "sequence consisting of three or more amino acid residues from the N-terminus" refers to an amino acid sequence ranging from the amino acid residue at position 1 to the amino acid residue at position 3 or more from the N-terminus.

Cell surface layer proteins CspB of coryneform bacteria will be described later. Specific examples of CspB include, for example, CspB of *C*. *glutamicum* ATCC13869, CspB of 28 *C. glutamicum* strains described later, and their variants. In the amino acid sequence of CspB of *C*. *glutamicum* ATCC13869 shown in SEQ ID NO: 11, amino acid residues at positions 1 to 30 correspond to the signal peptide, and amino acid residues at positions 31 to 499 correspond to the CspB mature protein. The amino acid sequence of the CspB mature protein of *C*. *glutamicum* ATCC13869, excluding the 30 amino acid residues of the signal peptide moiety, is shown in SEQ ID NO: 28. In mature CspB of *C*. *glutamicum* ATCC13869, the amino acid residues at positions 1 to 3 from the N-terminus correspond to Gln-Glu-Thr.

The insertion sequence used in the present invention is preferably an amino acid sequence ranging from the amino acid residue at position 1 to any of the amino acid residues at positions 3 to 50 of mature CspB. The insertion sequence used in the present invention is more preferably an amino acid sequence ranging from the amino acid residue at position 1 to any of the amino acid residues at positions 3 to 8, 17, and 50 of mature CspB. The insertion sequence used in the present invention is particularly preferably an amino acid sequence ranging from the amino acid residue at position 1 to any of the amino acid residues at positions 4, 6, 17, and 50 of mature CspB.

The insertion sequence used in the present invention is preferably, for example, an amino acid sequence selected from the group consisting of the following amino acid sequences A to H:
(A) Gln-Glu-Thr;
(B) Gln-Glu-Thr-Xaa1;
(C) Gln-Glu-Thr-Xaa1-Xaa2;
(D) Gln-Glu-Thr-Xaa1-Xaa2-Xaa3;
(E) amino acid sequence including addition of amino acid residues at positions 4 to 7 of mature CspB to Gln-Glu-Thr;
(F) amino acid sequence including addition of amino acid residues at positions 4 to 8 of mature CspB to Gln-Glu-Thr;
(G) amino acid sequence including addition of amino acid residues at positions 4 to 17 of mature CspB to Gln-Glu-Thr; and
(H) amino acid sequence including addition of amino acid residues at positions 4 to 50 of mature CspB to Gln-Glu-Thr.

Xaa1 is Asn, Gly, Thr, Pro, or Ala, Xaa2 is Pro, Thr, or Val, and Xaa3 is Thr or Tyr in the amino acid sequences A to H. The expression "including addition of amino acid residues at positions 4 to X of mature CspB to Gln-Glu-Thr" means that amino acid residues at positions 4 to X from the N-terminus of mature CspB are added to Thr of Gln-Glu-Thr in the amino acid sequences A to H. In general, amino acid residues at positions 1 to 3 from the N-terminus of mature CspB correspond to Gln-Glu-Thr. In this case, the expression " amino acid sequence including addition of amino acid residues at positions 4 to X to Gln-Glu-Thr" has the same meaning as an amino acid sequence consisting of amino acid residues at positions 1 to X of mature CspB.

In addition, specifically, the insertion sequence used in the present invention is preferably, for example, an amino acid sequence selected from the group consisting of Gln-Glu-Thr-Asn-Pro-Thr (SEQ ID NO: 32), Gln-Glu-Thr-Gly-Thr-Tyr (SEQ ID NO: 33), Gln-Glu-Thr-Thr-Val-Thr (SEQ ID NO: 34), Gln-Glu-Thr-Pro-Val-Thr (SEQ ID NO: 35), and Gln-Glu-Thr-Ala-Val-Thr (SEQ ID NO: 36).

The "amino acid residue at position X of mature CspB" refers to an amino acid residue corresponding to the amino acid residue at position X in SEQ ID NO: 28. Which amino acid residue is the "amino acid residue corresponding to the amino acid residue at position X in SEQ ID NO: 28" in the amino acid sequence of arbitrary mature CspB can be determined by alignment between the amino acid sequence of the arbitrary mature CspB and the amino acid sequence of SEQ ID NO: 28.

The " noncanonical amino acid-containing protein (ncAA-containing protein)" refers to a protein including ncAA in its amino acid sequence. The fact that a protein including ncAA is also referred to as the "protein including ncAA residues".

The "noncanonical amino acid (ncAA)" refers to any amino acid other than natural amino acids. ncAA may be an L-amino acid or a D-amino acid. In particular, ncAA may be an L-amino acid.

The "natural amino acids" refer to the following 20 amino acids: K (Lys), R (Arg), H (His), A (Ala), V (Val), L (Leu), I (Ile), G (Gly), S (Ser), T (Thr), P (Pro), F (Phe), W (Trp), Y (Tyr), C (Cys), M (Met), D (Asp), E (Glu), N (Asn), and Q (Gln). All natural amino acids (excluding Gly) are L-amino acids.

Examples of ncAA include natural amino acid derivatives. Particular examples of natural amino acid derivatives include tyrosine derivatives and lysine derivatives. The "natural amino acid derivatives" may refer to compounds having a partially modified natural amino acid structure. Examples of modification of the structure include substituting constituents of a natural amino acid with other constituents. Examples of constituents include atoms and functional groups. For example, in the case of a tyrosine derivative, examples of the constituent of tyrosine to be substituted include a phenolic hydroxyl group, a phenolic hydrogen atom, and a hydrogen atom of a benzene ring. The constituent introduced by substitution is not particularly limited, so long as secretory expression of a ncAA-containing protein is possible. The constituent introduced by substitution can be appropriately selected depending on conditions such as the intended use of ncAA. Examples of the constituent introduced by substitution include a halogen atom, an azide group, a nitro group, a sulfo group, a hydroxyl group, an alkyl group, an aryl group, an alkoxy group, and an acyl group. Examples of a halogen atom include a fluorine atom, a chlorine atom, a bromine atom, and an iodine atom. Modification of the structure also includes converting an L-amino acid to a D-amino acid. That is, examples of natural amino acid derivatives also include D-forms of natural amino acids.

Specific examples of ncAA include p-azido-L-phenylalanine (AzF), 3-azido-L-tyrosine (AzY), 3-chloro-L-tyrosine (ClY), 3-nitro-L-tyrosine (NOY), O-sulfo-L-tyrosine (SfY), L-pyrrolysine (Pyl), N_{δ}-alloc-L-lysine (AllocLys), compounds described in Liu CC, Schultz PG. Adding new chemistries to the genetic code. Annu Rev Biochem. 2010; 79:413-44., and compounds described in Wei Wan et. al., Pyrrolysyl-tRNA synthetase: an ordinary enzyme but an outstanding genetic code expansion tool, Biochim Biophys Acta. 2014 Jun;1844(6):1059-70. FIG. 1 shows compounds described in Liu CC, Schultz PG. Adding new chemistries to the genetic code. Annu Rev Biochem. 2010; 79:413-44. Figure 1 of the same reference is cited in FIG. 1. AzF is identical to Compound No. 7 in FIG. 1. In addition, Pyl is identical to Compound No. 59 in FIG. 1. In addition, AllocLys is identical to Compound No. 67 in FIG. 1. Particular examples of ncAA include AzF, AzY, ClY, NOY, and SfY. Particular examples of ncAA also include AllocLys.

For example, all of AzF, AzY, ClY, NOY, SfY, and Compounds Nos. 1 to 27, 31, 32, 34 to 36, 41 to 44, 46, and 48 to 50 in FIG. 1 can be tyrosine derivatives.

For example, all of Pyl, AllocLys, and Compounds Nos. 28, 33, 51, and 53 to 71 in FIG. 1 can be lysine derivatives.

The ncAA-containing protein may include one type of ncAA residue or two or more types of ncAA residues. The ncAA-containing protein may include a ncAA residue at one site or ncAA residues at two or more sites. In a case in which the ncAA-containing protein includes ncAA residues at two or more sites, the type of ncAA residue included in each site may be the same or different.

The ncAA-containing protein is not particularly limited except that it includes ncAA. The ncAA-containing protein may be a host-derived protein or a protein from different species (heterologous protein). The term "heterologous protein" refers to an exogenous protein relative to a host (i.e., the bacterium as described herein) that produces the protein. The ncAA-containing protein may be, for example, a protein derived from a microorganism, a protein derived from a plant, a protein derived from an animal, a protein derived from a virus, or even a protein of which the amino acid sequence is artificially designed. The ncAA-containing protein may particularly be a derived from human. The ncAA-containing protein may be a monomeric protein or a multimeric protein. The term "multimeric protein" refers to a protein that contains two or more subunits. In the multimer, the subunits may be linked by covalent bonds such as disulfide bonds, linked by noncovalent bonds such as hydrogen bonds and hydrophobic interaction, or linked by a combination thereof. The multimer can include one or more intermolecular disulfide bonds. The multimer may be a homo-multimer having a single kind of subunit, or may be a hetero-multimer having two or more kinds of subunits. When the multimeric protein is a hetero-multimer, it is sufficient that at least one subunit is a ncAA-containing protein. That is, all the subunits may include ncAA, or only some of the subunits may include ncAA. Although the ncAA-containing protein may be a secretory protein in nature, or may be a non-secretory protein in nature, it can be a secretory protein in nature. Furthermore, the ncAA-containing protein may be a Tat-dependent secretory protein in nature, or may be a Sec-dependent secretory protein in nature.

The ncAA-containing protein to be produced by secretory production may include single kind of protein, or two or more kinds of proteins. Moreover, when the ncAA-containing protein is a hetero-multimer, only a subunit including ncAA may be produced by secretory production. That is, when the ncAA-containing protein is a hetero-multimer, only one kind of subunit may be produced by secretory production, or two or more kinds of subunits may be produced by secretory production. That is, the term "secretory production of a ncAA-containing protein" includes not only secretory production of all the subunits constituting a ncAA-containing protein, but also secretory production of only a part of the subunits constituting a ncAA-containing protein.

Examples of the ncAA-containing protein include enzymes, physiologically active proteins, receptor proteins, antigenic proteins, and any other proteins including ncAA. In addition, "proteins" may encompass those referred to as peptides, such as oligopeptides and polypeptides.

Examples of enzymes include cellulase, xylanase, transglutaminase, protein glutaminase, protein-asparaginase, isomaltodextranase, protease, endopeptidase, exopeptidase, aminopeptidase, carboxypeptidase, collagenase, chitinase, γ-glutamylvaline synthase, glutamate-cysteine ligase, and glutathione synthase. Examples of transglutaminase include secretory-type transglutaminases of Actinomycetes such as *Streptoverticillium mobaraense* IFO 13819 (WO01/23591), *Streptoverticillium cinnamoneum* IFO 12852, *Streptoverticillium griseocarneum* IFO 12776, and *Streptomyces lydicus* (WO96/06931), and of filamentous fungi such as Oomycetes (WO96/22366). Examples of protein glutaminase include protein glutaminase of *Chryseobacterium proteolyticum* (WO2005/103278). Examples of isomaltodextranase include isomaltodextranase of *Arthrobacter globiformis* (WO2005/103278).

Examples of the physiologically active proteins include growth factors, hormones, cytokines, antibody-related molecules, and antibody mimetics.

Examples of the growth factors include epidermal growth factor (EGF), insulin-like growth factor-1 (IGF-1), transforming growth factor (TGF), nerve growth factor (NGF), brain-derived neurotrophic factor (BDNF), vascular endothelial growth factor (VEGF), granulocyte-colony stimulating factor (G-CSF), granulocyte-macrophage-colony stimulating factor (GM-CSF), platelet-derived growth factor (PDGF), erythropoietin (EPO), thrombopoietin (TPO), acidic fibroblast growth factor (aFGF or FGF1), basic fibroblast growth factor (bFGF or FGF2), keratinocyte growth factor (KGF-1 or FGF7, and, KGF-2 or FGF10), hepatocyte growth factor (HGF), stem cell factor (SCF), activin, and peptides that mimic their functions. Examples of activin include activins A, C, and E. Examples of peptides that mimic the functions of growth factors, such as those exemplified above, include aMD4dY-PA22 and EPO-PA22 (WO2021/112249).

Examples of hormones include insulin, glucagon, somatostatin, human growth hormone (hGH), parathyroid hormone (PTH), calcitonin, and exenatide.

Examples of cytokines include interleukins, interferons, and tumor necrosis factors (TNFs).

The growth factors, hormones, and cytokines may not be strictly distinguished from one another. For example, a physiologically active protein may be one of a growth factor, hormone, or cytokine, or may be a plurality of those.

Furthermore, a physiologically active protein may be an intact protein, or may be a part of a protein. Examples of a part of a protein include, for example, a part having physiological activity. Specific examples of a part having physiological activity include, for example, teriparatide, a physiologically active peptide consisting of the N-terminal 34 amino acid residues of parathyroid hormone (PTH).

The term "antibody-related molecule" may refer to a protein containing a molecular species having a single domain or a combination of two or more domains selected from a complete antibody. Examples of the domains from a complete antibody include heavy chain domains VH, CH1, CH2, and CH3, and light chain domains VL and CL. The antibody-related molecule may be a monomeric protein, or may be a multimeric protein, so long as it contains the above-mentioned molecular species. When the antibody-related molecule is a multimeric protein, it may be a homo-multimer having a single kind of subunit, or may be a hetero-multimer having two or more kinds of subunits. Specific examples of antibody-related molecules include, for example, complete antibody, Fab, F(ab'), F(ab')₂, Fc, dimer having a heavy chain (H chain) and a light chain (L chain), Fc-fusion protein, heavy chain (H chain), light chain (L chain), light chain Fv (scFv), sc(Fv)₂, disulfide-bonded Fv (sdFv), diabody, and VHH fragment (Nanobody(registered trademark)). Particular examples of antibody-related molecules include VHH fragment (Nanobody(registered trademark)). More specific examples of the antibody-related molecules include, for example, Trastuzumab, Adalimumab, Nivolumab, VHH antibody N15, and VHH antibody 9g8.

The "antibody mimetic" may refer to an organic compound capable of specifically binding to an antigen but is not structurally related to an antibody. Specific examples of the antibody mimetic include the Z domain of protein A (affibody). More specific examples of the antibody mimetic include, for example, the ZHER2 affibody.

Examples of receptor proteins include receptor proteins for physiologically active proteins and other physiologically active substances. Examples of the other physiologically active substances include neurotransmitters such as dopamine. A receptor protein may be an orphan receptor of which the corresponding ligand is not known.

The antigen proteins are not particularly limited, so long as they can induce an immune response. The antigen proteins can be appropriately selected depending on the intended object of the immune response. The antigen proteins can be used as vaccines, for example.

In addition, examples of other proteins include liver-type fatty acid-binding protein (LFABP), fluorescent protein, immunoglobulin-binding protein, albumin, fibroin-like protein, and extracellular protein. Examples of the fluorescent protein include green fluorescent protein (GFP) and monomeric red fluorescent protein (mRFP). Examples of the immunoglobulin-binding protein include Protein A, Protein G, and Protein L. Examples of albumin include human serum albumin. Examples of the fibroin-like protein include those disclosed in WO2017/090665 and WO2017/171001.

Examples of the extracellular protein include fibronectin, vitronectin, collagen, osteopontin, laminin, and partial sequences thereof. Laminin is a protein having a heterotrimeric structure having an α chain, a β chain, and a γ chain. Examples of laminin include laminin of mammals. Examples of the mammals include primates such as human, monkey, and chimpanzee; rodents such as mouse, rat, hamster, and guinea pig; and other various mammals such as rabbit, horse, cattle, sheep, goat, pig, dog, and cat. Particular examples of the mammals include human. Examples of the subunit chains of laminin (i.e., a, (α, β, and γ chains) include 5 kinds of α chains (α1 to α5), 3 kinds of β chains (β1 to β3), and 3 kinds of γ chains (γ1 to γ3). Laminin constitutes various isoforms depending on combinations of these subunits. Specific examples of laminin include, for example, laminin 111, laminin 121, laminin 211, laminin 213, laminin 221, laminin 311, laminin 321, laminin 332, laminin 411, laminin 421, laminin 423, laminin 511, laminin 521, and laminin 523. Examples of the partial sequence of laminin include laminin E8, which is an E8 fragment of laminin. Laminin E8 is a protein having a heterotrimeric structure having an E8 fragment of α chain (α chain E8), an E8 fragment of β chain (β chain E8), and an E8 fragment of γ chain (γ chain E8). The subunit chains of laminin E8 (i.e., α chain E8, β chain E8, and γ chain E8) are also collectively referred to as "E8 subunit chains". Examples of the E8 subunit chains includes E8 fragments of the laminin subunit chains exemplified above. Laminin E8 constitutes various isoforms depending on combinations of these E8 subunit chains. Specific examples of laminin E8 include, for example, laminin 111E8, laminin 121E8, laminin 211E8, laminin 221E8, laminin 332E8, laminin 421E8, laminin 411E8, laminin 511E8, and laminin 521E8.

The ncAA-containing protein gene is not particularly limited so long as it encodes a ncAA-containing protein as described above.

The ncAA-containing protein gene includes a codon encoding ncAA. The codon encoding ncAA is also referred to as "ncAA codon". The ncAA codon is not particularly limited, so long as secretory expression of a ncAA-containing protein is possible. Examples of the ncAA codon include a stop codon and an unnatural codon. Particular examples of the ncAA codon include a stop codon. Examples of the stop codon include UAA (ochre), UAG (amber), and UGA (opal). Particular examples of the stop codon include UAG (amber) and UGA (opal). Further, particular examples of the stop codon include UAG (amber). Note that "U" and "T" in nucleotide sequence should be read appropriately depending on the type of nucleic acid. Examples of the unnatural codon include codons with a length of 4 or more residues. Examples of a four-residue codons include CGGG and GGGU (Takahiro Hohsaka, Artificial Protein Synthesizing System Using Extended Genetic Code, SEIBUTSUBUTSURI 47(2), 124-128 (2007)).

The ncAA-containing protein gene may be, for example, a gene having a known or natural nucleotide sequence of a gene encoding a protein as described above, so long as it includes a ncAA codon. Similarly, the ncAA-containing protein may be, for example, a protein having a known or natural amino acid sequence of a protein as described above, so long as it includes ncAA. In addition, the ncAA-containing protein gene may be, for example, a variant of a gene having a known or natural nucleotide sequence of a gene encoding a protein as described above, so long as it includes a ncAA codon. Similarly, ncAA-containing protein may be, for example, a variant of a protein having a known or natural amino acid sequence of a protein as described above, so long as it includes ncAA. The aforementioned descriptions concerning conservative variants of ncAA-aaRS and the ncAA-aaRS gene can be similarly applied to variants of the ncAA-containing protein and ncAA-containing protein gene. For example, the ncAA-containing protein gene may be a gene encoding a protein having a known or natural amino acid sequence as described above, but including substitution, deletion, insertion, and/or addition of one or several amino acid residues at one or several positions, so long as it includes ncAA. Also, for example, the ncAA-containing protein gene may also be a gene encoding a protein having an amino acid sequence having an identity of 80% or more, 90% or more, 95% or more, 97% or more, or 99% or more, to the total amino acid sequence of a known or natural amino acid sequence as described above, so long as it includes ncAA. A protein specified with the type of organism from which the protein is derived is not limited to proteins per se found in that organism, and shall also include proteins having any of the amino acid sequences of proteins found in that organism and variants thereof. These variants may or may not be found in the organism. That is, for example, the term "protein derived from human" is not limited to proteins per se found in human, and shall also include proteins having any of the amino acid sequences of proteins found in human and variants thereof. The gene encoding a ncAA-containing protein may be modified to include a ncAA codon. Furthermore, in the gene encoding the ncAA-containing protein, any codon(s) may be replaced with respective equivalent codon(s) thereof. For example, the gene encoding the ncAA-containing protein may be modified so as to have optimal codons according to the codon usage frequency of the chosen host.

The genetic construct of the present invention may further include a nucleic acid sequence encoding an amino acid sequence used for enzymatic cleavage between a nucleic acid sequence encoding an amino acid sequence including Gln-Glu-Thr and a nucleic acid sequence encoding a ncAA-containing protein. By inserting an amino acid sequence used for enzymatic cleavage into the fusion protein of the present invention, the thus expressed fusion protein can be enzymatically cleaved, thereby obtaining a ncAA-containing protein.

The amino acid sequence used for enzymatic cleavage is not particularly limited, so long as it is a sequence recognized and cleaved by an enzyme that hydrolyzes a peptide bond. An available sequence depending on the amino acid sequence of the ncAA-containing protein can be appropriately selected. The nucleic acid sequence encoding an amino acid sequence used for enzymatic cleavage can be designed as appropriate based on the amino acid sequence. For example, the nucleic acid sequence encoding an amino acid sequence used for enzymatic cleavage can be designed so as to have optimal codons according to the codon usage frequency of the host.

The amino acid sequence used for enzymatic cleavage is preferably a recognition sequence of protease with high substrate specificity. Specific examples of such an amino acid sequence include, for example, a recognition sequence of Factor Xa protease and a recognition sequence of proTEV protease. Factor Xa protease recognizes the amino acid sequence of Ile-Glu-Gly-Arg (=IEGR) (SEQ ID NO: 37) in a protein, and proTEV protease recognizes the amino acid sequence of Glu-Asn-Leu-Tyr-Phe-Gln (=ENLYFQ) (SEQ ID NO: 38) in a protein so as to cleave the C-terminal side of each sequence specifically.

The N-terminal region of the ncAA-containing protein eventually obtained by the method as described herein may be the same as that of the natural protein, or may not be the same as that of the natural protein. For example, the N-terminal region of the eventually obtained ncAA-containing protein may be that of the natural protein including addition or deletion of one or several amino acid residues. Although the number of the "one or several" amino acid residues may differ depending on the full length or structure of the ncAA-containing protein, specifically, it is 1 to 20, 1 to 10, 1 to 5, or 1 to 3.

Furthermore, the ncAA-containing protein to be produced by secretory production may be a protein having a pro-structure moiety (proprotein). When the ncAA-containing protein to be produced by secretory production is a proprotein, the ncAA-containing protein to be eventually obtained may be the proprotein or may not be the proprotein. That is, the proprotein may be processed into the mature protein by cleavage of the pro-structure moiety. The cleavage can be attained with, for example, a protease. When a protease is used, generally, the proprotein is cleaved at a position substantially the same as that of the natural protein, or at exactly the same position as that of the natural protein so that the same mature protein as the natural mature protein is obtained, in view of the activity of the eventually obtained protein. Therefore, generally, a specific protease that cleaves the proprotein at such a position that the same protein as the naturally occurring mature protein is generated is most preferred. However, the N-terminal region of the ncAA-containing protein to be eventually obtained may not be the same as that of the natural protein as described above. For example, depending on type, purpose of use, etc. of the ncAA-containing protein to be produced, a protein having an N-terminus longer or shorter by one to several amino acid residues compared with the natural protein may have more appropriate activity. Proteases usable in the present invention include, for example, commercially available proteases such as Dispase (produced by Boehringer Mannheim) as well as those obtainable from culture broth of a microorganism such as culture broth of actinomycetes. Such proteases may be used in an un-purified state, or may be used after purification to an appropriate purity as required. When obtaining a mature protein by cleaving the pro-structure moiety, since the amino acid sequence including inserted Gln-Glu-Thr is cleaved and removed with the pro-structure moiety, an objective protein can be obtained without coordinating an amino acid sequence used for enzymatic cleavage after the amino acid sequence including Gln-Glu-Thr.

The method for introducing the genetic construct for secretory expression into the coryneform bacterium is not particularly limited. The phrase "introduction of the genetic construct for secretory expression" refers to making a host harbor the genetic construct. The phrase "introduction of the genetic construct for secretory expression" includes not only when the genetic construct that has been preliminarily constructed is collectively introduced into a host, but also includes when at least the ncAA-containing protein gene is introduced into a host and the genetic construct is constructed in the host. In the bacterium, the genetic construct for secretory expression may be present on a vector that autonomously replicates separate from the chromosome such as a plasmid, or may be incorporated into the chromosome. The genetic construct for secretory expression can be introduced, for example, in the same manner as for introduction of a gene in the "Methods for Increasing Activity of Protein" described later.

The genetic construct for secretory expression can be introduced into a host by using, for example, a vector that includes the genetic construct. For example, the genetic construct for secretory expression can be introduced into a host by linking the genetic construct with a vector to construct an expression vector of the genetic construct, and transforming the host with the expression vector. Also, when the vector contains a promoter that functions in a coryneform bacterium, an expression vector of the genetic construct for secretory expression can be constructed by linking the nucleic acid sequence encoding the fusion protein downstream from the promoter. The vector is not particularly limited so long as a vector autonomously replicable in a coryneform bacterium is chosen. The vector usable in a coryneform bacterium is as described above.

Furthermore, the genetic construct for secretory expression can be introduced into the chromosome of a host by using, for example, a transposon such as an artificial transposon. When a transposon is used, the genetic construct for secretory expression is introduced into the chromosome by homologous recombination or translocation ability of the transposon itself. Furthermore, the genetic construct for secretory expression can also be introduced into the chromosome of a host by other introduction methods utilizing homologous recombination. Examples of the introduction methods utilizing homologous recombination include, for example, methods utilizing a linear DNA, a plasmid having a temperature sensitive replication origin, a plasmid capable of conjugative transfer, a suicide vector not having a replication origin that functions in a host, and so forth. In addition, at least the ncAA-containing protein gene may be introduced into the chromosome so that the genetic construct for secretory expression is present on the chromosome. In this case, some or all of the constituents contained in the genetic construct for secretory expression, other than the ncAA-containing protein gene, may be inherently present on the chromosome of the host. Specifically, for example, by using a promoter sequence inherently present on the chromosome of the host and a nucleic acid sequence encoding a signal peptide linked downstream of the promoter sequence as they are, and replacing only a gene linked downstream of the nucleic acid sequence encoding a signal peptide with the ncAA-containing protein, the genetic construct for secretory expression can be incorporated into the chromosome, and the bacterium can be constructed. A part of the genetic construct for secretory expression, such as the ncAA-containing protein gene, can be introduced into the chromosome in the same manner as that for introduction of the genetic construct for secretory expression into the chromosome.

The genetic construct for secretory expression or a constituent thereof, such as promoter sequence, a nucleic acid sequence encoding a signal peptide, or nucleic acid sequence encoding a ncAA-containing protein, can be obtained by, for example, cloning. Specifically, for example, the genetic construct for secretory expression can be obtained by obtaining a ncAA-containing protein gene by cloning from an organism having the ncAA-containing protein, and then subjecting the gene to modification such as introduction of the ncAA codon, introduction of the nucleotide sequence encoding the signal peptide, and introduction of the promoter sequence. Furthermore, the genetic construct for secretory expression or a constituent thereof can also be obtained by chemical synthesis (Gene, 60(1), 115-127 (1987)). The obtained genetic construct or constituent thereof can be used as it is, or after being modified as required.

Furthermore, when two or more kinds of proteins are expressed, it is sufficient that the genetic constructs for secretory expression of the proteins are harbored by the bacterium so that secretory expression of the ncAA-containing proteins can be attained. Specifically, for example, all the genetic constructs for secretory expression of the proteins may be present on a single expression vector, or present on the chromosome. Alternatively, the genetic constructs for secretory expression of the proteins may be separately present on a plurality of expression vectors, or may be separately present on one or more expression vectors and the chromosome. The "case where two or more kinds of proteins are expressed" refers to, for example, a case where two or more kinds of ncAA-containing proteins are produced by secretory production, or a case where a hetero-multimeric protein is produced by secretory production.

The method for introducing the genetic construct for secretory expression into the coryneform bacterium is not particularly limited, and a generally used method, for example, the protoplast method (Gene, 39, 281-286 (1985)), the electroporation method (Bio/Technology, 7, 1067-1070 (1989)), the electric pulse method (Japanese Patent Laid-open (Kokai) No. 2-207791), and so forth can be used.

### <1-3> Expression of Orthogonal Pair of tRNA Corresponding to ncAA and aaRS

The coryneform bacterium as described herein has been modified to express an orthogonal pair of tRNA corresponding to ncAA and aaRS (i.e., orthogonal tRNA (ncAA)/ncAA-aaRS pair). The "expression of orthogonal tRNA (ncAA)/ncAA-aaRS pair" means the expression of tRNA and aaRS that constitute the orthogonal tRNA (ncAA)/ncAA-aaRS pair. tRNA and aaRS constituting the orthogonal tRNA (ncAA)/ncAA-aaRS pair are expressed from genes expressing them, respectively. That is, the coryneform bacterium as described herein has been modified to have genes encoding tRNA and aaRS constituting the orthogonal tRNA (ncAA)/ncAA-aaRS pair.

The tRNA constituting the orthogonal tRNA (ncAA)/ncAA-aaRS pair is tRNA corresponding to ncAA. tRNA corresponding to ncAA is also referred to as "tRNA (ncAA)" or "tRNA^{ncAA}". The gene encoding tRNA (ncAA) is also referred to as "tRNA gene corresponding to ncAA", "tRNA (ncAA) gene", or "tRNA^{ncAA} gene".

The aaRS constituting the orthogonal tRNA (ncAA)/ncAA-aaRS pair is aaRS corresponding to ncAA. The aaRS corresponding to ncAA is also referred to as "ncAA-aaRS". The gene encoding ncAA-aaRS is also referred to as "aaRS gene corresponding to ncAA" or "ncAA-aaRS gene".

The term "orthogonal" for an orthogonal tRNA (ncAA)/ncAA-aaRS pair means that the tRNA (ncAA) and ncAA-aaRS interact exclusively with each other. The expression "tRNA (ncAA) and ncAA-aaRS interact exclusively with each other" may mean that tRNA (ncAA) is recognized as a substrate by ncAA-aaRS but is not substantially recognized as a substrate by any host's endogenous aaRS, and ncAA-aaRS recognizes tRNA (ncAA) as a substrate but does not substantially recognize any host's endogenous tRNA as a substrate. Examples of the host's endogenous aaRS include the host's endogenous aaRSs corresponding to 20 types of natural amino acids. Examples of the host's endogenous tRNA include the host's endogenous tRNAs corresponding to 20 types of natural amino acids. The expression "tRNA (ncAA) is not substantially recognized as a substrate by any host's endogenous aaRS " may mean that, for example, for each host's endogenous aaRS, the Km for tRNA (ncAA) of the aaRS is 10 times or more, 100 times or more, or 1000 times or more the Km for tRNA (ncAA) of ncAA-aaRS, or tRNA (ncAA) is not recognized as a substrate by the aaRS at all. The expression "ncAA-aaRS does not substantially recognize any host's endogenous tRNA as a substrate" may mean that, for example, for each host's endogenous tRNA, the Km for the tRNA (ncAA) of ncAA-aaRS is 10 times or more, 100 times or more, or 1000 times or more the Km for tRNA (ncAA) of ncAA-aaRS, or ncAA-aaRS does not recognize the tRNA as a substrate at all.

"tRNA corresponding to ncAA" (tRNA (ncAA)) refers to RNA that functions as an adapter molecule for transferring ncAA to a peptide chain during translation. This function as an adapter molecule is also referred to as the "tRNA (ncAA) function". In addition, functioning as an adapter molecule is also referred to as "having the tRNA (ncAA) function". Specifically, "having the function of tRNA (ncAA)" may mean being aminoacylated by ncAA into aminoacyl tRNA and functioning as an adapter molecule for transferring ncAA to the peptide chain during translation. The aminoacyl tRNA of ncAA (i.e., tRNA aminoacylated by ncAA) is also referred to as "ncAA-tRNA".

tRNA (ncAA) has an anticodon corresponding to a ncAA codon. The anticodon corresponding to a ncAA codon is also referred to as "ncAA anticodon". tRNA (ncAA) may initially have a ncAA anticodon or may be modified to have a ncAA anticodon. The fact that tRNA (ncAA) has a ncAA anticodon is also referred to as that "tRNA (ncAA) gene has a ncAA anticodon".

Examples of tRNA (ncAA) include tRNA(Tyr) and tRNA(Pyl).

"tRNA(Tyr)" means tRNA corresponding to tyrosine. The gene encoding tRNA(Tyr) is also referred to as "tRNA(Tyr) gene".

Examples of the tRNA(Tyr) gene and tRNA(Tyr) include those from various organisms other than the host. Specific examples of the tRNA(Tyr) gene and tRNA(Tyr) include those of archaea such as methanogenic archaea, and those of bacteria such as bacteria of the genus *Escherichia* and bacteria of the genus *Geobacillus.* Examples of methanogenic archaea include archaea of the genus *Methanocaldococcus* such as *Methanocaldococcusjannaschii* (former name: *Methanococcus jannaschii*). Examples of bacteria of the genus *Escherichia* include *Escherichia coli.* Examples of bacteria of the genus *Geobacillus* include *Geobacillus stearothermophilus* (former name: *Bacillus stearothermophilus*). Examples of the tRNA(Tyr) gene and tRNA(Tyr) include those of archaea. More particular examples of the tRNA(Tyr) gene and tRNA(Tyr) include those of archaea of the genus *Methanocaldococcus* such as *Methanocaldococcusjannaschii.* The nucleotide sequences of the tRNA(Tyr) gene and tRNA(Tyr) from various organisms can be obtained from, for example, public databases such as NCBI and technical documents such as patent literature. The nucleotide sequence of the tRNA(Tyr) gene of *Methanocaldococcusjannaschii* and the nucleotide sequence of tRNA(Tyr) encoded by the gene are shown in SEQ ID NOS: 39 and 40, respectively. The nucleotide sequence of the tRNA gene becomes the nucleotide sequence of tRNA by replacing "T" with "U". In addition, the nucleotide sequence of tRNA becomes the nucleotide sequence of the tRNA gene by replacing "U" with "T".

The tRNA(Tyr) gene and tRNA(Tyr), as exemplified above, may be used as is or with appropriate modification as the tRNA (ncAA) gene and tRNA (ncAA). For example, in a case in which at least the anticodon of tRNA(Tyr) is not a ncAA anticodon, tRNA(Tyr) is used with a modification of the anticodon into a ncAA anticodon. Nucleotide sequences at positions 35 to 37 correspond to an anticodon in the nucleotide sequences of SEQ ID NOS: 39 and 40. One example of the nucleotide sequence of the modified tRNA(Tyr) gene of *Methanocaldococcus jannaschii* having an anticodon corresponding to UAG (amber) and the nucleotide sequence of modified tRNA(Tyr) encoded by the gene are shown in SEQ ID NOS: 41 and 42, respectively. Another example of the nucleotide sequence of the modified tRNA(Tyr) gene of *Methanocaldococcusjannaschii* having an anticodon corresponding to UAG (amber) and the nucleotide sequence of modified tRNA(Tyr) encoded by the gene are shown in SEQ ID NOS: 43 and 44, respectively. In addition, for example, tRNA(Tyr) may be modified to increase affinity for the host's elongation factors (Jiantao Guo et al., Evolution of amber suppressor tRNAs for efficient bacterial production of proteins containing nonnatural amino acids, Angew Chem Int Ed Engl, 2009;48(48):9148-51). Any modified tRNAs (Tyr) exemplified above may be modified to have increased affinity for the host's elongation factors. The modified tRNA(Tyr), as exemplified above, may be further modified and used. For example, the anticodon of modified tRNA(Tyr), as exemplified above, may be further modified.

"tRNA(Pyl)" means tRNA corresponding to pyrrolidine. The gene encoding tRNA(Pyl) is also referred to as "tRNA(Pyl) gene".

Examples of the tRNA(Pyl) gene and tRNA(Pyl) include those from various organisms other than the host. Specific examples of the tRNA(Pyl) gene and tRNA(Pyl) include those of archaea such as methanogenic archaea, and those of bacteria such as bacteria of the genus *Desulfitobacterium.* Examples of methanogenic archaea include archaea of the genus *Methanosarcina* such as *Methanosarcina barkerii* and *Methanosarcina mazei,* archaea of the genus *Methanomethylophilus* such as *Methanomethylophilus alvus,* unclassified archaea such as *Methanogenic* archaeon ISO4-G1. Examples of bacteria of the genus *Desulfitobacterium* include *Desulfitobacterium hafniense.* Particular examples of the tRNA(Pyl) gene and tRNA(Pyl) include those of archaea. More particular examples of the tRNA(Pyl) gene and tRNA(Pyl) include those of archaea of the genus *Methanosarcina* such as *Methanosarcina barkerii* and *Methanosarcina mazei.* The nucleotide sequences of the tRNA(Pyl) gene and tRNA(Pyl) from various organisms can be obtained from, for example, public databases such as NCBI and technical documents such as patent literature. The nucleotide sequence of the tRNA(Pyl) gene of *Methanosarcina barkerii* and the nucleotide sequence of tRNA(Pyl) encoded by the gene are shown in SEQ ID NOS: 45 and 46, respectively.

The tRNA(Pyl) gene and tRNA(Pyl), as exemplified above, may be used as is or with appropriate modification as the tRNA(Pyl) gene and tRNA(Pyl). For example, in a case in which at least the anticodon of tRNA(Pyl) is not a ncAA anticodon, tRNA(Pyl) is used with a modification of the anticodon into a ncAA anticodon. Nucleotide sequences at positions 31 to 33 correspond to an anticodon in the nucleotide sequences of SEQ ID NOS: 45 and 46. Anticodons of the nucleotide sequences of SEQ ID NOS: 45 and 46 are initially the anticodon corresponding to UAG (amber). The nucleotide sequence of the modified tRNA(Pyl) gene of *Methanosarcina mazei* having an anticodon corresponding to UAG (amber) and the nucleotide sequence of modified tRNA(Pyl) encoded by the gene are shown in SEQ ID NOS: 118 and 119, respectively. The nucleotide sequence of the modified tRNA(Pyl) gene of *Methanosarcina mazei* having an anticodon corresponding to UGA (opal) and the nucleotide sequence of modified tRNA(Pyl) encoded by the gene are shown in SEQ ID NOS: 120 and 121, respectively.

The "aaRS corresponding to ncAA (ncAA-aaRS)" means a protein having an activity of catalyzing a reaction of aminoacylating tRNA (ncAA) with ncAA to produce aminoacyl tRNA of ncAA (ncAA-tRNA). The activity is also referred to as "aaRS activity corresponding to ncAA" or "ncAA-aaRS activity". Specifically, the ncAA-aaRS activity may be an activity of catalyzing a reaction of aminoacylating tRNA (ncAA) with ncAA to produce aminoacyl tRNA of ncAA (ncAA-tRNA) in the presence of ATP.

Examples of ncAA-aaRS include Tyr-R and Pyl-RS.

"Tyr-RS" means tyrosyl tRNA synthetase. "Tyrosyl tRNA synthetase" means aaRS corresponding to tyrosine. The gene encoding Tyr-RS is also referred to as "Tyr-RS gene". Examples of ncAA that can be a substrate for Tyr-RS include tyrosine derivatives. Specific examples of ncAA that can be a substrate for Tyr-RS include AzF, AzY, ClY, NOY, SfY, and Compounds Nos. 1 to 15, 17 to 26, 31, 32, 34 to 36, 41 to 44, 46, and 48 to 50 in FIG. 1.

Examples of the Tyr-RS gene and Tyr-RS include those from various organisms other than the host. Specific examples of the Tyr-RS gene and Tyr-RS include those of organisms exemplified as the origins of the tRNA(Tyr) gene and tRNA(Tyr). Particular examples of the Tyr-RS gene and Tyr-RS include those of archaea. More particular examples of the Tyr-RS gene and Tyr-RS include those of archaea of the genus *Methanocaldococcus* such as *Methanocaldococcus jannaschii.* The nucleotide sequence of the Tyr-RS gene and the amino acid sequence of Tyr-RS from various organisms can be obtained from, for example, public databases such as NCBI and technical documents such as patent literature. The nucleotide sequence of the Tyr-RS gene of *Methanocaldococcus jannaschii* and the amino acid sequence of Tyr-RS encoded by the gene are shown in SEQ ID NOS: 47 and 48, respectively.

The Tyr-RS gene and Tyr-RS, as exemplified above, may be used as is or with appropriate modification as the ncAA-aaRS gene and ncAA-aaRS. For example, at least when Tyr-RS does not recognize ncAA as a substrate, Tyr-RS is used by modifying the substrate specificity so that it recognizes ncAA as a substrate (i.e., having the ncAA-aaRS activity). That is, Tyr-RS may have a mutation that modifies the substrate specificity.

Tyr-RS having a mutation that modifies the substrate specificity is also referred to as "mutant Tyr-RS" or "modified Tyr-RS". The gene encoding mutant Tyr-RS is also referred to as "mutant Tyr-RS gene" or "modified Tyr-RS gene". Tyr-RS that does not have a mutation that modifies the substrate specificity is also referred to as "wildtype Tyr-RS". The gene encoding wildtype Tyr-RS is also referred to as "wildtype Tyr-RS gene". The term "wildtype" referred to herein is used for convenience to distinguish "wildtype" Tyr-RS from "mutant" Tyr-RS, and Tyr-RS is not limited to those obtained in nature, so long as it does not have a mutation that modifies the substrate specificity. For example, the wildtype Tyr-RS may be a variant of wildtype Tyr-RS exemplified above (such as a protein having the amino acid sequence of SEQ ID NO: 48), so long as mutant Tyr-RS has the ncAA-aaRS activity. The descriptions concerning conservative variants of wildtype Tyr-RS can be applied mutatis mutandis to variants of ncAA-aaRS described below. The expression "Tyr-RS does not have a mutation that modifies the substrate specificity" may mean that Tyr-RS does not have a mutation selected as a mutation that modifies the substrate specificity. Wildtype Tyr-RS may or may not have mutations that were not selected as a mutation that modifies the substrate specificity, so long as they do not have mutations that were selected as a mutation that modifies the substrate specificity. The nucleotide sequence of the modified Tyr-RS gene of *Methanocaldococcus jannaschii,* which recognizes AzF as a substrate, and the amino acid sequence of modified Tyr-RS encoded by the gene are shown in SEQ ID NOS: 49 and 50, respectively. The nucleotide sequence of the Tyr-RS gene of *Methanocaldococcus jannaschii,* which recognizes halogenated tyrosine and AzY as substrates, and the amino acid sequence of Tyr-RS encoded by the gene are shown in SEQ ID NOS: 51 and 52, respectively. The modified Tyr-RS, as exemplified above, may be further modified and used. For example, the substrate specificity of modified Tyr-RS, as exemplified above, may be further modified.

Mutations that modify the substrate specificity of Tyr-RS include the following mutations in amino acid residues: (Jason W Chin et. al., Addition of p-azido-L-phenylalanine to the genetic code of Escherichia coli, J Am Chem Soc. 2002 Aug 7;124(31):9026-7; Biochem. Biophys. Res. Commun. 411, 757-761 (2011); and WO2004/070024): Y32, H70, E107, D158, I159, L162, and D286.

These mutations in amino acid residues can be effective, for example, to modify Tyr-RS to recognize AzF, AzY, and/or halogenated tyrosine (such as C1Y) as a substrate. Specifically, mutations at Y32, E107, D158, I159, and L162 may be effective, for example, in modifying Tyr-RS to recognize AzF as a substrate. Also, specifically, mutations at H70, D158, and I159 may be effective, for example, in modifying Tyr-RS to recognize AzY and/or halogenated tyrosine as a substrate. Also, specifically, a mutation at D286 may be effective, for example, in enhancing the substrate specificity for amber suppressor tRNA.

A mutation that modifies the substrate specificity of Tyr-RS may be a mutation in one amino acid residue or a combination of mutations in two or more amino acid residues. That is, mutations that modify the substrate specificity of Tyr-RS may include, for example, mutations of one or more mutations selected from these amino acid residues. The mutation that modifies the substrate specificity of Tyr-RS may be, for example, a mutation of one amino acid residue selected from these amino acid residues or a combination of mutations of two or more amino acid residues selected from these amino acid residues. A mutation that modifies the substrate specificity of Tyr-RS may be, for example, a combination of a mutation in one or more amino acid residues selected from Y32, H70, E107, D158, I159, and L162 and a mutation in D286. Specifically, the mutation that modifies the substrate specificity of Tyr-RS may be, for example, a combination of a mutation in one or more amino acid residues selected from Y32, E107, D158, I159, and L162, and a mutation in D286. Specifically, the mutation that modifies the substrate specificity of Tyr-RS may be, for example, a combination of a mutation in one or more amino acid residues selected from H70, D158, and 1159, and a mutation in D286.

In the notation above for specifying amino acid residues, the numbers indicate positions in the amino acid sequence of SEQ ID NO:48, and the letters to the left of the numbers indicate the amino acid residues at the respective positions in the amino acid sequence of SEQ ID NO: 48 (i.e., the amino acid residues at the respective positions before modification). That is, for example, "Y32" indicates a Y (Tyr) residue at position 32 in the amino acid sequence of SEQ ID NO: 48.

In any Tyr-RS, these amino acid residues each represent an "amino acid residue corresponding to the amino acid residue in the amino acid sequence of SEQ ID NO: 48". That is, for example, "Y32" in any Tyr-RS indicates an amino acid residue corresponding to the Y (Tyr) residue at position 32 in the amino acid sequence of SEQ ID NO: 48.

Each of the aforementioned mutations may be a substitution of an amino acid residue. In each of the aforementioned mutations, the amino acid residue after modification may be any amino acid residue other than the amino acid residue before modification as long as the desired substrate specificity can be obtained. That is, the modified amino acid residues may be selected from those that impart the desired substrate specificity. Specific examples of the modified amino acid residue include amino acid residues selected from K (Lys), R (Arg), H (His), A (Ala), V (Val), L (Leu), I (Ile), G (Gly), S (Ser), T (Thr), P (Pro), F (Phe), W (Trp), Y (Tyr), C (Cys), M (Met), D (Asp), E (Glu), N (Asn), and Q (Gln), which are other than the amino acid residues before modification.

Specific mutations that modify the substrate specificity of Tyr-RS include the following mutations: (Jason W Chin et. al., Addition of p-azido-L-phenylalanine to the genetic code of Escherichia coli, J Am Chem Soc. 2002 Aug 7;124(31):9026-7; Biochem. Biophys. Res. Commun. 411, 757-761 (2011); and WO2004/070024): Y32 (T, L, A, G), H70A, E107 (N, S, T, R, P), D158 (P, V, T, Q), 1159 (L, S, V, I, Y), L162 (Q, D, L, S), D286 (A, R, Y).

That is, mutations that modify the substrate specificity of Tyr-RS may include, for example, one or more mutations selected from these mutations. The mutation that modifies the substrate specificity of Tyr-RS may be, for example, one mutation selected from these mutations, or a combination of two or more mutations selected from these mutations. These mutations can be effective, for example, to modify Tyr-RS to recognize AzF, AzY, and/or halogenated tyrosine (such as C1Y) as a substrate. Specifically, Y32 (T, L, A, G), E107 (N, S, T, R, P), D158 (P, V, T, Q), 1159 (L, S, V, I, Y), and L162 (Q, D, L, S) may be effective, for example, in modifying Tyr-RS to recognize AzF as a substrate. Also, specifically, H70A, D158 (P, V, T, Q), and I159 (L, S, V, I, Y) may be effective, for example, in modifying Tyr-RS to recognize AzY and/or halogenated tyrosine as a substrate. Also, specifically, D286 (A, R, Y) may be effective, for example, in enhancing the substrate specificity for amber suppressor tRNA.

In the notation above for specifying mutations, the meanings of the numbers and the letters to the left thereof are the same as above. In the notation above for identifying mutations, the letters to the right of the numbers indicate the amino acid residues after modification at the respective positions. That is, for example, "Y32 (T, L, A, G)" means a mutation in which the Y (Tyr) residue at position 32 in the amino acid sequence of SEQ ID NO: 48 is substituted with a T (Thr) residue, an L (Leu) residue, an A (Ala) residue, or a G (Gly) residue.

In any Tyr-RS, these mutations each represent a "mutation corresponding to the mutation in the amino acid sequence of SEQ ID NO: 48". In any Tyr-RS, a "mutation corresponding to the mutation in which the amino acid residue at position X in the amino acid sequence of SEQ ID NO: 48 is substituted with one amino acid residue" should be read as a "mutation corresponding to the mutation in which an amino acid residue corresponding to the amino acid residue at position X in the amino acid sequence of SEQ ID NO: 48 is substituted with one amino acid residue". That is, for example, "Y32T" in any Tyr-RS indicates a mutation in which an amino acid residue corresponding to the Y (Tyr) residue at position 32 in the amino acid sequence of SEQ ID NO: 48 is substituted with a T (Thr) residue.

The combination of mutations is not particularly limited. Examples of the combination of mutations include the following combinations (Jason W Chin et. al., Addition of p-azido-L-phenylalanine to the genetic code of Escherichia coli, J Am Chem Soc. 2002 Aug 7;124(31):9026-7.; and Biochem. Biophys. Res. Commun. 411, 757-761 (2011)):
Y32T/E107N/D158P/I159L/L162Q;
Y32T/E107S/D158P/I159S/L162Q;
Y32T/E107S/D158P/I159L/L162Q;
Y32L/E107T/D158P/I159V/L162Q;
Y32A/E107A/D158V/I159I/L162A;
Y32G/E107T/D158T/I159Y/L162L;
Y32L/E107P/D158Q/I159I/L162S; and
H70A/D158T/I159S/D286R.

That is, mutations that modify the substrate specificity of Tyr-RS may include, for example, any of these mutations. Mutations that modify the substrate specificity of Tyr-RS may be, for example, a combination of any of these. A combination of these mutations can be effective, for example, to modify Tyr-RS to recognize AzF, AzY, and/or halogenated tyrosine (such as C1Y) as a substrate. Specifically, Y32T/E107N/D158P/I159L/L162Q, Y32T/E107S/D158P/I159S/L162Q, Y32T/E107S/D158P/I159L/L162Q, Y32L/E107T/D158P/I159V/L162Q, Y32A/E107A/D158V/I159I/L162A, Y32G/E107T/D158T/I159Y/L162L, and Y32L/E107P/D158Q/I159I/L162S may be effective, for example, in modifying Tyr-RS to recognize AzF as a substrate. Specifically, H70A/D158T/I159S/D286R may be effective, for example, in modifying Tyr-RS to recognize AzY and/or halogenated tyrosine as a substrate.

In the notation above for specifying a combination, the meanings of the numbers and the letters to the left and right thereof are the same as above. In the notation above for specifying a combination, the combination of two or more mutations separated by "/" indicates a double mutation or a multiple mutation. That is, for example, "Y32T/E107N/D158P/I159L/L162Q" indicates a quintuple mutation of Y32T, E107N, D158P, I159L, and L162Q.

The descriptions concerning the position of the "amino acid residue at position X of the wildtype PhoS protein" described later can be applied mutatis mutandis to the positions of amino acid residues mentioned in each of the aforementioned mutations in any Tyr-RS, except that the amino acid sequence of SEQ ID NO: 48 is used as a reference sequence.

"Pyl-RS" means pyrrolidyl tRNA synthetase. "Pyrrolidyl tRNA synthetase" means aaRS corresponding to pyrrolidine. The gene encoding Pyl-RS is also referred to as "Pyl-RS gene". Examples of ncAA that can be a substrate for Pyl-RS include lysine derivatives. Specific examples of ncAA that can be a substrate for Pyl-RS include Pyl, AllocLys, Compound Nos. 40, 51, and 59 to 71 in FIG. 1, and compounds described in Wei Wan et al., Pyrrolysyl-tRNA synthetase: an ordinary enzyme but an outstanding genetic code expansion tool, Biochim Biophys Acta. 2014 Jun;1844(6):1059-70.

Examples of the Pyl-RS gene and Pyl-RS include those from various organisms other than the host. Specific examples of the Pyl-RS gene and Pyl-RS include those of organisms exemplified as the origins of the tRNA(Pyl) gene and tRNA(Pyl). Particular examples of the Pyl-RS gene and Pyl-RS include those of archaea. More particular examples of the Pyl-RS gene and Pyl-RS include those of archaea of the genus *Methanosarcina* such as *Methanosarcina barkerii* and *Methanosarcina mazei.* The nucleotide sequences of the Pyl-RS gene and Pyl-RS from various organisms can be obtained from, for example, public databases such as NCBI and technical documents such as patent literature. The nucleotide sequence of the Pyl-RS gene of *Methanosarcina barkerii* and the nucleotide sequence of Pyl-RS encoded by the gene are shown in SEQ ID NOS: 53 and 54, respectively. The nucleotide sequence of the Pyl-RS gene of *Methanosarcina mazei* and the amino acid sequence of Pyl-RS encoded by the gene are shown in SEQ ID NOS: 114 and 115, respectively.

The Pyl-RS gene and Pyl-RS, as exemplified above, may be used as is or with appropriate modification as the ncAA-aaRS gene and ncAA-aaRS. For example, at least when Pyl-RS does not recognize ncAA as a substrate, Pyl-RS is used by modifying the substrate specificity so that it recognizes ncAA as a substrate (i.e., having the ncAA-aaRS activity). That is, Pyl-RS may have a mutation that modifies the substrate specificity.

Pyl-RS having a mutation that modifies the substrate specificity is also referred to as "mutant Pyl-RS" or "modified Pyl-RS". The gene encoding mutant Pyl-RS is also referred to as "mutant Pyl-RS gene" or "modified Pyl-RS gene". Pyl-RS that does not have a mutation that modifies the substrate specificity is also referred to as "wildtype Pyl-RS". The gene encoding wildtype Pyl-RS is also referred to as "wildtype Pyl-RS gene". The term "wildtype" referred to herein is used for convenience to distinguish "wildtype" Pyl-RS from "mutant" Pyl-RS, and Pyl-RS is not limited to those obtained in nature, so long as it does not have a mutation that modifies the substrate specificity. For example, the wildtype Pyl-RS may be a variant of wildtype Pyl-RS exemplified above (such as a protein having the amino acid sequence of SEQ ID NO: 54 or 115), so long as mutant Pyl-RS has the ncAA-aaRS activity. The descriptions concerning conservative variants of wildtype Pyl-RS can be applied mutatis mutandis to variants of ncAA-aaRS described below. The expression "Pyl-RS does not have a mutation that modifies the substrate specificity" may mean that Pyl-RS does not have a mutation selected as a mutation that modifies the substrate specificity. Wildtype Pyl-RS may or may not have mutations that were not selected as a mutation that modifies the substrate specificity, so long as they do not have mutations that were selected as a mutation that modifies the substrate specificity.

Mutations that modify the substrate specificity of Pyl-RS include the following mutations in amino acid residues: (Wei Wan et. al., Pyrrolysyl-tRNA synthetase: an ordinary enzyme but an outstanding genetic code expansion tool, Biochim Biophys Acta. 2014 Jun;1844(6):1059-70.):
M241, L266, A267, L270, Y271, L274, N311, C313, M315, Y349, V367, and W383.

A mutation that modifies the substrate specificity of Pyl-RS may be a mutation in one amino acid residue or a combination of mutations in two or more amino acid residues. That is, mutations that modify the substrate specificity of Pyl-RS may include, for example, mutations of one or more mutations selected from these amino acid residues. The mutation that modifies the substrate specificity of Pyl-RS may be, for example, a mutation of one amino acid residue selected from these amino acid residues or a combination of mutations of two or more amino acid residues selected from these amino acid residues. These mutations at amino acid residues may be effective, for example, in modifying Pyl-RS to recognize ncAA as a substrate as described in Wei Wan et. al., Pyrrolysyl-tRNA synthetase: an ordinary enzyme but an outstanding genetic code expansion tool, Biochim Biophys Acta. 2014 Jun;1844(6):1059-70.

Each of the aforementioned mutations may be a substitution of an amino acid residue. In each of the aforementioned mutations, the amino acid residue after modification may be any amino acid residue other than the amino acid residue before modification as long as the desired substrate specificity can be obtained. That is, the modified amino acid residues may be selected from those that impart the desired substrate specificity. Specific examples of the modified amino acid residue include amino acid residues selected from K (Lys), R (Arg), H (His), A (Ala), V (Val), L (Leu), I (Ile), G (Gly), S (Ser), T (Thr), P (Pro), F (Phe), W (Trp), Y (Pyl), C (Cys), M (Met), D (Asp), E (Glu), N (Asn), and Q (Gln), which are other than the amino acid residues before modification.

Specific mutations that modify the substrate specificity of Pyl-RS include the following mutations: (Wei Wan et. al., Pyrrolysyl-tRNA synthetase: an ordinary enzyme but an outstanding genetic code expansion tool, Biochim Biophys Acta. 2014 Jun;1844(6):1059-70.): M241F, L266(M, V, L), A267(S, L, F, T), L270(I, M, F), Y271(A, G, M, L, I, C, F), L274( A, G, M, P, L, S), N311(A, S, T, V, G), C313(A, V, S, F, C, T, K, L, W, G), M315F , Y349(F, W, L), V367(L, I), and W383(Y).

That is, mutations that modify the substrate specificity of Pyl-RS may include, for example, one or more mutations selected from these mutations. The mutation that modifies the substrate specificity of Pyl-RS may be, for example, one mutation selected from these mutations, or a combination of two or more mutations selected from these mutations. These mutations may be effective, for example, in modifying Pyl-RS to recognize ncAA as a substrate as described in Wei Wan et. al., Pyrrolysyl-tRNA synthetase: an ordinary enzyme but an outstanding genetic code expansion tool, Biochim Biophys Acta. 2014 Jun;1844(6):1059-70.

The combination of mutations is not particularly limited. Examples of the combination of mutations include the following combinations (Wei Wan et. al., Pyrrolysyl-tRNA synthetase: an ordinary enzyme but an outstanding genetic code expansion tool, Biochim Biophys Acta. 2014 Jun;1844(6):1059-70.): L274A/C313A/Y349F/, L274A/C313V/Y349F/, A267S/C313V/M315F/, C313V/, Y271A/Y349F/, L274A/C313V/, Y271A/Y349F/, Y271G/Y349F/, Y271M/L274G/C313A/, Y271A/L274M/C313A/, L274A/C313A/Y349F/, L274A/C313S/Y349F/, L266M/Y271L/L274A/C313F/Y349W/, L274M/C313A/Y349F/, Y271M/L274A/C313A/Y349F/, Y2711/L274A/C313A/Y349F/, A267S/Y271C/L274M/C313C/, M241F/Y349F/, Y271M/L274A/C313T/, Y271M/L274A/C313C/, Y271M/L274P/C313C/, Y271I/L274M/C313A/, Y349W/, Y271M/L274G/C313A/, Y271M/L274G/C313A/Y349W/, L266M/Y271L/L274A/C313F/, L266M/Y271L/L274L/C313 S/, L266V/L270I/Y271F/L274A/C313F/, L266L/L270I/Y271L/L274A/C313F/, L266M/L270I/Y271F/L274A/C313F/, Y271A/L274M/Y349F/, Y349W/, L274A/C313F/Y349F/, N311A/C313L/, N311A/C313K/, A267L/Y271M/N311S/C313L/Y349L/, A267F/Y271L/N311T/C313F/Y349L/, L270M/Y271L/L274S/N311S/C313M/, N311A/C313A/, A267T/N311V/C313W/Y349F/V367L/, L270F/Y271M/N311G/C313G/, A267T/N311T/C313T/, A267T/N311G/C313T/V367I/W383Y.

That is, mutations that modify the substrate specificity of Pyl-RS may include, for example, any of these mutations. Mutations that modify the substrate specificity of Pyl-RS may be, for example, a combination of any of these. These combinations may be effective, for example, in modifying Pyl-RS to recognize ncAA as a substrate as described in Wei Wan et. al., Pyrrolysyl-tRNA synthetase: an ordinary enzyme but an outstanding genetic code expansion tool, Biochim Biophys Acta. 2014 Jun;1844(6):1059-70.

For the notation of mutations that modify the substrate specificity of Pyl-RS, the description for the notation of mutations that modify the substrate specificity of Tyr-RS can be similarly applied, except that the amino acid sequence of SEQ ID NO: 54 is used as a reference sequence.

The tRNA (ncAA) gene may be, for example, a gene having the nucleotide sequence of tRNA (ncAA) gene (e.g., the nucleotide sequence of SEQ ID NO: 39, 41, 43, 45, 118, or 120 with a modified anticodon or the nucleotide sequence of SEQ ID NO: 41, 43, 45, 118, or 120) exemplified above. tRNA (ncAA) may be, for example, an RNA having the nucleotide sequence of tRNA (ncAA) (e.g., the nucleotide sequence of SEQ ID NO: 40, 42, 44, 46, 119, or 121 with a modified anticodon or the nucleotide sequence of SEQ ID NO: 42, 44, 46, 119, or 121) exemplified above. The ncAA-aaRS gene may be, for example, a gene having the nucleotide sequence of the ncAA-aaRS gene (e.g., the nucleotide sequence of SEQ ID NO: 47, 49, 51, 53, or 114 with a mutation that modifies the substrate specificity or the nucleotide sequence of SEQ ID NO: 49, 51, 53, or 114) exemplified above. ncAA-aaRS may be, for example, a protein having the amino acid sequence of ncAA-aaRS (e.g., the amino acid sequence of SEQ ID NO: 48, 50, 52, 54, or 115 with a mutation that modifies the substrate specificity or the amino acid sequence of SEQ ID NO: 50, 52, 54, or 115) exemplified above. The expression "gene or RNA has a nucleotide sequence" means that the gene or RNA has the nucleotide sequence unless otherwise stated. It may also encompass a case in which the gene or RNA consists of the nucleotide sequence. The expression "protein has an amino acid sequence " means that the protein has the amino acid sequence unless otherwise stated. It may also encompass a case in which the protein consists of the amino acid sequence.

The tRNA (ncAA) gene may be, for example, a variant of the tRNA (ncAA) gene (e.g., a gene having the nucleotide sequence of SEQ ID NO: 39, 41, 43, 45, 118, or 120 with a modified anticodon or a gene having the nucleotide sequence of SEQ ID NO: 41, 43, 45, 118, or 120) exemplified above, so long as the original function thereof is maintained. tRNA (ncAA) may be, for example, a variant of the tRNA (ncAA) (e.g., RNA having the nucleotide sequence of SEQ ID NO: 40, 42, 44, 46, 119, or 121 with a modified anticodon or RNA having the nucleotide sequence of SEQ ID NO: 42, 44, 46, 119, or 121) exemplified above, so long as the original function thereof is maintained. The ncAA-aaRS gene may be, for example, a variant of the ncAA-aaRS gene (e.g., a gene having the nucleotide sequence of SEQ ID NO: 47, 49, 51, 53, or 114 with a mutation that modifies the substrate specificity or a gene having the nucleotide sequence of SEQ ID NO: 49, 51, 53, or 114) exemplified above, so long as the original function thereof is maintained. Similarly, ncAA-aaRS may be, for example, a variant of the ncAA-aaRS (e.g., a protein having the amino acid sequence of SEQ ID NO: 48, 50, 52, 54, or 115 with a mutation that modifies the substrate specificity or a protein having the amino acid sequence of SEQ ID NO: 50, 52, 54, or 115) exemplified above, so long as the original function thereof is maintained. Such a variant is also referred to as "conservative variant". The term "tRNA (ncAA) gene" encompasses not only the tRNA (ncAA) gene exemplified above, but also encompasses conservative variants thereof. Similarly, the term "tRNA (ncAA)" encompasses not only the tRNA (ncAA) exemplified above, but also encompasses conservative variants thereof. The term "ncAA-aaRS gene" encompasses not only the ncAA-aaRS gene exemplified above, but also encompasses conservative variants thereof. Similarly, the term "ncAA-aaRS" encompasses not only the ncAA-aaRS exemplified above, but also encompasses conservative variants thereof. Examples of the conservative variants include, for example, homologues and artificially modified versions of the tRNA (ncAA) gene, tRNA (ncAA), the ncAA-aaRS gene, and ncAA-aaRS exemplified above.

The expression "the original function is maintained" means that a variant of a gene or protein has a function (such as activity or property) corresponding to the function (such as activity or property) of the original gene or protein. That is, the expression "the original function is maintained" used for the tRNA (ncAA) gene may mean that a variant of the gene encodes tRNA (ncAA). The expression "the original function is maintained" used for tRNA (ncAA) may mean that a variant of RNA has the function of tRNA (ncAA). The expression "the original function is maintained" used for the ncAA-aaRS gene may mean that a variant of the gene encodes ncAA-aaRS. In addition, the expression "the original function is maintained" used for ncAA-aaRS may mean that a variant of the protein has the ncAA-aaRS activity.

The ncAA-aaRS activity can be measured by, for example, incubating enzymes with substrates (ncAA and tRNA (ncAA)) in the presence of ATP and measuring the production of an enzyme- and substrate-dependent product (AMP or ncAA-tRNA).

Hereinafter, examples of the conservative variants will be explained.

Homologues of the tRNA (ncAA) gene, homologues of the ncAA-aaRS gene, or homologues of ncAA-aaRS can be easily obtained from public databases by, for example, BLAST search or FASTA search using any of the nucleotide sequences of the tRNA (ncAA) gene, the nucleotide sequences of the ncAA-aaRS gene, or the amino acid sequences of ncAA-aaRS exemplified above as a query sequence. Furthermore, homologues of the tRNA (ncAA) gene or homologues of the ncAA-aaRS gene can be obtained by, for example, PCR using a chromosome of an organism as the template, and oligonucleotides prepared on the basis of any of the nucleotide sequences of these known tRNA (ncAA) and ncAA-aaRS genes as primers.

The ncAA-aaRS gene may be a gene encoding a protein the aforementioned amino acid sequence (e.g., the amino acid sequence of SEQ ID NO: 48, 50, 52, 54, or 115 with a mutation that modifies the substrate specificity or the amino acid sequence of SEQ ID NO: 50, 52, 54, or 115), but which includes substitution, deletion, insertion, and/or addition of one or several amino acid residues at one or several positions, so long as the original function thereof is maintained. For example, the encoded protein may be extended or truncated at its N-terminus and/or C-terminus. Although the number meant by the term "one or several" mentioned above may differ depending on the positions of amino acid residues in the three-dimensional structure of the protein or the types of amino acid residues, specifically, it is, for example, 1 to 50, 1 to 40, 1 to 30, 1 to 20, 1 to 10, 1 to 5, or 1 to 3.

The aforementioned substitution, deletion, insertion, and/or addition of one or several amino acid residues is a conservative mutation that maintains the normal function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, or addition of amino acid residues as mentioned above includes a naturally occurring mutation due to an individual difference, or a difference of species of the organism from which the gene is derived (mutant or variant).

Also, for example, the ncAA-aaRS gene may also be a gene encoding a protein having an amino acid sequence having an identity of, for example, 50% or more, 65% or more, 80% or more, 90% or more, 95% or more, 97% or more, or 99% or more, to the total amino acid sequence of any of the aforementioned amino acid sequences, so long as the original function is maintained.

In addition, the tRNA (ncAA) gene or the ncAA-aaRS gene may be a gene (e.g., DNA) that hybridizes under stringent conditions with a probe that can be prepared from the aforementioned nucleotide sequence (e.g., the nucleotide sequence of SEQ ID NO: 39, 41, 43, 45, 118, or 120 with a modified anticodon or nucleotide sequence of SEQ ID NO: 41, 43, 45, 118, or 120 for tRNA (ncAA) gene; the nucleotide sequence of SEQ ID NO: 47, 49, 51, 53, or 114 with a mutation that modifies the substrate specificity or the nucleotide sequence of SEQ ID NO: 49, 51, 53, or 114 for the ncAA-aaRS gene), for example, a complementary sequence of the whole or a part of the aforementioned nucleotide sequence as long as the original function is maintained. tRNA (ncAA) may be RNA that hybridizes under stringent conditions with a probe that can be prepared from the aforementioned nucleotide sequence (e.g., the nucleotide sequence of SEQ ID NO: 40, 42, 44, 46, 119, or 121 with a modified anticodon or the nucleotide sequence of SEQ ID NO: 42, 44, 46, 119, or 121), for example, a complementary sequence of the whole or a part of the aforementioned nucleotide sequence as long as the original function is maintained. The term "stringent conditions" refers to conditions under which a so-called specific hybrid is formed, and a non-specific hybrid is not formed. Examples of the stringent conditions include those under which highly identical DNAs hybridize to each other, for example, DNAs not less than 50% identical, not less than 65% identical, not less than 80% identical, not less than 90% identical, not less than 95% identical, not less than 97% identical, or not less than 99% identical, hybridize to each other, and DNAs less identical than the above do not hybridize to each other, or conditions of washing of typical Southern hybridization, i.e., conditions of washing once, or 2 or 3 times, at a salt concentration and temperature corresponding to 1× SSC, 0.1% SDS at 60°C, 0.1×SSC, 0.1% SDS at 60°C, or 0.1×SSC, 0.1% SDS at 68°C.

The probe used for hybridization above may be a part of a sequence that is complementary to the gene as described above. Such a probe can be prepared by PCR using oligonucleotides prepared on the basis of known gene sequences as primers and a DNA fragment containing any of the aforementioned genes as a template. As the probe, for example, a DNA fragment having a length of about 300 bp can be used. In a case in which a DNA fragment approximately 300 bp in length is used as a probe, the washing conditions of the hybridization may be, for example, 50°C, 2×SSC and 0.1% SDS.

Furthermore, as codon degeneracy varies depending on the host, any codon(s) may be replaced with respective equivalent codon(s) thereof in the ncAA-aaRS gene. That is, the ncAA-aaRS gene may be a variant of the ncAA-aaRS gene exemplified above due to the degeneracy of the genetic code. For example, the ncAA-aaRS gene may have been modified so as to have optimal codons according to the codon usage frequency of the host to be used.

The term "identity" between amino acid sequences means an identity between the amino acid sequences calculated by blastp with default scoring parameters (i.e., Matrix, BLOSUM62; Gap Costs, Existence=11, Extension=1; Compositional Adjustments, Conditional compositional score matrix adjustment), unless otherwise stated. The term "identity" between nucleotide sequences means an identity between the nucleotide sequences calculated by blastn with default scoring parameters (i.e., Match/Mismatch Scores=1, -2; Gap Costs =Linear), unless otherwise stated.

The aforementioned descriptions concerning conservative variants of genes and proteins can be similarly applied to any gene or protein.

### <1-4> Other Characteristics

The bacterium may have desired characteristics, so long as it can produce a ncAA-containing protein by secretory production. For example, the activity of a cell surface layer protein may have been reduced in the bacterium (WO2013/065869, WO2013/065772, WO2013/118544, and WO2013/062029). Also, the bacterium may have been modified so that the activity of a penicillin-binding protein is reduced (WO2013/065869). Also, the bacterium may have been modified so that the expression of a gene encoding a metallopeptidase is increased (WO2013/065772). Also, the bacterium may have been modified so as to have a mutant ribosomal protein Si gene (mutant rpsA gene) (WO2013/118544). Also, the bacterium may have been modified so as to have a mutant phoS gene (WO2016/171224). Also, the bacterium may have been modified so that the activity of a RegX3 protein is reduced (WO2018/074578). Also, the bacterium may have been modified so that the activity of a HrrSA system is reduced (WO2018/074579). Also, the bacterium may have been modified so that the activity of the Tat secretion system is increased. These characteristics or modifications may occur alone or in any appropriate combination.

### <1-4-1> Introduction of Mutant phoS Gene

The bacterium may have been modified so as to harbor a mutant phoS gene. The expression "to harbor a mutant phoS gene" is also referred to as "to have a mutant phoS gene" or "to have a mutation in a phoS gene". In addition, the expression "to harbor a mutant phoS gene" is also referred to as "to have a mutant PhoS protein" or "to have a mutation in a PhoS protein".

Hereinafter, the phoS gene and the PhoS protein will be explained. The phoS gene is a gene encoding a PhoS protein, which is a sensor kinase of the PhoRS system. The PhoRS system is one of two-component regulatory systems, and induces a response against phosphate depletion. The PhoRS system has a sensor kinase PhoS encoded by a phoS gene and a response regulator PhoR encoded by a phoR gene.

A PhoS protein having the "specific mutation" is also referred to as "mutant PhoS protein", and a gene encoding it is also referred to as "mutant phoS gene". The mutant phoS gene is, in other words, a phoS gene having the "specific mutation". Furthermore, a PhoS protein not having the "specific mutation" is also referred to as "wildtype PhoS protein", and a gene encoding it is also referred to as "wildtype phoS gene". The wildtype phoS gene is, in other words, a phoS gene not having the "specific mutation". The term "wildtype" referred to herein is used for convenience to distinguish "wildtype" ones from "mutant" ones, and "wildtype" ones are not limited to those obtained as natural substances, so long as those do not have the "specific mutation". The "specific mutation" will be described later.

Examples of the wildtype phoS gene include, for example, phoS genes of coryneform bacteria. Specific examples of the phoS genes of coryneform bacteria include, for example, the phoS genes of *C*. *glutamicum* YDK010, *C. glutamicum* ATCC 13032, *C. glutamicum* ATCC 14067, *C*. *callunae, C. crenatum,* and *C*. *efficiens.* The nucleotide sequence of the phoS gene of C. *glutamicum* YDK010 is shown in SEQ ID NO: 1. The amino acid sequences of the wildtype PhoS proteins encoded by these phoS genes are shown in SEQ ID NOS: 2 to 7, respectively.

The wildtype phoS gene may be a variant of any of the wildtype phoS genes exemplified above, so long as it does not have the "specific mutation" and the original function thereof is maintained. Similarly, the wildtype PhoS protein may be a variant of any of the wildtype PhoS proteins exemplified above, so long as it does not have the "specific mutation" and the original function thereof is maintained. That is, the term "wildtype phoS gene" includes not only the wildtype phoS genes exemplified above, but also includes conservative variants thereof that do not have the "specific mutation". Similarly, the term "wildtype PhoS protein" includes not only the wildtype PhoS proteins exemplified above, but also includes conservative variants thereof that do not have the "specific mutation". The aforementioned descriptions concerning conservative variants of the ncAA-aaRS gene and ncAA-aaRS can be similarly applied to variants of the wildtype PhoS gene and wildtype PhoS protein. For example, the wildtype phoS gene may also be a gene encoding a protein having any of the aforementioned amino acid sequences, but which includes substitution, deletion, insertion, and/or addition of one or several amino acid residues at one or several positions, so long as it does not have the "specific mutation" and the original function thereof is maintained. Also, for example, the wildtype phoS gene may also be a gene encoding a protein having an amino acid sequence showing an identity of 80% or more, 90% or more, 95% or more, 97% or more, or 99% or more, to the total amino acid sequence of any of the aforementioned amino acid sequences, so long as it does not have the "specific mutation" and the original function thereof is maintained.

Incidentally, the expression "the original function is maintained" used for the wildtype PhoS may mean that a variant of the protein has a function as a PhoS protein (such as a function of a protein consisting of any of the amino acid sequences shown in SEQ ID NOS: 2 to 7). Furthermore, the expression "the original function is maintained" used for the wildtype PhoS protein may also mean that a variant of the protein has a function as a sensor kinase of the PhoRS system. That is, the term "function as a PhoS protein" may specifically refer to a function as a sensor kinase of the PhoRS system. The term "function as a sensor kinase of the PhoRS system" may specifically refer to a function of inducing a response against phosphate depletion in the environment in combination with a response regulator PhoR protein. The term "function as a sensor kinase of the PhoRS system" may more specifically refer to a function of sensing phosphate depletion in the environment to be autophosphorylated, and activating the PhoR protein via transfer of phosphate group.

Whether or not a variant of the PhoS protein has a function as a sensor kinase of the PhoRS system can be confirmed by, for example, introducing a gene encoding the variant into a phoS-gene-deletion strain of a coryneform bacterium, and confirming whether or not responsiveness against phosphate depletion is complemented. Complementation of responsiveness against phosphate depletion can be detected, for example, as improvement of growth under phosphate depletion conditions, or as induction of the expression of genes of which the expression is known to be induced under phosphate depletion conditions (J. Bacteriol., 188, 724-732(2006)). As the phoS-gene-deletion strain of a coryneform bacterium, for example, a phoS-gene-deletion strain of C. *glutamicum* YDK010 or a phoS-gene-deletion strain of C. *glutamicum* ATCC 13032 can be used.

It is preferred that a histidine residue that is autophosphorylated is conserved in the wildtype PhoS protein. That is, it is preferred that a conservative mutation occurs at an amino acid residue other than the histidine residue that is autophosphorylated. The term "histidine residue that is autophosphorylated" refers to a histidine residue at position 276 of the wildtype PhoS protein. Furthermore, it is preferred that, for example, the wildtype PhoS protein has a conservative sequence of the wildtype PhoS proteins exemplified above. That is, it is preferred that a conservative mutation occurs at, for example, an amino acid residue not conserved in the wildtype PhoS proteins exemplified above.

The mutant PhoS protein has the "specific mutation" in the amino acid sequence of such a wildtype PhoS protein as described above.

That is, in other words, the mutant PhoS protein may be identical to any of the wildtype PhoS proteins exemplified above or conservative variants thereof except that the mutant PhoS protein has the "specific mutation". Specifically, the mutant PhoS protein may be, for example, a protein having any of the amino acid sequences shown in SEQ ID NOS: 2 to 7 except that the mutant PhoS protein has the "specific mutation". Specifically, the mutant PhoS protein may also be, for example, a protein having any of the amino acid sequences shown in SEQ ID NOS: 2 to 7 but including substitution, deletion, insertion, and/or addition of one or several amino acid residues, except that the mutant PhoS protein has the "specific mutation". Specifically, the mutant PhoS protein may also be, for example, a protein showing an identity of 80% or more, 90% or more, 95% or more, 97% or more, or 99% or more, to any of the amino acid sequences shown in SEQ ID NOS: 2 to 7 except that the mutant PhoS protein has the "specific mutation".

Furthermore, in other words, the mutant PhoS protein may be a variant of any of the wildtype PhoS proteins exemplified above having the "specific mutation", and further including a conservative mutation at a site other than that of the "specific mutation". Specifically, the mutant PhoS protein may be, for example, a protein having any of the amino acid sequences shown in SEQ ID NOS: 2 to 7 but having the "specific mutation", and further including substitution, deletion, insertion, and/or addition of one or several amino acid residues at a site other than that of the "specific mutation".

The mutant phoS gene is not particularly limited so long as it encodes such a mutant PhoS protein as described above.

Hereinafter, the "specific mutation" of the mutant PhoS protein will be explained.

The "specific mutation" is not particularly limited, so long as it is a mutation that changes the amino acid sequence of such a wildtype PhoS protein described above, and that is effective for secretory production a ncAA-containing protein.

It is preferred that the "specific mutation" is a mutation that improves the secretory production amount of a ncAA-containing protein. The expression "to improve the secretory production amount of a ncAA-containing protein" means that a coryneform bacterium modified so as to have a mutant phoS gene (modified strain) is able to produce the ncAA-containing protein by secretory production in an amount larger than that obtainable with a non-modified strain. The "non-modified strain" refers to a control strain not having the "specific mutation" in the phoS gene, i.e., a control strain not having any mutant phoS gene, and it may be, for example, a wildtype strain or a parent strain. Although the degree of increase meant by the expression "to produce a ncAA-containing protein by secretory production in an amount larger than that obtainable with a non-modified strain" is not particularly limited so long as the secretory production amount of the ncAA-containing protein is increased compared with that obtainable with a non-modified strain, the expression may mean that the ncAA-containing protein is produced by secretory production in an amount of, for example, 1.1 times or more, 1.2 times or more, 1.3 times or more, or 2 times or more, of that obtainable with a non-modified strain, in terms of the accumulation amount in the medium and/or on the cell surface layer. In addition, the expression "to produce a ncAA-containing protein by secretory production in an amount larger than that obtainable with a non-modified strain" may also mean that whereas the ncAA-containing protein cannot be detected when a non-concentrated culture supernatant of a non-modified strain is applied to SDS-PAGE and stained with CBB, the ncAA-containing protein can be detected when a non-concentrated culture supernatant of a modified strain is applied to SDS-PAGE and stained with CBB. Incidentally, the expression "to improve the secretory production amount of a ncAA-containing protein" does not necessarily mean that the secretory production amount of every ncAA-containing protein is improved, and it is sufficient that the secretory production amount of a ncAA-containing protein chosen as the target of secretory production is improved. The expression "to improve the secretory production amount of a ncAA-containing protein" may specifically mean, for example, that the secretory production amount of a ncAA-containing protein described in the Example section is improved.

Whether a certain mutation improves the secretory production amount of a ncAA-containing protein can be confirmed by, for example, preparing a strain modified so as to have a gene encoding the PhoS protein having the certain mutation, quantifying the amount of the ncAA-containing protein produced by secretory production when the strain is cultured in a medium, and comparing it with the amount of the ncAA-containing protein produced by secretory production before the modification (non-modified strain) is cultured in the medium.

Examples of the change of the amino acid sequence include substitution of an amino acid residue. That is, it is preferred that the "specific mutation" is replacing an amino acid residue of the wildtype PhoS protein with another amino acid residue. The amino acid residue substituted by the "specific mutation" may be one residue, or may be a combination of two or more residues. The amino acid residue substituted by the "specific mutation" may be an amino acid residue other than the histidine residue that is autophosphorylated. The amino acid residue substituted by the "specific mutation" may be an amino acid residue in the HisKA domain other than the histidine residue that is autophosphorylated. The term "histidine residue that is autophosphorylated" refers to a histidine residue at position 276 of the wildtype PhoS protein. The term "HisKA domain" refers to a region having amino acid residues at positions 266-330 of the wildtype PhoS protein. The amino acid residue substituted by the "specific mutation" may be a tryptophan residue at position 302 of the wildtype PhoS protein (W302).

In the aforementioned mutation, examples of the amino acid residue after substitution include K(Lys), R(Arg), H(His), A(Ala), V(Val), L(Leu), I(Ile), G(Gly), S(Ser), T(Thr), P(Pro), F(Phe), W(Trp), Y(Tyr), C(Cys), M(Met), D(Asp), E(Glu), N(Asn), and Q(Gln), provided that the amino acid residue after substitution is other than the original one. As the amino acid residue after substitution, for example, one resulting in improvement in the secretory production amount of a ncAA-containing protein can be chosen.

When substitution occurs at W302, examples of the amino acid residue after substitution include amino acid residues other than aromatic amino acid and histidine residues. Specific examples of the "amino acid residues other than aromatic amino acid and histidine residues" include K(Lys), R(Arg), A(Ala), V(Val), L(Leu), I(Ile), G(Gly), S(Ser), T(Thr), P(Pro), C(Cys), M(Met), D(Asp), E(Glu), N(Asn), and Q(Gln). More specific examples of the "amino acid residues other than aromatic amino acid and histidine residues" include K(Lys), A(Ala), V(Val), S(Ser), C(Cys), M(Met), D(Asp), and N(Asn).

Incidentally, the term "specific mutation" used for the phoS gene refers to a mutation on the nucleotide sequence thereof that results in such a "specific mutation" as described above into the encoded PhoS protein.

The "amino acid residue at position X of the wildtype PhoS protein" refers to an amino acid residue corresponding to the amino acid residue at position X in SEQ ID NO: 2. For example, "W302" refers to an amino acid residue corresponding to the tryptophan residue at position 302 in SEQ ID NO: 2. The aforementioned positions of amino acid residues indicate relative positions, and the absolute positions thereof may shift due to deletion, insertion, addition, etc. of an amino acid residue or residues. For example, if one amino acid residue is deleted or inserted at a position on the N-terminal side of position X in the wildtype PhoS protein having the amino acid sequence shown in SEQ ID NO: 2, the amino acid residue originally at position X is relocated at position X-1 or X+1 counted from the N-terminus, however, it is still regarded as the "amino acid residue at position X of the wildtype PhoS protein". Specifically, for example, "W302" refers to the tryptophan residue at positions 302, 302, 302, 321, 275, and 286, respectively, in the amino acid sequences of wildtype PhoS proteins shown in SEQ ID NOS: 2 to 7. Furthermore, the "histidine residue at position 276 of the wildtype PhoS protein (histidine residue that is autophosphorylated)" refers to the histidine residue at positions 276, 276, 276, 295, 249, and 260, respectively, in the amino acid sequences of wildtype PhoS proteins shown in SEQ ID NOS: 2 to 7. Furthermore, the "region having amino acid residues at positions 266-330 of the wildtype PhoS protein (HisKA domain)" refers to the region having amino acid residues at positions 266-330, 266-330, 266-330, 285-349, 239-303, and 250-314, respectively, in the amino acid sequences of wildtype PhoS proteins shown in SEQ ID NOS: 2 to 7.

Incidentally, while "W302" referred to herein is typically a tryptophan residue, it may also be other than a tryptophan residue. That is, when the wildtype PhoS protein has an amino acid sequence other than the amino acid sequences shown in SEQ ID NOS: 2 to 7, "W302" can be other than a tryptophan residue. Hence, for example, the "mutation replacing W302 with a cysteine residue" includes not only a mutation, when "W302" is a tryptophan residue, for replacing this tryptophan residue with a cysteine residue, but also includes a mutation, when "W302" is K (Lys), R (Arg), H (His), A (Ala), V (Val), L (Leu), I (Ile), G (Gly), S (Ser), T (Thr), P (Pro), F (Phe), Y (Tyr), M (Met), D (Asp), E (Glu), N (Asn), or Q (Gln), for replacing this residue with a cysteine residue. The same can be similarly applied to the other mutations.

Which amino acid residue is the "amino acid residue corresponding to the amino acid residue at position X in SEQ ID NO: 2" in the amino acid sequence of an arbitrary PhoS protein can be determined by alignment between the amino acid sequence of the arbitrary PhoS protein and the amino acid sequence of SEQ ID NO: 2. The alignment can be performed by, for example, using known gene analysis software. Specific examples of such software include DNASIS produced by Hitachi Solutions, GENETYX produced by Genetyx, and so forth (Elizabeth C. Tyler et al., Computers and Biomedical Research, 24 (1) 72- 96, 1991; Barton G J et al., Journal of Molecular Biology, 198 (2), 327- 37, 1987).

The mutant phoS gene can be obtained by, for example, modifying a wildtype phoS gene so that the encoded PhoS protein has the aforementioned "specific mutation". The wildtype phoS gene to be modified can be obtained by, for example, cloning from an organism having the wildtype phoS gene, or chemical synthesis. Furthermore, the mutant phoS gene can also be obtained without using a wildtype phoS gene. For example, the mutant phoS gene may be directly obtained by chemical synthesis. The obtained mutant phoS gene may be further modified before use.

Genes can be modified by known methods. For example, an objective mutation can be introduced into a target site of DNA by the site-specific mutagenesis method. Examples of the site-specific mutagenesis method include a method of using PCR (Higuchi, R., 61, in PCR Technology, Erlich, H. A. Eds., Stockton Press (1989); Carter P, Meth. In Enzymol., 154, 382 (1987)), and a method of using a phage (Kramer, W and Frits, H. J., Meth. in Enzymol., 154, 350 (1987); Kunkel, T. A. et al., Meth. in Enzymol., 154, 367 (1987)).

Hereinafter, methods for modifying a coryneform bacterium so as to have a mutant phoS gene will be explained.

A coryneform bacterium can be modified so as to have a mutant phoS gene by introducing the mutant phoS gene into the coryneform bacterium. A coryneform bacterium can be modified so as to have a mutant phoS gene also by introducing a mutation into the phoS gene on the chromosome of the coryneform bacterium. A mutation can be introduced into a gene on a chromosome by natural mutation, mutagenesis treatment, or genetic engineering means.

Methods for introducing a mutant phoS gene into a coryneform bacterium are not particularly limited. It is sufficient that the mutant phoS gene is harbored by the bacterium so that it can be expressed under control of a promoter that functions in a coryneform bacterium. The promoter may be a promoter derived from the host, or may be a heterogenous promoter. The promoter may be the native promoter of the phoS gene, or a promoter derived from another gene. In the bacterium, the mutant phoS gene may be present on a vector that autonomously replicates out of the chromosome, such as plasmid, or may be incorporated into the chromosome. The bacterium may have only one copy of the mutant phoS gene, or two or more copies of the mutant phoS gene. The bacterium may have only one kind of mutant phoS gene, or two or more kinds of mutant phoS genes. The mutant phoS gene can be introduced, for example, in the same manner as that for introduction of a gene in methods for increasing the expression of a gene described below, or for introduction of the genetic construct for secretory expression as described below.

The bacterium may or may not have the wildtype phoS gene. It is preferred that the bacterium does not have the wildtype phoS gene.

A coryneform bacterium not having the wildtype phoS gene can be obtained by disrupting the wildtype phoS gene on the chromosome. The wildtype phoS gene can be disrupted by known methods. Specifically, the wildtype phoS gene can be disrupted by, for example, deleting a portion or the entire the promoter region and/or the coding region of the wildtype phoS gene.

Furthermore, by replacing the wildtype phoS gene on the chromosome with a mutant phoS gene, a coryneform bacterium modified so that it does not have the wildtype phoS gene and has the mutant phoS gene can be obtained. Examples of methods for performing such gene substitution include, for example, a method of using a linear DNA such as a method called "Red-driven integration" (Datsenko, K. A, and Wanner, B. L., Proc. Natl. Acad. Sci. USA, 97:6640-6645 (2000)), a method of utilizing the Red driven integration in combination with an excision system derived from λ phage (Cho, E. H., Gumport, R I., Gardner, J. F., J. Bacteriol., 184:5200-5203 (2002)) (refer to WO2005/010175), a method of using a plasmid including a temperature sensitive replication origin, a method of using a plasmid capable of conjugative transfer, a method of utilizing a suicide vector not including a replication origin that functions in a host (U.S. Pat. No. 6,303,383, Japanese Patent Laid-open (Kokai) No. 05-007491), and so forth.

The PhoS protein functions, that is, induces a response against phosphate depletion in the environment, in combination with a response regulator PhoR protein. Hence, the bacterium has a phoR gene so that the mutant PhoS protein functions. The phoR gene is a gene encoding a PhoR protein, which is a response regulator of the PhoRS system. The expression "to have a phoR gene" is also referred to as "to have a PhoR protein". Typically, it is sufficient that the PhoR protein inherently possessed by the bacterium functions in combination with the mutant PhoS protein. Alternatively, the bacterium may be introduced with an appropriate phoR gene, in addition to or instead of the phoR gene inherently possessed by the bacterium. The phoR gene to be introduced is not particularly limited, as long as it encodes a PhoR protein that functions in combination with the mutant PhoS protein.

Examples of the phoR gene include, for example, phoR genes of coryneform bacteria. Specific examples of the phoR genes of coryneform bacteria include, for example, the phoR genes of *C*. *glutamicum* YDK010, *C. glutamicum* ATCC 13032, *C. glutamicum* ATCC 14067, *C*. *callunae, C. crenatum,* and *C*. *efficiens.* The nucleotide and amino acid sequences of the phoR gene and protein of *C*. *glutamicum* ATCC 13032 are shown in SEQ ID NO: 8 and 9, respectively.

The phoR gene may be a variant of any of the phoR genes exemplified above, so long as the original function thereof is maintained. Similarly, the PhoR protein may be a variant of any of the PhoR proteins exemplified above, so long as the original function thereof is maintained. That is, the term "phoR gene" includes not only the phoR genes exemplified above, but also includes conservative variants thereof. Similarly, the term "PhoR protein" includes not only the PhoR proteins exemplified above, but also includes conservative variants thereof. The aforementioned descriptions concerning conservative variants of the ncAA-aaRS gene and ncAA-aaRS can be similarly applied to variants of the phoR gene and PhoR protein. For example, the phoR gene may be a gene encoding a protein having the aforementioned amino acid sequence, but including substitution, deletion, insertion, and/or addition of one or several amino acid residues at one or several positions, so long as the original function is maintained. Also, for example, the phoR gene may also be a gene encoding a protein having an amino acid sequence having an identity of 80% or more, 90% or more, 95% or more, 97% or more, or 99% or more, to the total amino acid sequence of any of the aforementioned amino acid sequences, so long as the original function is maintained. Incidentally, the expression "the original function is maintained" used for the PhoR protein may mean that a variant of the protein has a function as a PhoR protein (such as a function of a protein consisting of the amino acid sequence shown in SEQ ID NO: 9). Furthermore, the expression "the original function is maintained" used for the PhoR protein may also mean that a variant of the protein has a function as a response regulator of the PhoRS system. That is, the term "function as a PhoR protein" may specifically refer to a function as a response regulator of the PhoRS system. The term "function as a response regulator of the PhoRS system" may specifically refer to a function of inducing a response against phosphate depletion in the environment in combination with a sensor kinase PhoS protein. The term "function as a response regulator of the PhoRS system" may more specifically refer to a function of being activated via transfer of phosphate group from the PhoS protein that sensed phosphate depletion in the environment to be autophosphorylated, and regulating the expression of genes that respond to phosphate depletion in the environment.

Whether or not a variant of the PhoR protein functions as a response regulator of the PhoRS system can be confirmed by, for example, introducing a gene encoding the variant into a phoR-gene-deletion strain of a coryneform bacterium, and confirming whether or not responsiveness against phosphate depletion is complemented. Complementation of responsiveness against phosphate depletion can be detected, for example, as improvement of growth under phosphate depletion conditions, or as induction of the expression of genes of which the expression is known to be induced under phosphate depletion conditions (J. Bacteriol., 188, 724-732(2006)). As the phoR-gene-deletion strain of a coryneform bacterium, for example, a phoR-gene-deletion strain of *C*. *glutamicum* YDK010 or a phoR-gene-deletion strain of *C*. *glutamicum* ATCC 13032 can be used.

### <1-4-2>Reduction in Activity of Cell Surface Layer Protein

The bacterium may be a bacterium in which the activity(s) of cell surface layer protein(s) is/are reduced. Specifically, the bacterium may be a bacterium in which the activity(s) of cell surface layer protein(s) is/are reduced as compared with a non-modified strain. The phrase "the activity of a cell surface layer protein is reduced" may particularly mean that the number of molecules of the cell surface layer protein per cell is reduced. Hereinafter, the cell surface layer proteins and genes encoding them will be explained.

The cell surface layer protein is a protein constituting the surface layer (S layer) of bacteria or archaea. Examples of cell surface layer proteins of coryneform bacteria include PS1 and PS2 (CspB) of *C*. *glutamicum* (Japanese Patent Laid-open (Kohyo) No. 6-502548), and SlpA (CspA) of *C*. *stationis* (Japanese Patent Laid-open (Kokai) No. 10-108675). It is preferable to reduce the activity of the PS2 protein among these.

The nucleotide sequence of the cspB gene of *C*. *glutamicum* ATCC 13869 and the amino acid sequence of the PS2 protein (CspB protein) encoded by the gene are shown in SEQ ID NOS: 10 and 11, respectively.

Furthermore, for example, amino acid sequences of CspB homologues were reported for 28 strains of *C .glutamicum* (J. Biotechnol., 112, 177-193 (2004)). These 28 strains of *C*. *glutamicum* and the GenBank accession numbers of the cspB gene homologues in NCBI database are exemplified below (the GenBank accession numbers are shown in the parentheses).
*C. glutamicum* ATCC 13058 (AY524990)
*C. glutamicum* ATCC 13744 (AY524991)
*C. glutamicum* ATCC 13745 (AY524992)
*C. glutamicum* ATCC 14017 (AY524993)
*C. glutamicum* ATCC 14020 (AY525009)
*C. glutamicum* ATCC 14067 (AY524994)
*C. glutamicum* ATCC 14068 (AY525010)
*C. glutamicum* ATCC 14747 (AY525011)
*C. glutamicum* ATCC 14751 (AY524995)
*C. glutamicum* ATCC 14752 (AY524996)
*C. glutamicum* ATCC 14915 (AY524997)
*C. glutamicum* ATCC 15243 (AY524998)
*C. glutamicum* ATCC 15354 (AY524999)
*C. glutamicum* ATCC 17965 (AY525000)
*C. glutamicum* ATCC 17966 (AY525001)
*C. glutamicum* ATCC 19223 (AY525002)
*C. glutamicum* ATCC 19240 (AY525012)
*C. glutamicum* ATCC 21341 (AY525003)
*C.* glutamicum ATCC 21645 (AY525004)
*C. glutamicum* ATCC 31808 (AY525013)
*C. glutamicum* ATCC 31830 (AY525007)
*C. glutamicum* ATCC 31832 (AY525008)
*C. glutamicum* LP-6 (AY525014)
*C. glutamicum* DSM20137 (AY525015)
*C. glutamicum* DSM20598 (AY525016)
*C. glutamicum* DSM46307 (AY525017)
*C. glutamicum* 22220 (AY525005)
*C. glutamicum* 22243 (AY525006)

Since the nucleotide sequence of a gene encoding a cell surface layer protein may differ depending on species or strain to which the coryneform bacterium belongs, the gene encoding a cell surface layer protein may be a variant of any of genes encoding the cell surface layer proteins exemplified above, so long as the original function thereof is maintained. Similarly, the cell surface layer protein may be a variant of any of the cell surface layer proteins exemplified above, so long as the original function thereof is maintained. That is, the term "cspB gene" includes not only the cspB genes exemplified above, but also includes conservative variants thereof. Similarly, the term "CspB protein" includes not only the CspB proteins exemplified above, but also includes conservative variants thereof. The aforementioned descriptions concerning conservative variants of the ncAA-aaRS gene and ncAA-aaRS can be similarly applied to variants of the cell surface layer protein and the gene encoding it. For example, the gene encoding the cell surface layer protein may be a gene encoding a protein having the aforementioned amino acid sequence, but including substitution, deletion, insertion, and/or addition of one or several amino acid residues at one or several positions, so long as the original function is maintained. Also, for example, the gene encoding the cell surface layer protein may also be a gene encoding a protein having an amino acid sequence showing an identity of 80% or more, 90% or more, 95% or more, 97% or more, or 99% or more, to the total amino acid sequence of any of the aforementioned amino acid sequences, so long as the original function is maintained. Incidentally, the expression "original function is maintained" used for the cell surface layer protein may mean that the protein has a property that if the activity of the protein is reduced in a coryneform bacterium, the secretory production amount of a ncAA-containing protein is increased compared with that obtainable with a non-modified strain.

The "property that if the activity of the protein is reduced in a coryneform bacterium, the secretory production amount of a ncAA-containing protein is increased compared with that obtainable with a non-modified strain" refers to a property imparting an ability to produce a ncAA-containing protein by secretory production in an amount larger than that obtainable with a non-modified strain to a coryneform bacterium when the activity thereof is reduced in the coryneform bacterium. The "non-modified strain" refers to a control strain of which the activity(s) of cell surface layer protein(s) is/are not reduced, and it may be, for example, a wildtype strain or a parent strain. Although the degree of increase meant by the expression "to produce a ncAA-containing protein by secretory production in an amount larger than that obtainable with a non-modified strain" is not particularly limited so long as the secretory production amount of the ncAA-containing protein is increased compared with that obtainable with a non-modified strain, the expression may mean that the ncAA-containing protein is produced by secretory production in an amount of, for example, 1.1 times or more, 1.2 times or more, 1.3 times or more, or 2 times or more, of that obtainable with a non-modified strain, in terms of the accumulation amount in the medium and/or on the cell surface layer. In addition, the expression "to produce a ncAA-containing protein by secretory production in an amount larger than that obtainable with a non-modified strain" may also mean that whereas the ncAA-containing protein cannot be detected when a non-concentrated culture supernatant of a non-modified strain is applied to SDS-PAGE and stained with CBB, the ncAA-containing protein can be detected when a non-concentrated culture supernatant of a modified strain is applied to SDS-PAGE and stained with CBB.

Whether the activity of the protein is reduced in a coryneform bacterium, the secretory production amount of a ncAA-containing protein is increased compared with that obtainable with a non-modified strain can be confirmed by preparing a strain modified so that the activity of the protein is reduced from a strain belonging to the coryneform bacteria, quantifying the secretory production amount of the ncAA-containing protein observed when the modified strain is cultured in a medium, and comparing the quantified amount with the secretory production amount of the ncAA-containing protein observed before being modified (un-modified strain) is cultured in the medium.

The expression "activity of a cell surface layer protein is reduced" includes when a coryneform bacterium has been modified so that the activity of a cell surface layer protein is reduced and a case where the activity of a cell surface layer protein is inherently reduced in a coryneform bacterium. The "case where activity of a cell surface layer protein is inherently reduced in a coryneform bacterium" includes when a coryneform bacterium is inherently deficient in a cell surface layer protein. That is, examples of a coryneform bacterium in which the activity of a cell surface layer protein is reduced include a coryneform bacterium that is inherently deficient in a cell surface layer protein. Examples of the "case where a coryneform bacterium is inherently deficient in a cell surface layer protein" include a case where a coryneform bacterium is inherently deficient in the gene encoding a cell surface layer protein. The expression "a coryneform bacterium is inherently deficient in a cell surface layer protein" may mean that a coryneform bacterium is inherently deficient in one or more proteins selected from cell surface layer protein(s) found in other strain(s) of the species to which the coryneform bacterium belongs. For example, "C. *glutamicum* is inherently deficient in a cell surface layer protein" may mean that a C. *glutamicum* strain is inherently deficient in one or more proteins selected from cell surface layer protein(s) found in other C. *glutamicum* strain(s), i.e., for example, deficient in PS1 and/or PS2 (CspB). Examples of the coryneform bacterium that is inherently deficient in a cell surface layer protein include *C. glutamicum* ATCC 13032, which is inherently deficient in the cspB gene.

### <1-4-3> Protein Secretion System

The bacterium has a protein secretion system. The bacterium may inherently have the protein secretion system. The protein secretion system is not particularly limited so long as it can secrete a ncAA-containing protein. Examples of the protein secretion system include the Sec secretion system and the Tat secretion system. The bacterium may be modified so that the activity of the protein secretion system (e.g., Tat secretion system) is increased. Specifically, the bacterium may be modified so that the activity of the protein secretion system (e.g., Tat secretion system) is increased as compared with a non-modified strain. The activity of the Tat secretion system can be increased by, for example, increasing the expression of one or more genes encoding the Tat secretion system. That is, more specifically, the bacterium may be modified so that the expression of one or more genes encoding the Tat secretion system is increased. The increased activity of the Tat secretion system is preferred, particularly when the Tat-dependent signal peptide is used for secretory production of the ncAA-containing protein. Methods for increasing the expression of genes encoding the Tat secretion system are described in Japanese Patent No. 4730302.

Examples of the genes encoding the Tat secretion system include tatA, tatB, tatC, and tatE genes.

Specific examples of the genes encoding the Tat secretion system include tatA, tatB, and tatC genes of C. *glutamicum.* The tatA, tatB, and tatC genes of C. *glutamicum* ATCC 13032 correspond to the complementary sequence of positions 1571065-1571382, the sequence of positions 1167110-1167580, and the complementary sequence of positions 1569929-1570873 in the genome sequence registered as GenBank accession NC_003450 (VERSION NC_003450.3 GI:58036263) in NCBI database, respectively. The TatA, TatB, and TatC proteins of *C*. *glutamicum* ATCC 13032 have been registered as GenBank accession NP_600707 (version NP_600707.1 GI:19552705, locus_tag="NCg11434"), GenBank accession NP_600350 (version NP_600350.1 GI:19552348, locus_tag="NCg11077"), and GenBank accession NP_600706 (version NP_600706.1 GI:19552704, locus _tag="NCg11433"), respectively. The nucleotide sequences of the tatA, tatB, and tatC genes of *C*. *glutamicum* ATCC 13032 and the amino acid sequences of the TatA, TatB, and TatC proteins of the same are shown in SEQ ID NOS: 12 to 17.

Specific examples of the genes encoding the Tat secretion system also include tatA, tatB, tatC, and tatE genes of *E. coli.* The tatA, tatB, tatC, and tatE genes of *E. coli* K-12 MG1655 correspond to the sequence of positions 4019968-4020237, the sequence of positions 4020241-4020756, the sequence of positions 4020759-4021535, and the sequence of positions 658170-658373 in the genome sequence registered as GenBank accession NC_000913(VERSION NC_000913.2 GI:49175990) in NCBI database, respectively. The TatA, TatB, TatC, and TatE proteins of *E. coli* K-12 MG1655 have been registered as GenBank accession NP_418280 (version NP_418280.4 GI:90111653, locus_tag="b3836"), GenBank accession YP_026270 (version YP_026270.1 GI:49176428, locus_tag="b3838"), GenBank accession NP_418282 (version NP_418282.1 GI:16131687, locus_tag="b3839"), and GenBank accession NP_415160 (version NP_415160.1 GI:16128610, locus_tag="b0627"), respectively.

The gene encoding the Tat secretion system may be a variant of any of the genes encoding the Tat-secretion-system exemplified above, so long as the original function thereof is maintained. Similarly, the Tat-secretion-system may be a variant of any of the Tat-secretion-systems exemplified above, so long as the original function thereof is maintained. That is, the terms "tatA gene", "tatB gene", "tatC gene", and "tatE gene" include not only the tatA, tatB, tatC, and tatE genes exemplified above, respectively, but also includes conservative variants thereof. Similarly, the terms "TatA protein", "TatB protein", "TatC protein", and "TatE protein" include not only the TatA, TatB, TatC, and TatE proteins exemplified above, respectively, but also includes conservative variants thereof. The aforementioned descriptions concerning conservative variants of the ncAA-aaRS gene and ncAA-aaRS can be similarly applied to variants of the Tat-secretion-system and the gene encoding it. For example, the gene encoding the Tat-secretion-system may have any of the aforementioned amino acid sequences, but include substitution, deletion, insertion, and/or addition of one or several amino acid residues at one or several positions, so long as the original function is maintained. Also, for example, the gene encoding the Tat-secretion-system may also be a gene encoding a protein having an amino acid sequence showing an identity of 80% or more, 90% or more, 95% or more, 97% or more, or 99% or more, to the total amino acid sequence of any of the aforementioned amino acid sequences, so long as the original function is maintained. Incidentally, the expression "original function is maintained" in reference to the Tat-secretion-system may mean that the system has a function of secreting a protein fused with a Tat-dependent signal peptide at the N-terminus out of the cell.

An increase in the activity of the Tat secretion system can be confirmed by, for example, confirming an increase in the secretory production amount of a protein fused with a Tat-dependent signal peptide at the N-terminus. The secretory production amount of the protein fused with a Tat-dependent signal peptide at the N-terminus may be increased to, for example, 1.5 times or more, 2 times or more, or 3 times or more, of that of a non-modified strain.

### <1-5> Methods for Increasing Activity of Protein

Hereinafter, the methods for increasing the activity of a protein, including the methods for increasing the expression of a gene, will be explained.

The expression "the activity of a protein is increased" means that the activity of the protein is increased as compared with a non-modified strain. Specifically, the expression "the activity of a protein is increased" means that the activity of the protein per cell is increased as compared with that of a non-modified strain. The term "non-modified strain" refers to a control strain that has not been modified so that the activity of an objective protein is increased. Examples of the non-modified strain include a wildtype strain and parent strain. Specific examples of the non-modified strain include the respective type strains of the species of bacteria. Specific examples of the non-modified strain also include strains exemplified above in relation to the description of coryneform bacteria. That is, in an embodiment, the activity of a protein may be increased as compared with a type strain, i.e., the type strain of the species to which the chosen bacterium belongs. In another embodiment, the activity of a protein may also be increased as compared with C. *glutamicum* ATCC 13869. In another embodiment, the activity of a protein may also be increased as compared with *C. glutamicum* ATCC 13032. In another embodiment, the activity of a protein may also be increased as compared with *C. glutamicum* AJ12036 (FERM BP-734). In another embodiment, the activity of a protein may also be increased as compared with *C. glutamicum* YDK010. The state that "the activity of a protein is increased" may also be expressed as "the activity of a protein is enhanced". More specifically, the expression "the activity of a protein is increased" may mean that the number of molecules of the protein per cell is increased, and/or the function of each molecule of the protein is increased as compared with those of a non-modified strain. That is, the term "activity" in the expression "the activity of a protein is increased" is not limited to the catalytic activity of the protein, but may also mean the transcription amount of a gene (i.e., the amount of mRNA) encoding the protein, or the translation amount of the gene (i.e., the amount of the protein). The term "the number of molecules of a protein per cell" may mean an average value of the number of molecules of the protein per cell. Furthermore, the expression that "the activity of a protein is increased" can mean that the activity of an objective protein is increased in a strain inherently having the activity of the objective protein, and also that the activity of an objective protein is imparted to a strain not inherently having the activity of the objective protein. Furthermore, so long as the activity of the protein is eventually increased, the activity of an objective protein inherently contained in a host may be attenuated and/or eliminated, and then an appropriate type of the objective protein may be imparted to the host.

The degree of the increase in the activity of a protein is not particularly limited, so long as the activity of the protein is increased as compared with a non-modified strain. The activity of the protein may be increased to, for example, 1.5 times or more, 2 times or more, or 3 times or more of that of a non-modified strain. Furthermore, when the non-modified strain does not have the activity of the objective protein, it is sufficient that the protein is produced as a result of introduction of the gene encoding the protein, and for example, the protein may be produced to such an extent that the activity thereof can be measured.

The modification for increasing the activity of a protein can be attained by, for example, increasing the expression of a gene encoding the protein. The expression "the expression of a gene is increased" means that the expression of the gene is increased as compared with a non-modified strain such as a wildtype strain and parent strain. Specifically, the expression "the expression of a gene is increased" means that the expression amount of the gene per cell is increased as compared with that of a non-modified strain. The term "the expression amount of a gene per cell" may mean an average value of the expression amount of the gene per cell. More specifically, the expression "the expression of a gene is increased" may mean that the transcription amount of the gene (i.e., the amount of mRNA) is increased, and/or the translation amount of the gene (i.e., the amount of the protein expressed from the gene) is increased. The state that "the expression of a gene is increased" may also be referred to as "the expression of a gene is enhanced". The expression of a gene may be increased to, for example, 1.5 times or more, 2 times or more, or 3 times or more of that of a non-modified strain. Furthermore, the expression "the expression of a gene is increased" can mean not only that the expression amount of an objective gene is increased in a strain that inherently expresses the objective gene, but also that the gene is introduced into a strain that does not inherently express the objective gene, and expressed therein. That is, the phrase "the expression of a gene is increased" may also mean, for example, that an objective gene is introduced into a strain that does not possess the gene, and is expressed therein.

The expression of a gene can be increased by, for example, increasing the copy number of the gene.

The copy number of a gene can be increased by introducing the gene into the chromosome of a host. A gene can be introduced into a chromosome by, for example, using homologous recombination (Miller, J. H., Experiments in Molecular Genetics, 1972, Cold Spring Harbor Laboratory). Examples of the gene transfer method utilizing homologous recombination include, for example, a method of using a linear DNA such as Red-driven integration (Datsenko, K. A., and Wanner, B. L., Proc. Natl. Acad. Sci. USA, 97:6640- 6645 (2000)), a method of using a plasmid containing a temperature sensitive replication origin, a method of using a plasmid capable of conjugative transfer, a method of using a suicide vector not having a replication origin that functions in a host, and a transduction method using a phage. Specifically, a host can be transformed with a recombinant DNA containing an objective gene, so that homologous recombination occurs at a target region on the chromosome of the host, thereby introducing the objective gene into the chromosome of the host. The structure of the recombinant DNA to be used for homologous recombination is not particularly limited as long as it causes homologous recombination in a desired manner. For example, a host can be transformed with a linear DNA containing an objective gene and further containing nucleotide sequences homologous to upstream and downstream of a target region on the chromosome at the respective ends of the objective gene, so that homologous recombination occurs at each of upstream and downstream of the target region, thereby replacing the target region with the objective gene. The recombinant DNA to be used for homologous recombination may contain a marker gene for selection of transformants. Only one copy, or two or more copies of a gene may be introduced. For example, by performing homologous recombination using a nucleotide sequence which is present in multiple copies on a chromosome as a target, multiple copies of a gene can be introduced into the chromosome. Examples of such a sequence which is present in multiple copies on a chromosome include repetitive DNAs, and inverted repeats located at both ends of a transposon. Alternatively, homologous recombination may be performed by using an appropriate sequence on a chromosome such as a gene unnecessary for production of an objective substance as a target. Furthermore, a gene can also be randomly introduced into a chromosome by using a transposon or Mini-Mu (Japanese Patent Laid-open (Kokai) No. 2-109985, U.S. Pat. No. 5,882,888, EP805867B1). Such methods for modifying a chromosome using homologous recombination can be used for any modification on a chromosome, such as a modification of an expression control sequence, as well as for introduction of an objective gene.

Introduction of an objective gene into a chromosome can be confirmed by Southern hybridization using a probe having a sequence complementary to the whole gene or a part thereof, PCR using primers prepared on the basis of the sequence of the gene, etc.

Furthermore, the copy number of a gene can also be increased by introducing a vector containing the gene into a host. For example, the copy number of a target gene can be increased by linking a DNA fragment containing the target gene with a vector that functions in a host to construct an expression vector of the gene, and transforming the host with the expression vector. The DNA fragment containing the target gene can be obtained by, for example, PCR using the genomic DNA of a microorganism having the target gene as the template. As the vector, a vector autonomously replicable in the cell of the host can be used. The vector can be a multi-copy vector. Furthermore, the vector can have a marker such as an antibiotic resistance gene for selection of transformant. Furthermore, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, etc. Specific examples of vector autonomously replicable in coryneform bacteria include, for example, pHM1519 (Agric. Biol. Chem., 48, 2901-2903 (1984)); pAM330 (Agric. Biol. Chem., 48, 2901-2903 (1984)); plasmids obtained by improving these and having a drug resistance gene; plasmid pCRY30 (Japanese Patent Laid-open (Kokai) No. 3-210184); plasmids pCRY21, pCRY2KE, pCRY2KX, pCRY31, pCRY3KE, and pCRY3KX (Japanese Patent Laid-open (Kokai) No. 2-72876 and U.S. Pat. No. 5,185,262); plasmids pCRY2 and pCRY3 (Japanese Patent Laid-open (Kokai) No. 1-191686); pAJ655, pAJ611, and pAJ1844 (Japanese Patent Laid-open (Kokai) No. 58-192900); pCG1 (Japanese Patent Laid-open (Kokai) No. 57-134500); pCG2 (Japanese Patent Laid-open (Kokai) No. 58-35197); pCG4 and pCG11 (Japanese Patent Laid-open (Kokai) No. 57-183799); pVK7 (Japanese Patent Laid-open (Kokai) No. 10-215883); pVK9 (US2006-0141588); pVC7 (Japanese Patent Laid-open (Kokai) No. 9-070291); pVS7 (WO2013/069634); pPK4 (Japanese Patent Laid-open (Kokai) No. 9-322774); pPK5(WO2018/074579). Specific examples of vector autonomously replicable in coryneform bacteria also include, for example: pVC7 variants such as pVC7N (Shuhei Hashiro et al., High copy number mutants derived from Corynebacterium glutamicum cryptic plasmid pAM330 and copy number control, J Biosci Bioeng, 2019 May;127(5):529-538.); and pVC7H1, pVC7H2, pVC7H3, pVC7H4, pVC7H5, pVC7H6, and pVC7H7 (WO2018/179834). Specific examples of vector autonomously replicable in coryneform bacteria also include, for example, pPK4 variants such as pPK4H1, pPK4H2, pPK4H3, pPK4H4, pPK4H5, and pPK4H6 (WO2018/179834). Particular examples of the vector include pVC vectors and pPK vectors. Examples of pVC vectors include pVC7 and a variant thereof, vectors in which their antibiotic resistance genes are substituted with different antibiotic resistance genes, and vectors showing a nucleotide sequence identity of 90% or more, 95% or more, 97% or more, or 99% or more thereto. Examples of pPK vectors include pPK4, pPK5, and variants thereof, vectors in which their antibiotic resistance genes are substituted with different antibiotic resistance genes, and vectors showing a nucleotide sequence identity of 90% or more, 95% or more, 97% or more, or 99% or more thereto. Further, particular examples of the vector include pVC7, pVC7N, pPK4, and pPK5.

When a gene is introduced, it is sufficient that the gene is expressible by the host. Specifically, it is sufficient that the gene is harbored by a host so that it is expressed under control by a promoter that functions in the host. The promoter is not particularly limited so long as it functions in the host. The phrase "promoter that functions in a host" refers to a promoter that shows a promoter activity in the host. The promoter may be a promoter derived from the host, or a heterogenous promoter. The promoter may be the native promoter of the gene to be introduced, or a promoter of another gene. The promoter may be inducible or constitutive for gene expression.

Examples of promoters usable in coryneform bacteria include, for example, promoters of genes for glycolysis, pentose phosphate pathway, TCA cycle, amino acid biosynthesis system, and cell surface layer proteins. Specific examples of the promoters of amino acid biosynthesis system genes include, for example, promoters of the glutamate dehydrogenase gene of the glutamic acid biosynthesis system, the glutamine synthetase gene of the glutamine synthesis system, the aspartokinase gene of the lysine biosynthesis system, the homoserine dehydrogenase gene of the threonine biosynthesis system, the acetohydroxy acid synthetase gene of the isoleucine and valine biosynthesis system, 2-isopropylmalate synthetase gene of the leucine biosynthesis system, the glutamate kinase gene of the proline and arginine biosynthesis system, the phosphoribosyl-ATP pyrophosphorylase gene of the histidine biosynthesis system, the deoxyarabinoheptulonate phosphate (DAHP) synthetase gene of the aromatic amino acid biosynthesis systems such as those for tryptophan, tyrosine, and phenylalanine, the phosphoribosyl pyrophosphate (PRPP) amidotransferase gene of the nucleic acid biosynthesis systems such as those for inosinic acid and guanylic acid, the inosinic acid dehydrogenase gene, and the guanylic acid synthetase gene. In addition, examples of the promoters usable in coryneform bacteria include stronger promoters, as described below.

A terminator for termination of gene transcription may be located downstream of the gene. The terminator is not particularly limited so long as it functions in the host. The terminator may be a terminator derived from the host, or a heterogenous terminator. The terminator may be the native terminator of the gene to be introduced, or a terminator of another gene. Specific examples of the terminator include the terminator of bacteriophage BFK20.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

Furthermore, when two or more genes are introduced, it is sufficient that the genes each are expressible by the host. For example, two or more genes may be carried by a single expression vector or a chromosome. Furthermore, the two or more genes may be separately carried by two or more expression vectors, or separately carried by a single or two or more expression vectors and a chromosome. An operon having two or more genes may also be introduced. For example, the tRNA (ncAA) gene and the ncAA-aaRS gene may or may not be carried by a single expression vector. Also, for example, the tRNA (ncAA) gene and the ncAA-containing protein gene may or may not be carried by a single expression vector. Also, for example, the ncAA-aaRS gene and the ncAA-containing protein gene may or may not be carried by a single expression vector. Also, for example, the tRNA (ncAA) gene, the ncAA-aaRS gene, and the ncAA-containing protein gene may or may not be carried by a single expression vector. Also, for example, a plurality of copies of the tRNA (ncAA) gene may be carried by a plurality of expression vectors respectively. Also, for example, a plurality of copies of the ncAA-aaRS gene may be carried by a plurality of expression vectors respectively. Also, for example, a plurality of copies of the ncAA-containing protein gene may be carried by a plurality of expression vectors respectively.

That is, the bacterium as described herein may have, for example, a single expression vector carrying the ncAA-containing protein gene (specifically a genetic construct for secretory expression), the tRNA (ncAA) gene, and the ncAA-aaRS gene. The single expression vector may be, for example, a pPK vector such as pPK4 or pPK5.

In addition, the bacterium as described herein may have, for example, a first expression vector carrying the ncAA-containing protein gene (specifically a genetic construct for secretory expression) and a second expression vector carrying the tRNA (ncAA) gene and the ncAA-aaRS gene. The first expression vector may further carry the tRNA (ncAA) gene and/or the ncAA-aaRS gene. The first expression vector may be, for example, a pPK vector such as pPK4 or pPK5. The second expression vector may be, for example, a pVC vector such as pVC7 or pVC7N.

The gene to be introduced is not particularly limited so long as it encodes a protein that functions in the host. The gene to be introduced may be a gene derived from or native to the host, or may be a heterogenous gene. The gene to be introduced can be obtained by, for example, PCR using primers designed on the basis of the nucleotide sequence of the gene, and using the genomic DNA of an organism having the gene, a plasmid carrying the gene, etc. as a template. The gene to be introduced may also be totally synthesized, for example, on the basis of the nucleotide sequence of the gene (Gene, 60(1), 115-127 (1987)). The obtained gene can be used as it is, or after being modified as required. That is, a gene can be modified to obtain a variant thereof. A gene can be modified by a known technique. For example, an objective mutation can be introduced into an objective site of DNA by the site-specific mutation method. That is, the coding region of a gene can be modified by the site-specific mutation method so that a specific site of the encoded protein include substitution, deletion, insertion, and/or addition of amino acid residues. Examples of the site-specific mutation method include the method utilizing PCR (Higuchi, R., 61, in PCR Technology, Erlich, H. A. Eds., Stockton Press (1989); Carter, P, Meth. in Enzymol., 154, 382 (1987)), and the method utilizing phage (Kramer, W and Frits, H. J., Meth. in Enzymol., 154, 350 (1987); Kunkel, T. A. et al., Meth. in Enzymol., 154, 367 (1987)). Alternatively, a variant of a gene may be totally synthesized.

Incidentally, when a protein functions as a complex made up of a plurality of subunits, some or all of the subunits may be modified, so long as the activity of the protein is eventually increased. That is, for example, when the activity of a protein is increased by increasing the expression of a gene, the expression of some or all of the genes that encode the subunits may be enhanced. It is usually preferable to enhance the expression of all of genes encoding the subunits. Furthermore, the subunits constituting the complex may be derived from or native to a single kind of organism or two or more kinds of organisms, so long as the complex has a function of the objective protein. That is, for example, genes of the same organism encoding a plurality of subunits may be introduced into a host, or genes of different organisms encoding a plurality of subunits may be introduced into a host.

Furthermore, the expression of a gene can be increased by improving the transcription efficiency of the gene. In addition, the expression of a gene can also be increased by improving the translation efficiency of the gene. The transcription efficiency of the gene and the translation efficiency of the gene can be improved by, for example, modifying an expression control sequence of the gene. The phrase "expression control sequence" collectively refers to sites that affect the expression of a gene. Examples of the expression control sequence include, for example, promoter, Shine-Dalgarno (SD) sequence (also referred to as ribosome binding site (RBS)), and spacer region between RBS and the start codon. Expression control sequences can be identified by using a promoter search vector or gene analysis software such as GENETYX. These expression control sequences can be modified by, for example, a method of using a temperature sensitive vector, or the Red driven integration method (WO2005/010175).

The transcription efficiency of a gene can be improved by, for example, replacing the promoter of the gene on a chromosome with a stronger promoter. The term "stronger promoter" refers to a promoter providing improved transcription of a gene compared with an inherent wildtype promoter of the gene. Examples of stronger promoters usable in coryneform bacteria include, for example, the artificially modified P54-6 promoter (Appl. Microbiol. Biotechnol., 53, 674- 679 (2000)), pta, aceA, aceB, adh, and amyE promoters inducible with acetic acid, ethanol, pyruvic acid, etc., cspB, SOD, and tuf (EF-Tu) promoters, which are other strong promoters (Journal of Biotechnology, 104 (2003) 311- 323; Appl. Environ. Microbiol., 2005 December; 71 (12):8587- 96), as well as lac promoter, tac promoter, trc promoter, F1 promoter, T7 promoter, T5 promoter, T3 promoter, and SP6 promoter. Furthermore, as the stronger promoter, a highly-active type of an existing promoter may also be obtained by using various reporter genes. For example, by making the -35 and -10 regions in a promoter region closer to the consensus sequence, the activity of the promoter can be enhanced (WO00/18935). Examples of highly active-type promoter include various tac-like promoters (Katashkina J I et al., Russian Federation Patent Application No. 2006134574). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

The translation efficiency of a gene can be improved by, for example, replacing the Shine-Dalgarno (SD) sequence (also referred to as ribosome binding site (RBS)) for the gene on a chromosome with a stronger SD sequence. The "stronger SD sequence" means a SD sequence that provides an improved translation of mRNA compared with the inherent wildtype SD sequence of the gene. Examples of stronger SD sequences include, for example, RBS of the gene 10 derived from phage T7 (Olins P. O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and hence, the translation efficiency of a gene can also be improved by modifying them.

The translation efficiency of a gene can also be improved by, for example, modifying codons. For example, the translation efficiency of the gene can be improved by replacing a rare codon present in the gene with a synonymous codon more frequently used. That is, the gene to be introduced may be modified, for example, so as to have optimal codons according to the codon usage frequency of a host to be used. Codons can be replaced by, for example, the site-specific mutation method. Alternatively, a gene fragment in which objective codons are replaced may be totally synthesized. Frequencies of codons in various organisms are disclosed in the "Codon Usage Database" (kazusa.or.jp/codon; Nakamura, Y. et al, Nucl. Acids Res., 28, 292 (2000)).

Furthermore, the expression of a gene can also be increased by amplifying a regulator that increases the expression of the gene, or deleting or attenuating a regulator that reduces the expression of the gene.

Such methods for increasing the gene expression as mentioned above may be used independently or in any appropriate combination.

Furthermore, the modification that increases the activity of a protein can also be attained by, for example, enhancing the specific activity of the protein. A protein showing an enhanced specific activity can be obtained by, for example, searching various organisms. Furthermore, a highly-active type of an existing protein may also be obtained by introducing a mutation into the existing protein. The mutation to be introduced may be, for example, substitution, deletion, insertion, and/or addition of one or several amino acid residues at one or several position of the protein. The mutation can be introduced by, for example, such a site-specific mutation method as mentioned above. The mutation may also be introduced by, for example, a mutagenesis treatment. Examples of the mutagenesis treatment include irradiation of X-ray or ultraviolet and treatment with a mutation agent such as N-methyl-N'-nitro-N-nitrosoguanidine (MNNG), ethyl methanesulfonate (EMS), and methyl methanesulfonate (MMS). Furthermore, a random mutation may be induced by directly treating DNA in vitro with hydroxylamine. Enhancement of the specific activity may be independently used, or may be used in any appropriate combination with such methods for enhancing gene expression as mentioned above.

The method for the transformation is not particularly limited, and conventionally known methods can be used. There can be used, for example, a method of treating recipient cells with calcium chloride so as to increase the permeability thereof for DNA, which has been reported for the Escherichia coli K-12 strain (Mandel, M. and Higa, A., J. Mol. Biol., 1970, 53, 159-162), and a method of preparing competent cells from cells which are in the growth phase, followed by transformation with DNA, which has been reported for *Bacillus subtilis* (Duncan, C. H., Wilson, G. A. and Young, F. E., Gene, 1977, 1:153-167). Alternatively, there can also be used a method of making DNA-recipient cells into protoplasts or spheroplasts, which can easily take up recombinant DNA, followed by introducing a recombinant DNA into the DNA-recipient cells, which is known to be applicable to *Bacillus subtilis*, actinomycetes, and yeasts (Chang, S. and Choen, S. N., 1979, Mol. Gen. Genet., 168:111- 115; Bibb, M. J., Ward, J. M. and Hopwood, O. A., 1978, Nature, 274:398- 400; Hinnen, A., Hicks, J. B. and Fink, G. R., 1978, Proc. Natl. Acad. Sci. USA, 75:1929- 1933). Furthermore, the electric pulse method reported for coryneform bacteria (Japanese Patent Laid-open (Kokai) No. 2-207791) can also be used.

An increase in the activity of a protein can be confirmed by measuring the activity of the protein.

An increase in the activity of a protein can also be confirmed by confirming an increase in the expression of a gene encoding the protein. An increase in the expression of a gene can be confirmed by confirming an increase in the transcription amount of the gene, or by confirming an increase in the amount of a protein expressed from the gene.

An increase of the transcription amount of a gene can be confirmed by comparing the amount of mRNA transcribed from the gene with that of a non-modified strain such as a wildtype strain or parent strain. Examples of the method for evaluating the amount of mRNA include Northern hybridization, RT-PCR, microarray, RNA-seq, and so forth (Sambrook, J., et al., Molecular Cloning A Laboratory Manual/Third Edition, Cold Spring Harbor Laboratory Press, Cold Spring Harbor (USA), 2001). The amount of mRNA (such as the number of molecules of the mRNA per cell) may be increased to, for example, 1.5 times or more, 2 times or more, or 3 times or more of that of a non-modified strain.

An increase in the amount of a protein can be confirmed by Western blotting using antibodies (Sambrook, J., et al., Molecular Cloning A Laboratory Manual/Third Edition, Cold Spring Harbor Laboratory Press, Cold Spring Harbor (USA), 2001). The amount of the protein (such as the number of molecules of the protein per cell) may be increased to, for example, 1.5 times or more, 2 times or more, or 3 times or more of that of a non-modified strain.

The aforementioned methods for increasing the activity of a protein can be used for enhancement of the activities of any proteins and enhancement of the expression of any genes.

### <1-6> Method for Reducing Activity of Protein

Hereinafter, methods for reducing the activity of a protein will be explained. The methods for reducing the activity of a protein described below can also be utilized for disruption of the wildtype PhoS protein.

The expression "the activity of a protein is reduced" means that the activity of the protein is reduced as compared with a non-modified strain. Specifically, the expression "the activity of a protein is reduced" means that the activity of the protein per cell is reduced as compared with that of a non-modified strain. The term "non-modified strain" used herein refers to a control strain that has not been modified so that the activity of an objective protein is reduced. Examples of the non-modified strain include a wildtype strain and parent strain. Specific examples of the non-modified strain include the respective type strains of the species of bacteria. Specific examples of the non-modified strain also include strains exemplified above in relation to the description of coryneform bacteria. That is, in an embodiment, the activity of a protein may be reduced as compared with a similar strain, i.e., the same or similar strain of the species to which the bacterium belongs. In another embodiment, the activity of a protein may also be reduced as compared with *C*. *glutamicum* ATCC 13032. In another embodiment, the activity of a protein may also be reduced as compared with *C. glutamicum* ATCC 13869. In another embodiment, the activity of a protein may also be reduced as compared with *C*. *glutamicum* AJ12036 (FERM BP-734). In another embodiment, the activity of a protein may also be reduced as compared with *C*. *glutamicum* YDK010. The state that "the activity of a protein is reduced" also includes a state that the activity of the protein has completely disappeared. More specifically, the expression "the activity of a protein is reduced" may mean that the number of molecules of the protein per cell is reduced, and/or the function of each molecule of the protein is reduced as compared with those of a non-modified strain. That is, the term "activity" in the expression "the activity of a protein is reduced" is not limited to the catalytic activity of the protein, but may also mean the transcription amount of a gene (i.e., the amount of mRNA) encoding the protein or the translation amount of the gene (i.e., the amount of the protein). The phrase "the number of molecules of a protein per cell" may mean an average value per cell of the number of molecules of the protein. The state that "the number of molecules of the protein per cell is reduced" also includes a state that the protein does not exist at all. The state that "the function of each molecule of the protein is reduced" also includes a state that the function of each protein molecule has completely disappeared. The degree of the reduction in the activity of a protein is not particularly limited, so long as the activity is reduced as compared with that of a non-modified strain. The activity of a protein may be reduced to, for example, 50% or less, 20% or less, 10% or less, 5% or less, or 0% of that of a non-modified strain.

The modification for reducing the activity of a protein can be attained by, for example, reducing the expression of a gene encoding the protein. The expression "the expression of a gene is reduced" means that the expression of the gene is reduced as compared with a non-modified strain. Specifically, the expression "the expression of a gene is reduced" means that the expression amount of the gene per cell is reduced as compared with that of a non-modified strain. The phrase "the expression amount of a gene per cell" may mean an average value per cell of the expression amount of the gene. More specifically, the expression "the expression of a gene is reduced" may mean that the transcription amount of the gene (i.e., the amount of mRNA) is reduced, and/or the translation amount of the gene (i.e., the amount of the protein expressed from the gene) is reduced. The phrase that "the expression of a gene is reduced" also includes when the gene is not expressed at all. The phrase that "the expression of a gene is reduced" can also mean "the expression of a gene is attenuated". The expression of a gene may be reduced to, for example, 50% or less, 20% or less, 10% or less, 5% or less, or 0% of that of a non-modified strain.

The reduction in gene expression may be due to, for example, a reduction in the transcription efficiency, a reduction in the translation efficiency, or a combination of them. The expression of a gene can be reduced by modifying an expression control sequence of the gene. The term "expression control sequence" collectively refers to sites that affect the expression of a gene, such as a promoter, Shine-Dalgarno (SD) sequence (also referred to as ribosome-binding site (RBS)), and spacer region between RBS and the start codon. Expression control sequences can be identified by, for example, using a promoter search vector or gene analysis software such as GENETYX. When an expression control sequence is modified, one or more nucleotides, two or more nucleotides, three or more nucleotides of the expression control sequence are modified. The transcription efficiency of a gene can be reduced by, for example, replacing the promoter of the gene on a chromosome with a weaker promoter. The term "weaker promoter" means a promoter providing an attenuated transcription of a gene compared with an inherently existing wildtype promoter of the gene. Examples of weaker promoters include, for example, inducible promoters. That is, an inducible promoter may function as a weaker promoter under a non-induced condition, such as in the absence of the corresponding inducer. Furthermore, portion of or the entire expression control sequence may be deleted. The expression of a gene can also be reduced by, for example, manipulating a factor responsible for expression control. Examples of the factor responsible for expression control include low molecules responsible for transcription or translation control (inducers, inhibitors, etc.), proteins responsible for transcription or translation control (transcription factors, etc.), nucleic acids responsible for transcription or translation control (siRNA, etc.), and so forth. Furthermore, the expression of a gene can also be reduced by, for example, introducing a mutation that reduces the expression of the gene into the coding region of the gene. For example, the expression of a gene can be reduced by replacing a codon in the coding region of the gene with a synonymous codon used less frequently in a host. Furthermore, for example, the gene expression may be reduced due to disruption of a gene as described herein.

The modification for reducing the activity of a protein can also be attained by, for example, disrupting a gene encoding the protein. The expression "a gene is disrupted" means that a gene is modified so that a protein that can normally function is not produced. The phrase "a protein that normally functions is not produced" includes when the protein is not produced at all from the gene, and when the protein of which the function (such as activity or property) per molecule is reduced or eliminated is produced from the gene.

Disruption of a gene can be attained by, for example, deleting the gene on a chromosome. The term "deletion of a gene" refers to deletion of a partial or entire region of the coding region of the gene. Furthermore, the entire gene including sequences upstream and downstream from the coding region of the gene on a chromosome may be deleted. The sequences upstream and downstream from the coding region of the gene may include, for example, an expression control sequence of the gene. The region to be deleted may be any region such as an N-terminal region (region encoding an N-terminal region of a protein), an internal region, or a C-terminal region (region encoding a C-terminal region of a protein), so long as the activity of the protein can be reduced. Deletion of a longer region can usually more surely inactivate the gene. The region to be deleted may be, for example, a region having a length of 10% or more, 20% or more, 30% or more, 40% or more, 50% or more, 60% or more, 70% or more, 80% or more, 90% or more, or 95% or more of the total length of the coding region of the gene. Furthermore, it is preferred that reading frames of the sequences upstream and downstream from the region to be deleted are not the same. Inconsistency of reading frames may cause a frameshift downstream of the region to be deleted.

Disruption of a gene can also be attained by, for example, introducing a mutation for an amino acid substitution (missense mutation), a stop codon (nonsense mutation), addition or deletion of one or two nucleotide residues (frame shift mutation), etc. into the coding region of the gene on a chromosome (Journal of Biological Chemistry, 272:8611-8617 (1997); Proceedings of the National Academy of Sciences, USA, 95 5511-5515 (1998); Journal of Biological Chemistry, 26 116, 20833-20839 (1991)).

Disruption of a gene can also be attained by, for example, inserting another nucleotide sequence into a coding region of the gene on a chromosome. Site of the insertion may be in any region of the gene, and insertion of a longer nucleotide sequence can usually more surely inactivate the gene. It is preferred that reading frames of the sequences upstream and downstream from the insertion site are not the same. Inconsistency of reading frames may cause a frameshift downstream of the region to be deleted. The other nucleotide sequence is not particularly limited so long as a sequence that reduces or eliminates the activity of the encoded protein is chosen, and examples thereof include, for example, a marker gene such as antibiotic resistance genes, and a gene useful for production of an objective substance.

Particularly, disruption of a gene may be carried out so that the amino acid sequence of the encoded protein is deleted. In other words, the modification for reducing the activity of a protein can be attained by, for example, deleting the amino acid sequence of the protein, specifically, modifying a gene so as to encode a protein of which the amino acid sequence is deleted. The term "deletion of the amino acid sequence of a protein" refers to deletion of a partial or entire region of the amino acid sequence of the protein. In addition, the term "deletion of the amino acid sequence of a protein" means that the original amino acid sequence is not present in the protein, and also includes when the original amino acid sequence is changed to another amino acid sequence. That is, for example, a region that was changed to another amino acid sequence by frameshift may be regarded as a deleted region. When the amino acid sequence of a protein is deleted, the total length of the protein is typically shortened, but there can also be cases where the total length of the protein is not changed or is extended. For example, by deletion of a portion of or the entire coding region of a gene, a region encoded by the deleted region can be deleted in the encoded protein. In addition, for example, by introduction of a stop codon into the coding region of a gene, a region encoded by the downstream region of the introduction site can be deleted in the encoded protein. In addition, for example, by frameshift in the coding region of a gene, a region encoded by the frameshift region can be deleted in the encoded protein. The aforementioned descriptions concerning the position and length of the region to be deleted in deletion of a gene can be similarly applied to the position and length of the region to be deleted in deletion of the amino acid sequence of a protein.

Such modification of a gene on a chromosome as described above can be attained by, for example, preparing a disruption-type gene modified so that it is unable to produce a protein that normally functions, and transforming a host with a recombinant DNA containing the disruption-type gene to cause homologous recombination between the disruption-type gene and the wildtype gene on a chromosome and substitute the disruption-type gene for the wildtype gene on the chromosome. In this procedure, if a marker gene selected according to the characteristics of the host such as auxotrophy is included in the recombinant DNA, the operation becomes easier. Examples of the disruption-type gene include a gene in which a portion or the entire coding region is deleted, gene including a missense mutation, gene including a nonsense mutation, gene including a frame shift mutation, and gene inserted with an insertion sequence such as a transposon or marker gene. The protein encoded by the disruption-type gene has a conformation different from that of the wildtype protein, even if it is produced, and thus the function thereof is reduced or eliminated. The structure of the recombinant DNA to be used for homologous recombination is not particularly limited as long as it causes homologous recombination in a desired manner. For example, a host can be transformed with a linear DNA containing the disruption-type gene and further containing upstream and downstream sequences of the wildtype gene on the chromosome at the respective ends, so that homologous recombination occurs at each of upstream and downstream sides of the wildtype gene, thereby replacing the wildtype gene with the disruption-type gene. Such gene disruption based on gene substitution utilizing homologous recombination has already been established, and there are methods of using a linear DNA such as a method called "Red driven integration" (Datsenko, K. A, and Wanner, B. L., Proc. Natl. Acad. Sci. USA, 97:6640-6645 (2000)), and a method utilizing the Red driven integration in combination with an excision system derived from λ phage (Cho, E. H., Gumport, R I., Gardner, J. F., J. Bacteriol., 184:5200-5203 (2002)) (refer to WO2005/010175), a method of using a plasmid having a temperature sensitive replication origin, a method of using a plasmid capable of conjugative transfer, a method of utilizing a suicide vector not having a replication origin that functions in a host (U.S. Pat. No. 6,303,383, Japanese Patent Laid-open (Kokai) No. 05-007491), and so forth. Such methods for modifying a chromosome using homologous recombination can be used for any modification on a chromosome, such as a modification of an expression control sequence, as well as for disruption of an objective gene.

A modification for reducing activity of a protein can also be attained by, for example, a mutagenesis treatment. Examples of the mutagenesis treatment include irradiation of X-ray or ultraviolet and treatment with a mutation agent such as N-methyl-N'-nitro-N-nitrosoguanidine (MNNG), ethyl methanesulfonate (EMS), and methyl methanesulfonate (MMS).

Such methods for reducing the activity of a protein as mentioned above may be used independently or in an arbitrary combination.

A reduction in the activity of a protein can be confirmed by measuring the activity of the protein.

A reduction in the activity of a protein can also be confirmed by confirming a reduction in the expression of a gene encoding the protein. A reduction in the expression of a gene can be confirmed by confirming a reduction in the transcription amount of the gene or a reduction in the amount of the protein expressed from the gene.

A reduction in the transcription amount of a gene can be confirmed by comparing the amount of mRNA transcribed from the gene with that of a non-modified strain. Examples of the method for evaluating the amount of mRNA include Northern hybridization, RT-PCR, microarray, RNA-Seq, and so forth (Sambrook, J., et al., Molecular Cloning: A Laboratory Manual/Third Edition, Cold Spring Harbor Laboratory Press, Cold Spring Harbor (USA), 2001). The amount of mRNA may be reduced to, for example, 50% or less, 20% or less, 10% or less, 5% or less, or 0% of that of a non-modified strain.

A reduction in the amount of a protein can be confirmed by performing SDS-PAGE and confirming the intensity of the separated protein band. A reduction in the amount of a protein can be confirmed by Western blotting using antibodies (Sambrook, J., et al., Molecular Cloning: A Laboratory Manual/Third Edition, Cold Spring Harbor Laboratory Press, Cold Spring Harbor (USA), 2001). The amount of the protein (such as the number of molecules of the protein per cell) may be reduced to, for example, 50% or less, 20% or less, 10% or less, 5% or less, or 0% of that of a non-modified strain.

Disruption of a gene can be confirmed by determining nucleotide sequence of a part or the whole of the gene, restriction enzyme map, full length, etc. of the gene depending on the means used for the disruption.

The aforementioned methods for reducing the activity of a protein as mentioned above can be applied to reduction in the activities of any proteins and reduction in the expression of any genes.

### <2> Method for Producing ncAA-containing Protein

By culturing the bacterium obtained as described above in a medium containing ncAA to express a ncAA-containing protein, a large amount of the ncAA-containing protein secreted out of the cells is obtained.

The medium used is not particularly limited as long as it contains ncAA, allows the bacterium to grow, and produces a ncAA-containing protein. As the medium, for example, a typical medium used for culturing bacteria such as coryneform bacteria to which ncAA is added can be used. Specifically, examples of the medium include, in addition to ncAA, a typical medium containing a carbon source, a nitrogen source, inorganic ions, and so forth. Organic micronutrients such as vitamins and amino acids can also be added to the medium as required. The culture conditions are not particularly limited as long as they allow the bacterium to grow and a ncAA-containing protein to be produced. Culture can be carried out, for example, under usual conditions used for culturing bacteria such as coryneform bacteria. The types and concentrations of medium components and culture may be appropriately set depending on conditions such as the type of coryneform bacteria and the type of ncAA-containing protein.

As the carbon source, for example, carbohydrates such as glucose and sucrose, organic acids such as acetic acid, alcohols, or other suitable carbon sources can be used. As the nitrogen source, for example, ammonia gas, aqueous ammonia, ammonium salts, or other suitable nitrogen sources can be used. As the inorganic ions, for example, calcium ions, magnesium ions, phosphate ions, potassium ions, or iron ions are appropriately used as required. The culture can be performed within appropriate ranges of pH 5.0 to 8.5 and 15°C to 37°C under aerobic conditions for 1 to 7 days, for example. Furthermore, the culture conditions for L-amino acid production by coryneform bacteria and other conditions described for the methods for producing a protein using other Sec-dependent and Tat-dependent signal peptides can be used (refer to WO01/23591 and WO2005/103278). When inducible promoters are used for the expression of ncAA-containing proteins and/or an orthogonal pair of tRNA (ncAA)/ncAA-aaRS, their expression may or may not be induced. By culturing the bacterium under such conditions, a large amount of the ncAA-containing protein is produced in cells and efficiently secreted out of the cells. In addition, according to the method as described herein, the produced ncAA-containing protein is secreted out of the cells, and therefore a protein that is generally lethal if it is accumulated in a large amount in cells of microorganisms, such as transglutaminases, can also be continuously produced without lethal effect.

Culture can be performed by batch culture, fed-batch culture, continuous culture, or a combination thereof. Note that the medium at the start of culture is also referred to as the "initial medium". A medium supplied to a culture system (fermenter) in fed-batch culture or continuous culture is also referred to as a "fed-batch medium". Supplying a fed-batch medium to a culture system in fed-batch culture or continuous culture is also referred to as "fed-batch". Culture may be performed separately into seed culture and main culture.

The concentration of ncAA in the medium is not particularly limited as long as a ncAA-containing protein is produced. For example, ncAA may be contained in the medium at a concentration of 0.1 mM or more, 0.2 mM or more, 0.3 mM or more, 0.4 mM or more, 0.5 mM or more, 0.7 mM or more, or 1 mM or more and 20 mM or less, 10 mM or less, 5 mM or less, 3 mM or less, 2 mM or less, 1 mM or less, 0.7 mM or less, or 0.5 mM or less, or a consistent combination thereof. ncAA may be contained in the initial medium in the concentration range exemplified above and/or may be fed in batch mode during culture so as to have the concentration range exemplified above.

ncAA may or may not be contained in the medium during the entire culture period. For example, ncAA may or may not be contained in the medium at a predetermined concentration range, such as the concentration range exemplified above during the entire culture period. That is, for example, ncAA may be contained in the medium at a concentration other than the predetermined concentration range, such as the concentration range exemplified above, during a part of the period. ncAA may be deficient during a part of the period, for example. "Insufficient" refers to not meeting the required amount and may mean, for example, that the concentration in the medium is zero. For example, ncAA may be contained in the medium from the start of culture or may not be present. When ncAA is not contained in the medium at the start of culture, ncAA is supplied to the medium after the start of culture. The timing of supply can be appropriately set depending on various conditions, such as culture time. For example, ncAA may be supplied to the culture medium after the bacterium has grown sufficiently. Further, for example, ncAA may be consumed during culture, and its concentration in the medium may become zero. The "part of the period" may be, for example, 1% or less, 5% or less, 10% or less, 20% or less, 30% or less, or 50% or less of the entire culture period. The "entire period of culture" may mean the entire period of main culture when culture is performed separately into seed culture and main culture. In this way, even when ncAA is insufficient for a part of the period, as long as there is a culture period in a medium containing ncAA, it is included in "culturing a bacterium in a medium containing ncAA". ncAA may generally be included in the medium at least throughout the period during which expression of the ncAA-containing protein is desired.

The ncAA-containing protein secreted in the medium according to the method can be separated and purified from the medium after the culture by a method well known to those skilled in the art. For example, after the cells are removed by centrifugation etc., the ncAA-containing protein can be separated and purified by a known appropriate method such as salting out, ethanol precipitation, ultrafiltration, gel filtration chromatography, ion exchange column chromatography, affinity chromatography, medium or high pressure liquid chromatography, reverse phase chromatography, and hydrophobic chromatography, or a combination of these. Furthermore, in some cases, a fraction containing a ncAA-containing protein, such as a culture or a culture supernatant, may be used as it is as a ncAA-containing protein. The ncAA-containing protein secreted in the cell surface layer according to the method can also be separated and purified in the same manner as that for the case where the protein is secreted in the medium, after solubilizing it by a method well known to those skilled in the art such as elevation of salt concentration and use of a surfactant. Furthermore, in a certain case, the ncAA-containing protein secreted in the cell surface layer may be used as, for example, an immobilized enzyme, without solubilizing it.

Secretory production of the ncAA-containing protein can be confirmed by performing SDS-PAGE for the culture supernatant and/or a fraction containing the cell surface layer as a sample, and confirming the molecular weight of the separated protein band. Furthermore, secretory production of the ncAA-containing protein can also be confirmed by performing Western blotting using antibodies for the culture supernatant and/or a fraction containing the cell surface layer as a sample (Molecular Cloning, Cold spring Harbor Laboratory Press, Cold Spring Harbor (USA), 2001). Furthermore, secretory production of the ncAA-containing protein can also be confirmed by detecting an N-terminal amino acid sequence of the protein produced by secretory production using a protein sequencer. Furthermore, secretory production of the ncAA-containing protein can also be confirmed by determining the mass of the protein produced by secretory production using a mass spectrometer. Furthermore, when the ncAA-containing protein is an enzyme or a protein having a certain measurable physiological activity, secretory production of the ncAA-containing protein can be confirmed by measuring enzymatic activity or the physiological activity of the ncAA-containing protein in the culture supernatant and/or a fraction containing the cell surface layer as a sample.

The produced ncAA-containing protein may be, for example, modified before use. That is, the method as described herein may further include a step of modifying the produced ncAA-containing protein. The aspect of modification can be appropriately selected depending on conditions such as the intended use of the ncAA-containing protein. Examples of modification include PEGylation, fluorescent labeling, addition of fatty acid molecules, and addition of functional molecules (e.g., antibodies, antibody fragments, peptides, proteins, nucleic acids, organic compounds, inorganic compounds, sugar chains, lipids, polymers, metals (e.g., gold), and chelators). Modifications may or may not be site-specific, for example. For example, the azide group of a ncAA residue having an azide group such as AzF can be modified site-specifically by strain-promoted alkyne azide cycloaddition (SPAAC) reaction.

### Examples

The present invention will be further specifically explained with reference to the following non-limiting Examples.

In Examples, "azidophenylalanine" (AzF) refers to p-azido-L-phenylalanine, "3-chlorotyrosine" refers to 3-chloro-L-tyrosine, "3-azidotyrosine" (AzY) refers to 3-azido-L-tyrosine, "pyrrolysine" (Pyl) refers to L-pyrrolysine, and "alloclysine" (AllocLys) refers to N_{δ}-alloc-L-lysine.

### <Example 1: Expression of Mutant of Anti-Epidermal-Growth-Factor Receptor (EGFR) VHH antibody 9g8 Site-specifically Substituted with azidophenylalanine (AzF)>

### 1. Preparation of Plasmid Vector (AzFN3 vector) Containing AzFN3 DNA Cassette

An AzFN3 DNA cassette (SEQ ID NO: 55) was obtained by total synthesis. The AzFN3 DNA cassette includes: a T7 promoter and the azidophenylalanyl tRNA synthetase (AzFRS) gene linked downstream thereof; and an F1 promoter and the suppressor tRNA (tRNA_{CTA}) gene linked downstream thereof, which can translate a UAG codon of the promoter into azidophenylalanine (AzF). AzFRS is an archaea (Methanococcus jannaschii)-derived modified tyrosyl tRNA synthetase having a Y32T/E107N/D158P/I159L/L162Q mutation, which is modified so that azidophenylalanine is a substrate therefor (J. AM. CHEM. SOC. 2002, 124, 9026-9027). The nucleotide and amino acid sequences of the AzFRS gene and AzFRS are shown in SEQ ID NOS: 49 and 50, respectively. tRNA_{CTA} is an archaea (Methanococcus jannaschii)-derived modified tyrosyl tRNA, which is modified to have an anticodon (CTA) corresponding to UAG (amber). The nucleotide sequence of the tRNA_{CTA} gene is shown in SEQ ID NO: 41, and the nucleotide sequence of the tRNA(Tyr) encoded by this gene is shown in SEQ ID NO: 42. This AzFN3 DNA cassette is introduced into a coryneform bacterium and cultured in a medium to which azidophenylalanine is added; thus, azidophenylalanine is incorporated into the coryneform bacterium and binds to the end of tRNA_{CTA} by AzFRS, thereby producing azidophenylalanyl tRNA. Azidophenylalanyl tRNA binds to a UAG codon in mRNA during the protein translation process, and the UAG codon is translated as azidophenylalanine, thereby synthesizing a protein in which azidophenylalanine has been introduced at the position of the UAG codon.

The AzFN3 DNA cassette was cloned into a pVC7T7pol1 vector (Shuhei Hashiro et al., Efficient production of long double-stranded RNAs applicable to agricultural pest control by Corynebacterium glutamicum equipped with coliphage T7-expression system, Appl Microbiol Biotechnol, 2021 Jun 7), thereby obtaining an AzFN3 vector. The pVC7T7pol1 vector is a pVC7N vector (resistant to chloramphenicol drugs) into which the lacI gene and T7 RNA polymerase gene are integrated.

### 2. Preparation of Plasmid Vector (9g8+AzFRS vector) Containing Wildtype or Mutant 9g8 DNA Cassette and AzFRS DNA Cassette

A wildtype 9g8 DNA cassette (SEQ ID NO: 56) was obtained by total synthesis. This wildtype 9g8 DNA cassette contains a cspB promoter and a gene linked downstream thereof, the gene encoding wildtype 9g8 having a CspA signal sequence added to the N-terminus and a His tag sequence added to the C-terminus. 9g8 is a VHH antibody for the epidermal-growth-factor receptor (EGFR). The nucleotide sequence of the gene encoding wildtype 9g8 (excluding additional sequences) and the amino acid sequence of wildtype 9g8 (excluding additional sequences) are shown in SEQ ID NOS: 57 and 58, respectively. The wildtype 9g8 DNA cassette was cloned into a pPK4 vector (Japanese Patent Laid-open (Kokai) No. 9-322774; resistant to kanamycin drugs), thereby obtaining a pPK4 vector (wildtype 9g8 vector) containing the wildtype 9g8 DNA cassette.

Next, the wildtype 9g8 vector was modified to express 11 types of mutant 9g8 in which each of the amino acid residues at 11 locations at positions 32, 59, 60, 80, 94, 95, 100, 107, 112, 114, and 116 in wildtype 9g8 was substituted with azidophenylalanine. Specifically, the wildtype 9g8 vector was used as a template, and PCR using a primer pair for mutagenesis was performed to change a triplet encoding the aforementioned amino acid residues at 11 locations in the wildtype 9g8 gene to a TAG triplet corresponding to one of the stop codons, a UAG codon, thereby obtaining a pPK4 vector (mutant 9g8 vector) containing 11 types of mutant 9g8 DNA cassettes. The primer pairs used were a pair of SEQ ID NOS: 59 and 60, a pair of SEQ ID NOS: 61 and 62, a pair of SEQ ID NOS: 63 and 64, a pair of SEQ ID NOS: 65 and 66, a pair of SEQ ID NOS: 67 and 68, a pair of SEQ ID NOS: 69 and 70, a pair of SEQ ID NOS: 71 and 72, a pair of SEQ ID NOS: 73 and 74, a pair of SEQ ID NOS: 75 and 76, a pair of SEQ ID NOS: 77 and 78, and a pair of SEQ ID NOS: 79 and 80 for positions 32, 59, 60, 80, 94, 95, 100, 107, 112, 114, and 116 of wildtype 9g8, respectively. Modification sites are shown in FIG. 2.

The synthesized AzFRS DNA cassette (SEQ ID NO: 81) was cloned into the downstream regions of the wildtype and mutant 9g8 genes in the wildtype and mutant 9g8 vectors, thereby obtaining wildtype and mutant 9g8+AzFRS vectors. The AzFRS DNA cassette contains the T7 promoter and the AzFRS gene linked downstream thereof.

### 3. Expression of Mutant 9g8

The *C*. *glutamicum* YDK0107 strain was transformed with the AzFN3 vector and each mutant 9g8+AzFRS vector, thereby obtaining 11 types of mutant 9g8-expressing strains, each in which any of the 11 types of mutant 9g8 genes and the AzFN3 DNA cassette were introduced. The *C. glutamicum* YDK0107 strain was transformed with the wildtype 9g8 vector or with the AzFN3 vector and the wildtype 9g8+AzFRS vector, thereby obtaining wildtype 9g8-expressing strains (also referred to as control strain PC and control strain WT, respectively) as controls. The YDK0107 strain is a natural mutant strain obtained from the YDK010 strain (WO2002/081694), which is a cell surface layer protein CspB-deficient strain of *C*. *glutamicum* AJ12036 (FERM BP-734), by introducing PhoS(W302C) mutation into phoS gene on the chromosome thereof (WO2016/171224). Each 9g8-expressing strain was cultured at 30°C for 72 hours in a medium to which 0.3 mM azidophenylalanine was added. After completion of the culture, 10 µL of the culture supernatant obtained by centrifuging the culture broth of each strain was subjected to reduced SDS-PAGE, and then staining was carried out with coomassie brilliant blue (CBB)R-250 (Bio-Rad). As a result, a band was observed at the location corresponding to the full length of 9g8 in the presence of azidophenylalanine for the 11 types of mutant 9g8-expressing strains, thus confirming the expression of mutant 9g8 into which azidophenylalanine was introduced (FIG. 3).

### <Example 2: Expression of Mutant of Anti-Epidermal-Growth-Factor Receptor (Human Epidermal-Growth-Factor Receptor 2: HER2) Antibody ZHER2 Affibody Site-specifically Substituted with Azidophenylalanine (AzF)>

### 1. Preparation of Plasmid Vector (AzFN3 vector) Containing AzFN3 DNA Cassette The AzFN3 vector was prepared as described in 1. of Example 1.

### 2. Preparation of Plasmid Vector (ZHER2 affibody+AzFRS vector) Containing Wildtype or Mutant ZHER2 Affibody DNA Cassette and AzFRS DNA Cassette

A wildtype ZHER2 affibody DNA cassette (SEQ ID NO: 82) was obtained by total synthesis. This wildtype ZHER2 affibody DNA cassette contains a cspB promoter and a gene linked downstream thereof, the gene encoding wildtype ZHER2 affibody having a CspB signal sequence, six N-terminal amino acid residues of CspB mature protein, a TEV protease recognition sequence, and a His tag sequence added to the N-terminus in that order. The ZHER2 affibody is an affibody for the epidermal-growth-factor receptor (human epidermal-growth-factor receptor 2: HER2). The nucleotide sequence of the gene encoding wildtype ZHER2 affibody (excluding additional sequences) and the amino acid sequence of wildtype ZHER2 affibody (excluding additional sequences) are shown in SEQ ID NOS: 83 and 84, respectively. The wildtype ZHER2 affibody DNA cassette was cloned into a pPK5 vector (WO2018/074579; resistant to kanamycin drugs), thereby obtaining a pPK5 vector (wildtype ZHER2 affibody vector) containing the wildtype ZHER2 affibody DNA cassette.

Next, the wildtype ZHER2 affibody vector was modified to express four types of mutant ZHER2 affibody in which each of the amino acid residues at four locations at positions 7, 16, 22, and 37 in the wildtype ZHER2 affibody was substituted with azidophenylalanine. Specifically, the wildtype ZHER2 affibody vector was used as a template, and PCR using a primer pair for mutagenesis was performed to change a triplet encoding the aforementioned amino acid residues at four locations in the wildtype ZHER2 affibody gene to a TAG triplet corresponding to one of the stop codons, a UAG codon, thereby obtaining a pPK4 vector (mutant ZHER2 affibody vector) containing four types of mutant ZHER2 affibody DNA cassettes. The primer pairs used were a pair of SEQ ID NO: 85 and 86, a pair of SEQ ID NO: 87 and 88, a pair of SEQ ID NO: 89 and 90, and a pair of SEQ ID NO: 91 and 92 for positions 7, 16, 22, and 37 of the wildtype ZHER2 affibody, respectively. Modification sites are shown in FIG. 4.

The synthesized AzFRS DNA cassette (2. of Example 1) was cloned into the downstream regions of the wildtype and mutant ZHER2 affibody genes in the wildtype and mutant ZHER2 affibody vectors, thereby obtaining wildtype and mutant ZHER2 affibody+AzFRS vectors.

### 3. Expression of Mutant ZHER2 Affibody

The *C*. *glutamicum* YDK0107 strain was transformed with the AzFN3 vector and each mutant ZHER2 affibody+AzFRS vector, thereby obtaining four types of mutant ZHER2 affibody-expressing strains, each in which any of the four types of mutant ZHER2 affibody genes and the AzFN3 DNA cassette were introduced. The *C. glutamicum* YDK0107 strain was transformed with the wildtype ZHER2 affibody vector or with the AzFN3 vector and the wildtype ZHER2 affibody+AzFRS vector, thereby obtaining wildtype ZHER2 affibody-expressing strains (also referred to as control strain PC and control strain WT, respectively) as controls. Each ZHER2 affibody-expressing strain was cultured at 30°C for 72 hours in a medium to which 0.3 mM azidophenylalanine was added. After completion of the culture, 10 µL of the culture supernatant obtained by centrifuging the culture broth of each strain was subjected to reduced SDS-PAGE, and then staining was carried out with coomassie brilliant blue (CBB)R-250 (Bio-Rad). As a result, a band was observed at the location corresponding to the full length of ZHER2 affibody in the presence of azidophenylalanine for the 4 types of mutant ZHER2 affibody-expressing strains, thus confirming the expression of mutant ZHER2 affibody into which azidophenylalanine was introduced (FIG. 5).

### <Example 3: Expression 1 of Mutant of Monomeric Red Fluorescent Protein (mRFP) Site-specifically Substituted with Azidophenylalanine (AzF)>

### 1. Preparation of Plasmid Vector (AzFN3 vector) Containing AzFN3 DNA Cassette

The AzFN3 vector was prepared as described in 1. of Example 1.

### 2. Preparation of Plasmid Vector (mRFP+AzFRS vector) Containing Wildtype or Mutant mRFP DNA Cassette and AzFRS DNA Cassette and Plasmid Vector (mRFP+tRNA_{CTA} vector) Containing Wildtype or Mutant mRFP DNA Cassette and tRNA_{CTA} DNA Cassette

A wildtype mRFP DNA cassette (SEQ ID NO: 93) was obtained by total synthesis. This wildtype mRFP DNA cassette contains a cspB promoter and a gene linked downstream thereof, the gene encoding wildtype mRFP having a CspB signal sequence and six N-terminal amino acid residues of CspB mature protein added to the N-terminus in that order. The nucleotide sequence of the gene encoding wildtype mRFP (excluding additional sequences) and the amino acid sequence of wildtype mRFP (excluding additional sequences) are shown in SEQ ID NOS: 94 and 95, respectively. The wildtype mRFP DNA cassette was cloned into a pPK4 vector (Japanese Patent Laid-open (Kokai) No. 9-322774; resistant to kanamycin drugs), thereby obtaining a pPK4 vector (wildtype mRFP vector) containing the wildtype mRFP DNA cassette.

Next, the wildtype mRFP vector was modified to express mutant mRFP in which the amino acid residue at one location at position 36 in wildtype mRFP was substituted with azidophenylalanine. Specifically, the wildtype mRFP vector was used as a template, and PCR using a primer pair (a pair of SEQ ID NOS: 96 and 97) for mutagenesis was performed to change a triplet encoding the aforementioned amino acid residues at one location in the wildtype mRFP gene to a TAG triplet corresponding to one of the stop codons, a UAG codon, thereby obtaining a pPK4 vector (mutant mRFP vector) containing a mutant mRFP DNA cassette. Modification sites (including the modification at position 80 described in Example 5 below) are shown in FIG. 6.

The synthesized AzFRS DNA cassette (2. of Example 1) was cloned into the downstream regions of the wildtype and mutant mRFP genes in the wildtype and mutant mRFP vectors, thereby obtaining wildtype and mutant mRFP +AzFRS vectors. The synthesized tRNA_{CTA} DNA cassette (SEQ ID NO: 100) was cloned into the downstream regions of the wildtype and mutant mRFP genes in the wildtype and mutant mRFP vectors, thereby obtaining wildtype and mutant mRFP+tRNA_{CTA} vectors. The tRNA_{CTA} DNA cassette contains the F1 promoter and the tRNA_{CTA} gene linked downstream thereof.

### 3. Expression of Mutant mRFP

The *C*. *glutamicum* YDK0107 strain was transformed with the AzFN3 vector and the mutant mRFP+AzFRS vector or mutant mRFP+tRNA_{CTA} vector, thereby obtaining a mutant mRFP-expressing strain, in which the mutant mRFP gene and the AzFN3 DNA cassette were introduced. The *C. glutamicum* YDK0107 strain was transformed with the AzFN3 vector and the wildtype mRFP+AzFRS vector or the wildtype mRFP+tRNA_{CTA} vector, thereby obtaining wildtype mRFP-expressing strains (also referred to as control strain WT+AzFRS and control strain WT+tRNA, respectively) as controls. Each mRFP-expressing strain was cultured at 30°C for 72 hours in a medium to which 0.3 mM azidophenylalanine was added. After completion of the culture, 10 µL of the culture supernatant obtained by centrifuging the culture broth of each strain was subjected to reduced SDS-PAGE, and then staining was carried out with coomassie brilliant blue (CBB)R-250 (Bio-Rad). As a result, a band was observed at the location corresponding to the full length of mRFP in the presence of azidophenylalanine for the mutant mRFP-expressing strain, thus confirming the expression of mutant mRFP into which azidophenylalanine was introduced (FIG. 7).

### <Example 4: Expression 2 of Mutant of Monomeric Red Fluorescent Protein (mRFP) Site-specifically Substituted with Azidophenylalanine (AzF)>

### 1. Preparation of Plasmid Vector (AzFN3 vector) Containing AzFN3 DNA Cassette

The AzFN3 vector was prepared as described in 1. of Example 1.

### 2. Creation of Plasmid Vector Containing Wildtype or Mutant mRFP Gene

The wildtype mutant mRFP vectors were prepared as described in 2. of Example 3.

### 3. Expression of Mutant mRFP

The *C*. *glutamicum* YDK0107 strain was transformed with the AzFN3 vector and the mutant mRFP vector, thereby obtaining a mutant mRFP-expressing strain, in which the mutant mRFP gene and the AzFN3 DNA cassette were introduced. The *C. glutamicum* YDK0107 strain was transformed with the AzFN3 vector and the wildtype mRFP vector, thereby obtaining a wildtype mRFP-expressing strain (also referred to as control strain WT) as a control. Each mRFP-expressing strain was cultured at 30°C for 72 hours in a medium to which 0.3 mM azidophenylalanine was added. After completion of the culture, 10 µL of the culture supernatant obtained by centrifuging the culture broth of each strain was subjected to reduced SDS-PAGE, and then staining was carried out with coomassie brilliant blue (CBB)R-250 (Bio-Rad). As a result, a band was observed at the location corresponding to the full length of mRFP in the presence of azidophenylalanine for the mutant mRFP-expressing strain, thus confirming the expression of mutant mRFP into which azidophenylalanine was introduced (FIG. 8).

### <Example 5: Expression 3 of Mutant of Monomeric Red Fluorescent Protein (mRFP) Site-specifically Substituted with Azidophenylalanine (AzF)>

### 1. Preparation of Plasmid Vector (mRFP+AzFN3 vector) Containing Mutant mRFP DNA Cassette and AzFN3 DNA Cassette

The wildtype mRFP vector was prepared as described in 2. of Example 3.

Next, the wildtype mRFP vector was modified to express two types of mutant mRFP in which the amino acid residues at two locations at positions 36 and 80 in wildtype mRFP were each substituted with azidophenylalanine. Specifically, the wildtype mRFP vector was used as a template, and PCR using a primer pair for mutagenesis was performed to change a triplet encoding the aforementioned amino acid residues at two locations in the wildtype mRFP gene to a TAG triplet, thereby obtaining a pPK4 vector (mutant mRFP vector) containing two types of a mutant mRFP DNA cassettes. The primer pairs used were a pair of SEQ ID NO: 96 and 97 and a pair of SEQ ID NO: 98 and 99 for positions 36 and 80 of wildtype mRFP, respectively.

The AzFN3 DNA cassette (1. of Example 1) was cloned into the downstream regions of the wildtype and mutant mRFP genes in the wildtype and mutant mRFP vectors, thereby obtaining wildtype and mutant mRFP+AzFN3 vectors.

### 2. Expression of Mutant mRFP

The *C*. *glutamicum* YDK0107 strain was transformed with the pVC7T7pol1 vector and the mutant mRFP+AzFN3 vector, thereby obtaining two types of mutant mRFP-expressing strains, each in which any of two types of mutant mRFP genes and the AzFN3 DNA cassette were introduced. The *C. glutamicum* YDK0107 strain was transformed with the pVC7T7pol1 vector and the wildtype mRFP+AzFN3 vector, thereby obtaining a wildtype mRFP-expressing strain (also referred to as control strain WT) as a control. Each mRFP-expressing strain was cultured at 30°C for 72 hours in a medium to which 0.3 mM azidophenylalanine was added. After completion of the culture, 10 µL of the culture supernatant obtained by centrifuging the culture broth of each strain was subjected to reduced SDS-PAGE, and then staining was carried out with coomassie brilliant blue (CBB)R-250 (Bio-Rad). As a result, a band was observed at the location corresponding to the full length of mRFP in the presence of azidophenylalanine for the mutant mRFP-expressing strain, thus confirming the expression of mutant mRFP into which azidophenylalanine was introduced (FIG. 9).

### <Example 6: Expression of Mutant of Anti-Izumo Protein 1 N-terminal Extracellular Domain (NDOM) VHH Antibody N15 Site-specifically Substituted with 3-Chlorotyrosine (ClY)>

### 1. Preparation of Plasmid Vector (N15+IYN3 vector) Containing Mutant N15 DNA Cassette and IYN3 DNA Cassette

An IYN3 DNA cassette (SEQ ID NO: 101) was obtained by total synthesis. The IYN3 DNA cassette includes: a T7 promoter and the halogenated tyrosyl tRNA synthetase (IYRS) gene linked downstream thereof; and a cspB promoter and the suppressor tRNA (tRNA_{CTA}) gene linked downstream thereof, which can translate a UAG codon into halogenated tyrosine. IYRS is an archaea (*Methanococcus jannaschii*)*-derived* modified tyrosyl tRNA synthetase having a H70A/D158T/I159S, D286R D286R mutation, which is modified so that halogenated tyrosine having mutations of H70A, D158T, I159S, and D286R is a substrate therefor (Biochem. Biophys. Res. Commun. 411, 757-761 (2011)). The nucleotide and amino acid sequences of the IYRS gene and IYRS are shown in SEQ ID NOS: 51 and 52, respectively. tRNA_{CTA} is an archaea (Methanococcus jannaschii)-derived modified tyrosyl tRNA, which is modified to have an anticodon (CTA) corresponding to UAG (amber). The nucleotide sequence of the tRNA_{CTA} gene is shown in SEQ ID NO: 43, and the nucleotide sequence of the tRNA(Tyr) encoded by this gene is shown in SEQ ID NO: 44. This IYN3 DNA cassette is introduced into a coryneform bacterium and cultured in a medium to which halogenated tyrosine is added; thus, halogenated tyrosine is incorporated into the coryneform bacterium and binds to the end of tRNA_{CTA} by IYRS, thereby producing halogenated tyrosyl tRNA. Halogenated tyrosyl tRNA binds to a UAG codon in mRNA during the protein translation process, and the UAG codon is translated as halogenated tyrosine, thereby synthesizing a protein in which halogenated tyrosine has been introduced at the position of the UAG codon.

A wildtype N15 DNA cassette (SEQ ID NO: 102) was obtained by total synthesis. The wildtype N15 DNA cassette contains a cspB promoter and a gene linked downstream thereof, the gene encoding wildtype N15 having a CspB signal sequence and six N-terminal amino acid residues of CspB mature protein added to the N-terminus in that order. N15 is an anti-NDOM VHH antibody. The nucleotide sequence of the gene encoding wildtype N15 (excluding additional sequences) and the amino acid sequence of wildtype N15 (excluding additional sequences) are shown in SEQ ID NOS: 103 and 104, respectively. The wildtype N15 DNA cassette was cloned into a pPK4 vector (Japanese Patent Laid-open (Kokai) No. 9-322774; resistant to kanamycin drugs), thereby obtaining a pPK4 vector (wildtype N15 vector) containing the wildtype N15 DNA cassette.

Next, the wildtype N15 vector was modified to express three types of mutant N15 in which each of the amino acid residues at three locations at positions 60, 81, and 96 in wildtype N15 was substituted with3-chlorotyrosine. Specifically, the wildtype N15 vector was used as a template, and PCR using a primer pair for mutagenesis was performed to change a triplet encoding the aforementioned amino acid residues at three locations in the wildtype N15 gene to a TAG triplet corresponding to one of the stop codons, a UAG codon, thereby obtaining a pPK4 vector (mutant N15 vector) containing three types of mutant N15 DNA cassettes. The primer pairs used were a pair of SEQ ID NOS: 105 and 106, a pair of SEQ ID NOS: 107 and 108, and a pair of SEQ ID NOS: 109 and 110 for positions 60, 81, and 96 of wildtype N15, respectively. Modification sites are shown in FIG. 10.

The IYN3 DNA cassette was cloned into the downstream regions of the wildtype and mutant N15 genes in the wildtype and mutant N15 vectors, thereby obtaining wildtype and mutant N15+IYN3 vectors.

### 2. Expression of Mutant N15

The *C*. *glutamicum* YDK0107 strain was transformed with the pVC7T7pol1 vector and the mutant N15+IYN3 vector, thereby obtaining three types of mutant N15-expressing strains, each in which any of the three types of mutant N15 genes and the IYN3 DNA cassette were introduced. The *C*. *glutamicum* YDK0107 strain was transformed with the wildtype N15 vector or with the pVC7T7pol 1 vector and the wildtype N15+IYN3 vector, thereby obtaining wildtype N15-expressing strains (also referred to as control strain PC and control strain WT, respectively) as controls. Each N15-expressing strain was cultured at 30°C for 72 hours in a medium to which 1 mM 3-chlorotyrosine was added. After completion of the culture, 10 µL of the culture supernatant obtained by centrifuging the culture broth of each strain was subjected to reduced SDS-PAGE, and then staining was carried out with coomassie brilliant blue (CBB)R-250 (Bio-Rad). As a result, a band was observed at a location corresponding to the full length of N15 in the presence of 3-chlorotyrosine for the mutant N15-expressing strain, thus confirming the expression of mutant N15 into which 3-chlorotyrosine was introduced (FIG. 11).

### <Example 7: Expression of Mutant of anti-Izumo protein 1 N-terminal Extracellular Domain (NDOM) VHH antibody Site-specifically Substituted with 3-Azidotyrosine (AzY)>

It is known that 3-azidotyrosine (AzY) is also a substrate for halogenated tyrosyl tRNA synthetase (IYRS) (Nucleic Acids Research 2010, Vol. 38, 3682-3691). Therefore, the IYN3 DNA cassette (1. of Example 6) is introduced into a coryneform bacterium and cultured in a medium to which 3-azidotyrosine is added; thus, 3-azidotyrosine is incorporated into the coryneform bacterium and binds to the end of tRNA_{CTA} by IYRS, thereby producing 3-azidotyrosyl tRNA. 3-Azidotyrosyl tRNA binds to a UAG codon in mRNA during the protein translation process, and the UAG codon is translated as 3-azidotyrosine, thereby synthesizing a protein in which 3-azidotyrosine has been introduced at the position of the UAG codon.

Each N15-expressing strain obtained in 2. of Example 6 was cultured at 30°C for 72 hours in a medium to which 0.3 mM 3-azidotyrosine was added. After completion of the culture, 10 µL of the culture supernatant obtained by centrifuging the culture broth of each strain was subjected to reduced SDS-PAGE, and then staining was carried out with coomassie brilliant blue (CBB)R-250 (Bio-Rad). As a result, a band was observed at a location corresponding to the full length of N15 in the presence of 3-azidotyrosine for the mutant N15-expressing strain, thus confirming the expression of mutant N15 into which3 -azidotyrosine was introduced (FIG. 12).

### <Example 8: Expression of Mutant of Anti-Epidermal-Growth-Factor Receptor (Epidermal-Growth-Factor Receptor: EGFR) VHH antibody 9g8 Site-specifically Substituted with Alloclysine (AllocLys)>

### Preparation of Plasmid Vector (PylRS+9g8+tRNA(Pyl)CTA/tRNA(Pyl)TCA vector) Containing Wildtype or Mutant 9g8 DNA Cassette, PylRS DNA Cassette, and tRNA(Pyl)CTA or tRNA(Pyl)TCA DNA Cassette

A wildtype 9g8 DNA cassette (SEQ ID NO: 56) was obtained by total synthesis. This wildtype 9g8 DNA cassette contains a cspB promoter and a gene linked downstream thereof, the gene encoding wildtype 9g8 having a CspA signal sequence added to the N-terminus and a His tag sequence added to the C-terminus. 9g8 is a VHH antibody for the epidermal-growth-factor receptor (EGFR). The nucleotide sequence of the gene encoding wildtype 9g8 (excluding additional sequences) and the amino acid sequence of wildtype 9g8 (excluding additional sequences) are shown in SEQ ID NOS: 57 and 58, respectively. The wildtype 9g8 DNA cassette was cloned into a pPK4 vector (Japanese Patent Laid-open (Kokai) No. 9-322774; resistant to kanamycin drugs), thereby obtaining a pPK4 vector (wildtype 9g8 vector) containing the wildtype 9g8 DNA cassette.

Next, the wildtype 9g8 vector was modified to express two types of mutant 9g8 in which each of the amino acid residues at two locations at positions 32 and 107 in wildtype 9g8 was substituted with alloclysine. Specifically, the wildtype 9g8 vector was used as a template, and PCR using a primer pair for mutagenesis was performed to change a triplet encoding the amino acid residue at position 32 in the wildtype 9g8 gene to a TGA triplet corresponding to one of the stop codons, a UAG codon (amber), (this mutant 9g8 is also referred to as9g8 (Y32TAG)) or to change a triplet encoding the amino acid residue at position 107 in the wildtype 9g8 gene to a TGA triplet corresponding to one of the stop codons, a UGA codon (opal), (this mutant 9g8 is also referred to as 9g8 (Y107TGA)), thereby obtaining a pPK4 vector (mutant 9g8 vector) containing two types of mutant 9g8 DNA cassettes. The primer pairs used were a pair of SEQ ID NO: 59 and 60 and a pair of SEQ ID NO: 111 and 112 for positions 32 and 107 of wildtype 9g8, respectively. Modification sites are shown in FIG. 2.

A PylRS DNA cassette (SEQ ID NO: 113) was obtained by total synthesis. The PylRS DNA cassette contains lac and T7 promoters and the pyrrolidyl tRNA synthetase (PylRS) gene linked downstream thereof. PylRS is an archaea (*Methanosarcina mazei*)-derived pyrrolidyl tRNA synthetase (Chemistry & Biology 2008 15, 1187-1197). The nucleotide and amino acid sequences of the PylRS gene and PylRS are shown in SEQ ID NOS: 114 and 115, respectively.

A tRNA(Pyl)CTA DNA cassette (SEQ ID NO: 116) and a tRNA(Pyl)TCA DNA cassette (SEQ ID NO: 117) were obtained by total synthesis. Each of the tRNA(Pyl)CTA DNA cassette and the tRNA(Pyl)TCA DNA cassette contains an F1+44 promoter, a suppressor tRNA gene linked downstream thereof, which can translate a stop codon into a pyrrolidine (Pyl)-related compound, and an rrnC terminator linked downstream of the gene. tRNA(Pyl)CTA is an archaea (*Methanosarcina mazei*)-derived modified pyrrolidyl tRNA, which is modified to have an anticodon (CTA) corresponding to UAG (amber). The nucleotide sequence of the tRNA(Pyl)CTA gene and the nucleotide sequence of tRNA(Pyl)CTA encoded by the gene are shown in SEQ ID NOS: 118 and 119, respectively. tRNA(Pyl)TCA is an archaea (*Methanosarcina mazei*)-derived modified pyrrolidyl tRNA, which is modified to have an anticodon (TCA) corresponding to UGA (opal). The nucleotide sequence of the tRNA(Pyl)TCA gene and the nucleotide sequence of tRNA(Pyl)TCA encoded by the gene are shown in SEQ ID NOS: 120 and 121, respectively.

The PylRS DNA cassette and the tRNA(Pyl)CTA DNA cassette or the tRNA(Pyl)TCA DNA cassette are introduced into a coryneform bacterium and cultured in a medium to which a pyrrolidine-related compound is added; thus, the pyrrolidine-related compound is incorporated into the coryneform bacterium and binds to the end of tRNA(Pyl)CTA or tRNA(Pyl)TCA by PylRS, thereby producing pyrrolidyl tRNA. Pyrrolidyl tRNA binds to a UAG codon or a UGA codon in mRNA during the protein translation process, and each codon is translated as a pyrrolidine-related compound, thereby synthesizing a protein in which the pyrrolidine-related compound has been introduced at the position of the UAG codon or the UGA codon.

The PylRS DNA cassette was cloned into the upstream regions of the wildtype and mutant 9g8 genes in the wildtype and mutant 9g8 vectors, and the tRNA(Pyl)CTA DNA cassette or the tRNA(Pyl)TCA DNA cassette was cloned into the downstream regions thereof, thereby obtaining PylRS+WT and mutant 9g8+tRNA(Pyl)CTA vectors or PylRS+WT and mutant 9g8+tRNA(Pyl)TCA vectors.

### 2. Expression of Mutant 9g8

The *C*. *glutamicum* YDK0107 strain was transformed with the PylRS+mutant 9g8+tRNA(Pyl)CTA vector or the PylRS+mutant 9g8+tRNA(Pyl)TCA vector, thereby obtaining four types of mutant 9g8-expressing strains into which any one of two types of mutant 9g8 genes were introduced, and the PylRS DNA cassette and the tRNA(Pyl)CTA DNA cassette or the tRNA(Pyl)TCA DNA cassette were introduced. The *C*. *glutamicum* YDK0107 strain was transformed with the wildtype 9g8 vector or the PylRS+WT 9g8+tRNA(Pyl)CTA vector, thereby obtaining wildtype 9g8-expressing strains (also referred to as control strain PC and control strain WT, respectively) as controls. Each 9g8-expressing strain was cultured at 30°C for 72 hours in a medium to which 1 mM alloclysine was added. After completion of the culture, 10 µL of the culture supernatant obtained by centrifuging the culture broth of each strain was subjected to reduced SDS-PAGE, and then staining was carried out with coomassie brilliant blue (CBB)R-250 (Bio-Rad). As a result, a band was observed at a location corresponding to the full length of 9g8 in the presence of alloclysine for two types of mutant 9g8-expressing strains, in which a combination of the codon on mutant 9g8 gene and the anticodon on tRNA(Pyl) is correct, thus confirming the expression of mutant 9g8 into which alloclysine was introduced (lanes 7 and 9 of Panel (A) in FIG. 13).

### <Example 9: Expression of Mutant of Anti-Epidermal-Growth-Factor Receptor (EGFR) VHH antibody 9g8 Site-specifically Substituted with azidophenylalanine (AzF) by Escherichia coli>

### 1. Preparation of Plasmid Vector (AzFN3 vector for E. coli) Containing AzFN3 DNA Cassette

Similar to the coryneform bacterium, a protein in which azidophenylalanine is introduced at the position of the UAG codon is synthesized by introducing the AzFN3 DNA cassette (1. of Example 1) into *Escherichia coli.*

The AzFN3 DNA cassette was cloned downstream of the lacI gene of a plasmid in which the drug resistance of the pCDF-1b vector (Novagen) was changed to kanamycin resistance, thereby obtaining an AzFN3 vector for *E*. *coli.*

### 2. Preparation of Plasmid Vector (9g8 vector for E. coli) Containing Wildtype or Mutant 9g8 DNA Cassette

A wildtype 9g8 DNA cassette for *E. coli.* (SEQ ID NO: 122) was obtained by total synthesis. This wildtype 9g8 DNA cassette for *E. coli* contains a T7 promoter and a gene linked downstream thereof, the gene encoding wildtype 9g8 having a pelB leader sequence added to the N-terminus and a His tag sequence added to the C-terminus. The wildtype 9g8 DNA cassette for *E. coli* was cloned into a pET26b vector (kanamycin resistance of the pET26b(+) vector (Novagen) was changed to ampicillin resistance), thereby obtaining a pET26b vector containing the wildtype 9g8 DNA cassette for *E. coli* (wildtype 9g8 vector for *E. coli*)*.*

Next, the wildtype 9g8 vector for *E. coli* was used as a template, and a triplet encoding the amino acid residue at position 32 of wildtype 9g8 was changed to a TAG triplet (this mutant 9g8 is also referred to as 9g8 (Y32TAG)) according to the procedure as in 2. of Example 1, thereby obtaining a pET26b vector containing the mutant 9g8 DNA cassette for *E. coli* (mutant 9g8 vector for *E. coli*)*.*

### 3. Expression of Mutant 9g8

The *E. coli* BL21(DE3) strain was transformed with the AzFN3 vector for *E*. *coli* and the mutant 9g8 vector for *E. coli*, thereby obtaining a mutant 9g8-expressing strain, into which the mutant 9g8 gene and the AzFN3 DNA cassette were introduced. The *E. coli* BL21 (DE3) strain was transformed with the AzFN3 vector for *E. coli* and the wildtype 9g8 vector for *E. coli*, thereby obtaining a wildtype 9g8-expressing strain as a control. Each 9g8-expressing strain was cultured overnight at 37°C in a medium to which 0, 0.1, 0.3, or 1.5 mM azidophenylalanine was added so that mutant 9g8, into which wildtype 9g8 was introduced, and mutant 9g8, into which azidophenylalanine was introduced, were expressed. Thereafter, the bacterial cells were collected, 100 µL of TE buffer (2.5% SDS) was added, and the mixture was heated at 95°C for 5 minutes, thereby obtaining a protein extract containing wildtype or mutant 9g8. Each extract in an amount of 10 µL was subjected to reduced SDS-PAGE and then stained with coomassie brilliant blue (CBB) R-250 (Bio-Rad). Concerning the obtained SDS PAGE results, the expression level ratio (Amb/wt) of mutant 9g8 to wildtype 9g8 at each AzF addition concentration was calculated using the automatic image detection system ChemiDoc (Bio-Rad) (Panel (A) in FIG. 14). The expression of mutant 9g8 in the absence of AzF is thought to be due to non-specific uptake of tyrosine. Furthermore, the proteins were transferred from the gel after SDS PAGE to a 0.2 µm PVDF membrane (Bio-Rad), bound to an alkaline phosphatase-fused anti-C-terminal His tag antibody (Invitrogen), and detected by coloring with a coloring reagent for alkaline phosphatase (Bio-Rad) (Panel (B) in FIG. 14).

### 4. Comparison and Evaluation of Azidophenylalanine Introduction Efficiency in C. glutamicum and E. coli

A pPK4 vector containing the AzFRS DNA cassette, the wildtype 9g8 or mutant 9g8 DNA cassette, and the tRNA_{CTA} DNA cassette (AzFRS+WT 9g8+tRNA_{CTA} vector or AzFRS+mutant 9g8+tRNA_{CTA} vector) was prepared according to the procedure as in 2. of Example 1 and 1. of Example 8. This mutant 9g8 DNA cassette encodes 9g8 (Y32TAG). *C*. *glutamicum* was cultured in a medium to which 0, 0.1, 0.3, or 1.5 mM azidophenylalanine was added according to the procedure as in 3. of Example 1 so that wildtype 9g8 and mutant 9g8 were expressed. After completion of the culture, 10 µL of the culture broth of each strain was subjected to reduced SDS-PAGE, and then staining was carried out with coomassie brilliant blue (CBB)R-250 (Bio-Rad). Concerning the obtained SDS PAGE results, the expression level ratio (Amb/wt) of mutant 9g8 to wildtype 9g8 at each AzF addition concentration was calculated using the automatic image detection system ChemiDoc (Bio-Rad) (FIG. 15). The expression of mutant 9g8 in the absence of AzF is thought to be due to non-specific uptake of tyrosine.

At every AzF addition concentration (from 0.1 to 1.5 mM), *C. glutamicum* showed a higher expression level ratio (Amb/wt) than *E. coli.* That is, the azidophenylalanine introduction efficiency was higher than that of *E. coli* (Panel (A) in FIG. 14 and FIG. 15). In addition, when AzF was not added, *C. glutamicum* showed a lower expression level ratio (Amb/wt) than *E. coli.* That is, the non-specific uptake of tyrosine was more suppressed than in *E. coli* (Panel (A) in FIG. 14 and FIG. 15). The above revealed that coryneform bacteria can efficiently and/or specifically produce ncAA-containing proteins, such as azidophenylalanine-containing proteins, more than *E. coli.*

### <Example 10: Expression of aMD4dY-PA22 Mutant Site-specifically Substituted with Azidophenylalanine (AzF)>

### 1. Construction of pPK5 Vector Carrying AzFRS DNA Cassettes (pPK14c and pPK14d)

The synthesized AzFRS DNA cassettes (SEQ ID NOS: 123 and 124) were incorporated into the KpnI-XbaI site of the pPK5 vector by an infusion reaction as described in WO2016/171224, thereby basic vectors pPK14c and pPK14d into which the AzFRS DNA cassettes were incorporated. The AzFRS DNA cassette of pPK14c (SEQ ID NO: 123) contains a T7 promoter, the AzFRS gene linked downstream thereof, the KpnI site at the 5'-end, and the ApaI and XbaI sites at the 3'-end. The AzFRS DNA cassette of pPK14d (SEQ ID NO: 124) contains a rrnC terminator, aT7 promoter and the AzFRS gene linked downstream thereof, the KpnI and BamHI sites at the 5'-end, and the ApaI and XbaI sites at the 3'-end. The infusion reaction was carried out with In-Fusion(registered trademark) HD Cloning Kit (Takara Bio), and the reaction conditions were according to the protocol recommended by the manufacturer. As a result of nucleotide sequencing of the inserted fragment, it was confirmed that vectors carrying AzFRS DNA cassettes were constructed as designed. Nucleotide sequencing was carried out with BigDye(registered trademark) Terminator v3.1 Cycle Sequencing Kit (Applied Biosystems) and 3500xL Genetic Analyzer (Applied Biosystems).

The pPK14c and pPK14d vectors can be used as basic vectors for AzF-introduced protein expression. When subcloning an expression cassette of an AzF-introduced protein using pPK14c as an expression vector, the insertion order of the AzFRS DNA cassette and the expression cassette of the AzF-introduced protein can be controlled by using the KpnI site or ApaI site. Similarly, when subcloning an expression cassette of an AzF-introduced protein using pPK14c as an expression vector, the insertion order of the AzFRS DNA cassette and the expression cassette of the AzF-introduced protein can be controlled by using the BamHI site or ApaI site.

### 2. Preparation of Plasmid Vector (aMD4dY-PA22+AzFRS vector) Containing Wildtype or Mutant aMD4dY-PA22 DNA Cassette and AzFRS DNA Cassette

As a peptide for introducing azidophenylalanine, aMD4dY-PA22, which is an HGF-PAS dimer peptide described in WO2021/112249, was used. aMD4dY-PA22 is a HGF-like active peptide.

A wildtype aMD4dY-PA22 DNA cassette (SEQ ID NO: 125) was obtained by total synthesis. In addition, three types of mutant aMD4dY-PA22 DNA cassettes were obtained by total synthesis for the expression of mutant aMD4dY-PA22 in which AzF was inserted on the N-terminal side (between positions 3 and 4) of wildtype aMD4dY-PA22, mutant aMD4dY-PA22 in which AzF was inserted in the PA22 linker (between positions 32 and 33) of the wildtype aMD4dY-PA22, and mutant aMD4dY-PA22 in which Pro (Pro at position 31) in the PA22 linker of wildtype aMD4dY-PA22 was substituted with AzF. The nucleotide sequence of each mutant aMD4dY-PA22 DNA cassette is the same as the nucleotide sequence of the wildtype aMD4dY-PA22 DNA cassette, except that it contains each mutant aMD4dY-PA22 gene instead of the wildtype aMD4dY-PA22 gene. Specifically, the mutant aMD4dY-PA22 gene includes an insertion or substitution of a TAG triplet corresponding to one of the stop codons, a UAG codon (amber), in the nucleotide sequence of the wildtype aMD4dY-PA22 gene, and the TAG triplet can be translated into AzF. Wildtype aMD4dY-PA22 is expressed as WT-aMD4dY-PA22, and three types of mutant aMD4dY-PA22 are expressed as N-AzF-aMD4dY-PA22, L1-AzF-aMD4dY-PA22, and L2-AzF-aMD4dY-PA22. The wildtype or mutant aMD4dY-PA22 DNA cassette contains a cspB promoter and a gene linked downstream thereof, the gene encoding wildtype or mutant aMD4dY-PA22 having a CspB signal sequence added to the N-terminus. The nucleotide and amino acid sequences of the gene encoding WT-aMD4dY-PA22 (excluding additional sequences) are shown in SEQ ID NOS: 126 and 127, respectively. The nucleotide and amino acid sequences of the gene encoding N-AzF-aMD4dY-PA22 (excluding additional sequences) are shown in SEQ ID NOS: 128 and 129, respectively. The nucleotide and amino acid sequences of the gene encoding L1-aMD4dY-PA22 (excluding additional sequences) are shown in SEQ ID NOS: 130 and 131, respectively. The nucleotide and amino acid sequences of the gene encoding L2-aMD4dY-PA22 (excluding additional sequences) are shown in SEQ ID NOS: 132 and 133, respectively.

FIG. 16 shows the nucleotide sequences of the wildtype and mutant aMD4dY-PA22 genes and the alignment of wildtype aMD4dY-PA22 and mutant aMD4dY-PA22.

Each synthesized aMD4dY-PA22 DNA cassette was incorporated into the ApaI site of the pPK14d vector constructed in 1. of Example 10 by an infusion reaction, thereby obtaining pPK14d(A)_WT-aMD4dY-PA22, pPK14d(A)_N-AzF-aMD4dY-PA22, pPK14d(A)_L1-AzF-aMD4dY-PA22, and pPK14d(A)_L2-AzF-aMD4dY-PA22, each of which is the wildtype or mutant aMD4dY-PA22 expression vector. As a result of nucleotide sequencing of the inserted fragment, it was confirmed that vectors carrying wildtype or mutant aMD4dY-PA22+AzFRS were constructed as designed.

### 3. Expression of Mutant aMD4dY-PA22

The *C*. *glutamicum* YDK0107 strain was transformed with the AzFN3 vector described in Example 1 and pPK14d(A)_N-AzF-aMD4dY-PA22, pPK14d(A)_L1-AzF-aMD4dY-PA22, or pPK14d(A)_L2-AzF-aMD4dY-PA22, which was the mutant aMD4dY-PA22+AzFRS expression vector constructed in 2. of Example 10, thereby obtaining three types of mutant aMD4dY-PA22-expressing strains, into which any one of three types of mutant aMD4dY-PA22 genes was introduced, and the AzFRS DNA cassette and the AzFN3 DNA cassette were also introduced. The *C*. *glutamicum* YDK0107 strain was transformed with the AzFN3 vector and pPK14d(A)_WT-aMD4dY-PA22, which was the wildtype aMD4dY-PA22+AzFRS expression vector, thereby obtaining a wildtype aMD4dY-PA22-expressing strain as a control.

Each aMD4dY-PA22-expressing strain was cultured at 30°C for 96 hours in a medium to which 0.3 mM azidophenylalanine was added. After completion of the culture, 6.5 µL of the culture supernatant obtained by centrifuging each culture broth was subjected to reduced SDS-PAGE using NuPAGE(registered trademark) 12% Bis-Tirs Gel (Thermo Fisher Scientific), and then staining was carried out with Quick-CBB (Wako).

As a result, a band was not observed at a location corresponding to the full length of aMD4dY-PA22 in the absence of azidophenylalanine in the culture supernatants of the L1-AzF-aMD4dY-PA22-expressing strain and the L2-AzF-aMD4dY-PA22-expressing strain, while a band was observed at a location corresponding to the full length of aMD4dY-PA22 in the presence of azidophenylalanine, thus confirming the expression of mutant aMD4dY-PA22 into which azidophenylalanine was introduced (lanes 6-9 in FIG. 17).

A band was observed at a location corresponding to the full length of aMD4dY-PA22 even in the absence of azidophenylalanine in the culture supernatant of the N-AzF-aMD4dY-PA22-expressing strain (lane 4 in FIG. 17). As a result of analyzing the N-terminal amino acid sequence of this band, the sequence "AETY..." was observed, thus confirming that tyrosine was incorporated into the UAG codon. Since the codon of tyrosine is UAC or UAU, it is known that false uptake of tyrosine into the UAG codon (amber) might occur in the absence of a noncanonical amino acid (Nucleic Acids Research, 2002, 30(21), 4692-4699). It was confirmed that false uptake of tyrosine into the UAG codon (amber) might occur in the absence of azidophenylalanine also in the secretory expression of N-AzF-aMD4dY-PA22 by *C*. *glutamicum*.

Similarly, as a result of analyzing the N-terminal amino acid sequence of the expression band of N-AzF-aMD4dY-PA22 in the culture supernatant obtained in the presence of azidophenylalanine (lane 5 in FIG. 17), the sequence "AETF..." was observed. That is, tyrosine was not detected at the azidophenylalanine introduction site of the UAG codon, but phenylalanine was detected. This revealed that in the secretory expression of N-AzF-aMD4dY-PA22 by *C. glutamicum*, azidophenylalanine introduction is prioritized over false uptake of tyrosine into the UAG codon (amber) in the presence of azidophenylalanine so that secretory expression of N-AzF-aMD4dY-PA22 containing azidophenylalanine is possible with high efficiency.

### <Example 11: Expression of EPO-PA22 Mutant Site-specifically Substituted with Azidophenylalanine (AzF)>

### 1. Preparation of Plasmid Vector (EPO-PA22+AzFRS vector) Containing Wildtype or Mutant EPO-PA22 DNA Cassette and AzFRS DNA Cassette

As a peptide for introducing azidophenylalanine, EPO-PA22, which is an EPO-PAS dimer peptide described in WO2021/112249, was used. EPO-PA22 is an erythropoietin-like active peptide.

A wildtype EPO-PA22 DNA cassette (SEQ ID NO: 134) was obtained by total synthesis. In addition, three types of mutant EPO-PA22 DNA cassettes were obtained by total synthesis for the expression of mutant EPO-PA22 in which AzF was inserted on the N-terminal side (between positions 3 and 4) of wildtype EPO-PA22, mutant EPO-PA22 in which AzF was inserted in the PA22 linker (between positions 35 and 36) of the wildtype EPO-PA22, and mutant EPO-PA22 in which Pro (Pro at position 34) in the PA22 linker of wildtype EPO-PA22 was substituted with AzF. The nucleotide sequence of each mutant EPO-PA22 DNA cassette is the same as the nucleotide sequence of the wildtype EPO-PA22 DNA cassette, except that it contains each mutant EPO-PA22 gene instead of the wildtype EPO-PA22 gene. Specifically, the mutant EPO-PA22 gene includes an insertion or substitution of a TAG triplet corresponding to one of the stop codons, a UAG codon (amber), in the nucleotide sequence of the wildtype EPO-PA22 gene, and the TAG triplet can be translated into AzF. Wildtype EPO-PA22 is expressed as WT-EPO-PA22, and three types of mutant EPO-PA22 are expressed as N-AzF-EPO-PA22, L1-AzF-EPO-PA22, and L2-AzF-EPO-PA22. The wildtype or mutant EPO-PA22 DNA cassette contains a cspB promoter and a gene linked downstream thereof, the gene encoding wildtype or mutant EPO-PA22 having a CspB signal sequence added to the N-terminus. The nucleotide and amino acid sequences of the gene encoding WT-EPO-PA22 (excluding additional sequences) are shown in SEQ ID NOS: 135 and 136, respectively. The nucleotide and amino acid sequences of the gene encoding N-AzF-EPO-PA22 (excluding additional sequences) are shown in SEQ ID NOS: 137 and 138, respectively. The nucleotide and amino acid sequences of the gene encoding L1-AzF-EPO-PA22 (excluding additional sequences) are shown in SEQ ID NOS: 139 and 140, respectively. The nucleotide and amino acid sequences of the gene encoding L2-AzF-EPO-PA22 (excluding additional sequences) are shown in SEQ ID NOS: 141 and 142, respectively.

FIG. 18 shows the nucleotide sequences of the wildtype and mutant EPO-PA22 genes and the alignment of wildtype EPO-PA22 and mutant EPO-PA22.

Each synthesized EPO-PA22 DNA cassette was incorporated into the ApaI site of the pPK14c vector constructed in 1. of Example 10 by an infusion reaction, thereby obtaining pPK14c(A)_WT-EPO-PA22, pPK14c(A)_N-AzF-EPO-PA22, pPK14c(A)_L1-AzF-EPO-PA22, and pPK14c(A)_L2-AzF-WPO-PA22, each of which is the wildtype or mutant EPO-PA22 expression vector. As a result of nucleotide sequencing of the inserted fragment, it was confirmed that vectors carrying wildtype or mutant EPO-PA22+AzFRS were constructed as designed.

### 2. Expression of Mutant EPO-PA22

The *C*. *glutamicum* YDK0107 strain was transformed with the AzFN3 vector described in Example 1 and pPK14c(A)_N-AzF-EPO-PA22, pPK14c(A)_L1-AzF-EPO-PA22, and pPK14c(A)_L2-AzF-EPO-PA22, which were the mutant EPO-PA22+AzFRS expression vectors constructed in 1. of Example 11, thereby obtaining three types of mutant EPO-PA22-expressing strains, into which any one of three types of mutant EPO-PA22 genes was introduced, and the AzFRS DNA cassette and the AzFN3 DNA cassette were also introduced. The *C. glutamicum* YDK0107 strain was transformed with the AzFN3 vector and pPK14c(A)_WT-EPO-PA22, which was the wildtype EPO-PA22+AzFRS expression vector, thereby obtaining a wildtype EPO-PA22-expressing strain as a control.

Each EPO-PA22-expressing strain was cultured at 30°C for 96 hours in a medium to which 0.3 mM azidophenylalanine was added. After completion of the culture, 6.5 µL of the culture supernatant obtained by centrifuging each culture broth was subjected to reduced SDS-PAGE using NuPAGE (registered trademark) 12% Bis-Tirs Gel (Thermo Fisher Scientific), and then staining was carried out with Quick-CBB (Wako).

As a result, a band was substantially not observed at a location corresponding to the full length of EPO-PA22 in the absence of azidophenylalanine in the culture supernatants of the three types of mutant EPO-PA22-expressing strain, while a band was observed at a location corresponding to the full length of EPO-PA22in the presence of azidophenylalanine, thus confirming the expression of mutant EPO-PA22 into which azidophenylalanine was introduced (lanes 4-9 in FIG. 19).

### <Example 12: Molecular Modification of Azidophenylalanine-introduced Polypeptide>

### 1. Molecular Modification of Azidophenylalanine-introduced Polypeptide

An example of site-selective molecular modification of strain-promoted alkyne azide cycloaddition (SPAAC) reaction on an azidophenylalanine-introduced polypeptide is described below.

Culture supernatants (Examples 10 and 11) containing aMD4dY-PA22 (N-AzF-aMD4dY-PA22, L1-AzF-aMD4dY-PA22, and L2-AzF-aMD4dY-PA22) to which azidophenylalanine was introduced, EPO-PA22 (N-AzF-EPO-PA22, L1-AzF-EPO-PA22, and L2-AzF-EPO-PA22) to which azidophenylalanine was introduced, aMD4dY-PA22 (WT-aMD4dY-PA22) to which azidophenylalanine was not introduced, and EPO-PA22 (WT-EPO-PA22) to which azidophenylalanine was not introduced were each dialyzed with 1 mL of PBS using Amicon Ultra-0.5 3 kDa (Merck Millipore) and then concentrated to result in a 2.5-fold concentration. The concentrated culture supernatant was mixed with 3 mM m-dPEG(registered trademark)₂₄-DBCO(Quanta Biodesign) and incubated 4°C for 72 hours.

### 2. Confirmation of Azidophenylalanine Introduction and Molecular Modification Using MALDI-TOF-MS

The introduction of azidophenylalanine into aMD4dY-PA22 and EPO-PA22 and the modification with m-dPEG(registered trademark)₂₄-DBCO were confirmed by analysis of matrix-assisted laser desorption ionization-time of flight mass spectrometry (MALDI-TOF-MS).

Culture supernatants containing WT-aMD4dY-PA22, N-AzF-aMD4dY-PA22, L1-AzF-aMD4dY-PA22, L2-AzF-aMD4dY-PA22, WT-EPO-PA22, N-AzF-EPO-PA22, L1-AzF-EPO-PA22, and L2-AzF-EPO-PA22 and samples subjected to the aforementioned m-dPEG(registered trademark)₂₄-DBCO modification reaction were each purified with ZipTip C18 (Merck Millipore) and then mixed with α-Cyano-4-hydroxycinnamic Acid (CHCA), followed by molecular weight measurement using MALDI-TOF-MS (Shimadzu, AXIMA-TOF²).

The results of aMD4dY-PA22 are shown in FIG. 20. The molecular weight of WT-aMD4dY-PA22 did not change even after the m-dPEG(registered trademark)₂₄-DBCO modification reaction. That is, it revealed that WT-aMD4dY-PA22 is not modified by the SPAAC reaction. N-AzF-aMD4dY-PA22 and L1-AzF-aMD4dY-PA22 had an increase in molecular weight of about 162 compared to WT-aMD4dY-PA22, which is consistent with the increase in molecular weight when 4-amino-L-phenylalanine was inserted in WT-aMD4dY-PA22. It is known by MALDI-TOF-MS analysis that azidophenylalanine is converted to 4-amino-L-phenylalanine. Thus, the results described above indicate that azidophenylalanine has been inserted in N-AzF-aMD4dY-PA22 and L1-AzF-aMD4dY-PA22. L2-AzF-aMD4dY-PA22 had an increase in molecular weight of about 65 compared to WT-aMD4dY-PA22, which is consistent with the increase in molecular weight when L-proline of WT-aMD4dY-PA22 was converted to 4-amino-L-phenylalanine. The results indicate that L-proline has been converted to azidophenylalanine in N-AzF-aMD4dY-PA22 and L1-AzF-aMD4dY-PA22. In addition, N-AzF-aMD4dY-PA22, L1-AzF-aMD4dY-PA22, and L2-AzF-aMD4dY-PA22 had an increase in molecular weight of about 1400 after the dPEG(registered trademark)24-DBCO modification reaction. This value is substantially identical to a difference between the molecular weight of 4-amino-L-phenylalanine and the molecular weight of azidophenylalanine to which m-dPEG(registered trademark)₂₄-DBCO was bound. These results confirmed that azidophenylalanine was introduced into N-AzF-aMD4dY-PA22, L1-AzF-aMD4dY-PA22, and L2-AzF-aMD4dY-PA22, indicating that the azidophenylalanine was modified with dPEG(registered trademark)₂₄-DBCO.

The results of EPO-PA22 are shown in FIG. 21. The molecular weight of WT-EPO-PA22 did not change even after the m-dPEG(registered trademark)₂₄-DBCO modification reaction. That is, it revealed that WT-EPO-PA22 is not modified by the SPAAC reaction. N-AzF-EPO-PA22 and L1-AzF-EPO-PA22 had an increase in molecular weight of about 162 compared to WT-EPO-PA22, which is consistent with the increase in molecular weight when 4-amino-L-phenylalanine was inserted in WT-EPO-PA22. The results indicate that azidophenylalanine has been inserted in N-AzF-EPO-PA22 and L1-AzF-EPO-PA22. L2-AzF-EPO-PA22 had an increase in molecular weight of about 65 compared to WT-EPO-PA22, which is consistent with the increase in molecular weight when L-proline of WT-EPO-PA22 was converted to 4-amino-L-phenylalanine. The results indicate that L-proline has been converted to azidophenylalanine in L2-AzF-EPO-PA22. In addition, N-AzF-EPO-PA22, L1-AzF-EPO-PA22, and L2-AzF-EPO-PA22had an increase in molecular weight of about 1400 after the dPEG(registered trademark)₂₄-DBCO modification reaction. This value is substantially identical to a difference between the molecular weight of 4-amino-L-phenylalanine and the molecular weight of azidophenylalanine to which m-dPEG(registered trademark)₂₄-DBCO was bound. These results confirmed that azidophenylalanine was introduced into N-AzF-EPO-PA22, L1-AzF-EPO-PA22, and L2-AzF-EPO-PA22, indicating that the azidophenylalanine was modified with dPEG(registered trademark)₂₄-DBCO.

### 3. Confirmation of Cell Activity of PEG-modified Azidophenylalanine-introduced aMD4dY-PA22 Using Reporter Assay

When a polypeptide is introduced with an unnatural amino acid or subjected to molecular modification, the activity of the original polypeptide may be lost. It has been reported that aMD4dY-PA22 induces Met-Erk-SRE signals, and EPO-PA22 induces EPOR-JAK signals (WO2021/112249; Communications Biology, 5, 56 (2022)). Therefore, it was confirmed whether the activity of a polypeptide was maintained after the noncanonical amino acid introduction and molecular modification by evaluating cell activity for aMD4dY-PA22 and EPO-PA22 into which azidophenylalanine was introduced as described above and aMD4dY-PA22 and EPO-PA22 in which the introduced azidophenylalanine was modified with m-dPEG(registered trademark)₂₄-DBCO.

The activity of aMD4dY-PA22 was evaluated by the following reporter assay. To 25 µL of an Opti-MEM medium (Thermo Fisher Scientific), 0.6 µL of Attractene Transfection Reagent (QIAGEN) was added, followed by incubation at room temperature for 5 minutes. To this mixed solution, a mixed solution of 25 µL of Opti-MEM and 1 µL of SRE reporter vector (QIAGEN) was added in a total amount, followed by incubation at room temperature for 5 minutes. The resulting mixed solution was added to a 96-well plate for cell culture. HEK293 cells were seeded on the plate at a density of 40,000 cells/well and cultured overnight at 37°C and 5% CO₂, thereby carrying out transfection. After transfection, the culture supernatant was removed entirely, and 100 µL of an evaluation medium (Opti-MEM medium containing 0.5% FBS (Thermo Fisher Scientific), a 1% non-essential amino acid solution (Thermo Fisher Scientific), and Penicillin-Streptomycin (NACALAI TESQUE, INC.)) was added. The cells were cultured at 37°C for 4 hours so as to be starved. A sample in which WT-aMD4dY-PA22 and azidophenylalanine-introduced aMD4dY-PA22 (N-AzF-aMD4dY-PA22, L1-AzF-aMD4dY-PA22, and L2-AzF-aMD4dY-PA22) were PEG-modified using m-dPEG(registered trademark)₂₄-DBCO (PEG+) and a sample in which such modification was not made (PEG-) were each 2500-fold diluted and added to the evaluation medium. Then, 100 µL of each mixed solution was added to the cells. The cells were cultured overnight at 37°C in a 5% CO₂ incubator so as to be stimulated. Dual-Luciferase Reporter Assay System (Promega) was used for signal intensity detection. The culture supernatant in an amount of 100 µL was removed, and 50 µL of a Glo reagent was added. The cells were lysed at room temperature for 10 minutes. Activation of the Erk-SRE pathway was quantified by detecting the luminescence of firefly luciferase emitted from the cell lysate using a plate reader. Subsequently, 50 µL of a Glo & Stop reagent solution was added. After 10 minutes, the luminescence of Renilla luciferase, an internal standard, was detected, and the number of cells was quantified. For each well, signal activity value = (luminescence intensity of firefly luciferase)/(luminescence intensity of renilla luciferase) was calculated. For each sample-added group, the relative signal activity value with respect to the sample-free group (Mock) was determined and defined as reporter activity.

The activity evaluation results are shown in FIG. 22. Reporter activity was observed in all sample-added groups to be more than 10 times that of the sample-free group (Mock). That is, it was revealed that the introduction of a noncanonical amino acid and modification of the dPEG(registered trademark)₂₄-DBCO molecule did not result in the loss of the activity possessed by the original aMD4dY-PA22 molecule.

The activity of EPO-PA22 was evaluated by the following reporter assay using PathHunter(registered trademark)eXpress EpoR-JAK2 Functional Assay Kit (DiscoverX). Cells for EPO activity evaluation included in a PathHunter(registered trademark)eXpress EpoR-JAK2 Functional Assay Kit were seeded on a 384-well plate and cultured at 37°C and 5% CO₂ for 24 hours. A sample in which WT-EPO-PA22 and azidophenylalanine-introduced EPO-PA22(N-AzF-EPO-PA22, L1-AzF-EPO-PA22, and L2-AzF-EPO-PA22) were PEG-modified using m-dPEG(registered trademark)₂₄-DBCO (PEG+) and a sample in which such modification was not made (PEG-) were each 1000-fold diluted and added to the cells. A substrate reagent prepared in advance was added to the cells stimulated at room temperature for 3 hours, followed by incubation for 60 minutes. Chemiluminescence intensity was quantified using a Nivo plate reader (Perkin Elmer). For each sample-added group, the relative chemiluminescence intensity with respect to the sample-free group (Mock) was determined and defined as reporter activity.

The activity evaluation results are shown in FIG. 23. Reporter activity was observed in all sample-added groups to be more than 1.5 times that of the sample-free group (Mock). That is, it was revealed that the introduction of a noncanonical amino acid and modification of the dPEG(registered trademark)₂₄-DBCO molecule did not result in the loss of the activity possessed by EPO-PA22.

### Industrial Applicability

According to the present invention, noncanonical amino acid (ncAA)-containing proteins can be efficiently produced by secretory production.

### <Explanation of Sequence Listing>

1: Nucleotide sequence of mutant phoS gene of *C*. *glutamicum* YDK010
2: Amino acid sequence of mutant PhoS protein of *C*. *glutamicum* YDK010
3: Amino acid sequence of PhoS protein of *C*. *glutamicum* ATCC 13032
4: Amino acid sequence of PhoS protein of *C*. *glutamicum* ATCC 14067
5: Amino acid sequence of PhoS protein of *C*. *callunae*
6: Amino acid sequence of PhoS protein of *C*. *crenatum*
7: Amino acid sequence of PhoS protein of *C*. *efficiens*
8: Nucleotide sequence of phoR gene of *C*. *glutamicum* ATCC 13032
9: Amino acid sequence of PhoR protein of *C*. *glutamicum* ATCC 13032
10: Nucleotide sequence of cspB gene of *C*. *glutamicum* ATCC 13869
11: Amino acid sequence of CspB protein of *C*. *glutamicum* ATCC 13869
12: Nucleotide sequence of tatA gene of *C*. *glutamicum* ATCC 13032
13: Amino acid sequence of TatA protein of *C*. *glutamicum* ATCC 13032
14: Nucleotide sequence of tatB gene of *C*. *glutamicum* ATCC 13032
15: Amino acid sequence of TatB protein of *C*. *glutamicum* ATCC 13032
16: Nucleotide sequence of tatC gene of *C*. *glutamicum* ATCC 13032
17: Amino acid sequence of TatC protein of *C*. *glutamicum* ATCC 13032
18: Amino acid sequence of TorA signal peptide
19: Amino acid sequence of Sufl signal peptide
20: Amino acid sequence of PhoD signal peptide
21: Amino acid sequence of LipA signal peptide
22: Amino acid sequence of IMD signal peptide
23: Amino acid sequence of twin-arginine motif
24: Skipped sequence
25: Amino acid sequence of PS1 signal peptide
26: Amino acid sequence of PS2 signal peptide
27: Amino acid sequence of SlpA signal peptide
28: Amino acid sequence of CspB mature protein of *C*. *glutamicum* ATCC 13869
29 to 31: Skipped sequence
32 to 36: Amino acid sequence in one aspect of insertion sequence used herein
37: Recognition sequence of factor Xa protease
38: Recognition sequence of ProTEV protease
39: Nucleotide sequence of tRNA(Tyr) gene of *Methanocaldococcusjannaschii*
40: Nucleotide sequence of tRNA(Tyr) of *Methanocaldococcusjannaschii*
41: Nucleotide sequence of modified tRNA(Tyr) gene of *Methanocaldococcus jannaschii*
42: Nucleotide sequence of modified tRNA(Tyr) of *Methanocaldococcusjannaschii*
43: Nucleotide sequence of modified tRNA(Tyr) gene of *Methanocaldococcus jannaschii*
44: Nucleotide sequence of modified tRNA(Tyr) of *Methanocaldococcusjannaschii*
45: Nucleotide sequence of tRNA(Pyl) gene of *Methanosarcina barkeri*
46: Nucleotide sequence of tRNA(Pyl) of *Methanosarcina barkeri*
47: Nucleotide sequence of Tyr-RS gene of *Methanocaldococcusjannaschii*
48: Amino acid sequence of Tyr-RS of *Methanocaldococcusjannaschii*
49: Nucleotide sequence of modified Tyr-RS gene of *Methanocaldococcus jannaschii*
50: Amino acid sequence of modified Tyr-RS of *Methanocaldococcus jannaschii*
51: Nucleotide sequence of modified Tyr-RS gene of *Methanocaldococcus jannaschii*
52: Amino acid sequence of modified Tyr-RS of *Methanocaldococcus jannaschii*
53: Nucleotide sequence of Pyl-RS gene of *Methanosarcina barkeri*
54: Amino acid sequence of Pyl-RS of *Methanosarcina barkeri*
55: Nucleotide sequence of AzFN3 DNA cassette
56: Nucleotide sequence of wildtype 9g8 DNA cassette
57: Nucleotide sequence of wildtype 9g8 gene
58: Amino acid sequence of wildtype 9g8
59 to 80: Primers
81: Nucleotide sequence of AzFRS DNA cassette
82: Nucleotide sequence of wildtype ZHER2 affibody DNA cassette
83: Nucleotide sequence of wildtype ZHER2 affibody gene
84: Amino acid sequence of wildtype ZHER2 affibody
85 to 92: Primers
93: Nucleotide sequence of wildtype mRFP DNA cassette
94: Nucleotide sequence of wildtype mRFP gene
95: Amino acid sequence of wildtype mRFP
96 to 99: Primers
100: Nucleotide sequence of tRNACTA DNA cassette
101: Nucleotide sequence of IYN3 DNA cassette
102: Nucleotide sequence of wildtype N15 DNA cassette
103: Nucleotide sequence of wildtype N15 gene
104: Amino acid sequence of wildtype N15
105 to 112: Primers
113: Nucleotide sequence of PylRS DNA cassette
114: Nucleotide sequence of Pyl-RS gene of *Methanosarcina mazei*
115: Amino acid sequence of Pyl-RS of *Methanosarcina mazei*
116: Nucleotide sequence of tRNA(Pyl)CTA DNA cassette
117: Nucleotide sequence of tRNA(Pyl)TCA DNA cassette
118: Nucleotide sequence of modified tRNA(Pyl) gene of *Methanosarcina mazei*
119: Nucleotide sequence of modified tRNA(Pyl) of *Methanosarcina mazei*
120: Nucleotide sequence of modified tRNA(Pyl) gene of *Methanosarcina mazei*
121: Nucleotide sequence of modified tRNA(Pyl) of *Methanosarcina mazei*
122: Nucleotide sequence of wildtype 9g8 DNA cassette for *E. coli*
123: Nucleotide sequence of AzFRS DNA cassette
124: Nucleotide sequence of AzFRS DNA cassette
125: Nucleotide sequence of wildtype aMD4dY-PA22 DNA cassette
126: Nucleotide sequence of WT-aMD4dY-PA22 gene
127: Amino acid sequence of WT-aMD4dY-PA22
128: Nucleotide sequence of N-AzF-aMD4dY-PA22 gene
129: Amino acid sequence of N-AzF-aMD4dY-PA22
130: Nucleotide sequence of L1-AzF-aMD4dY-PA22 gene
131: Amino acid sequence of L1-AzF-aMD4dY-PA22
132: Nucleotide sequence of L2-AzF-aMD4dY-PA22 gene
133: Amino acid sequence of L2-AzF-aMD4dY-PA22
134: Nucleotide sequence of wildtype EPO-PA22 DNA cassette
135: Nucleotide sequence of WT-EPO-PA22 gene
136: Amino acid sequence of WT-EPO-PA22
137: Nucleotide sequence of N-AzF-EPO-PA22 gene
138: Amino acid sequence of N-AzF-EPO-PA22
139: Nucleotide sequence of L1-AzF-EPO-PA22 gene
140: Amino acid sequence of L1-AzF-EPO-PA22
141: Nucleotide sequence of L2-AzF-EPO-PA22 gene
142: Amino acid sequence of L2-AzF-EPO-PA22

## Claims

1. A method for producing a protein containing a noncanonical amino acid, comprising:
culturing a coryneform bacterium having a genetic construct for secretory expression of the protein containing a noncanonical amino acid in a medium containing the noncanonical amino acid; and
collecting the protein containing a noncanonical amino acid produced by secretory production,
wherein the coryneform bacterium is modified to express an orthogonal pair of a tRNA corresponding to the noncanonical amino acid and an aminoacyl tRNA synthetase.

2. The method according to claim 1, wherein
the genetic construct comprises, in the direction from 5' to 3', a promoter sequence that functions in a coryneform bacterium, a nucleic acid sequence encoding a signal peptide that functions in a coryneform bacterium, and a nucleic acid sequence encoding the protein containing a noncanonical amino acid, and
the protein containing a noncanonical amino acid is expressed as a fusion protein with the signal peptide.

3. The method according to claim 1 or 2, wherein the noncanonical amino acid is encoded by a stop codon or a four-residue codon.

4. The method according to claim 3, wherein the stop codon is UAG or UGA.

5. The method according to any one of claims 1 to 4, wherein the noncanonical amino acid is a tyrosine derivative or a lysine derivative.

6. The method according to any one of claims 1 to 5, wherein the noncanonical amino acid is selected from the group consisting of p-azido-L-phenylalanine, 3-azido-L-tyrosine, 3-chloro-L-tyrosine, 3-nitro-L-tyrosine, O-sulfo-L-tyrosine, L-pyrrolidine, and N_{δ}-alloc-L-lysine.

7. The method according to any one of claims 1 to 6, wherein the tRNA is tRNA(Tyr) or tRNA(Pyl).

8. The method according to claim 7, wherein the tRNA(Tyr) is an RNA in (a), (b), or (c) described below:
(a) RNA comprising the nucleotide sequence of SEQ ID NO: 42 or 44;
(b) RNA comprising a nucleotide sequence with a modified anticodon in the nucleotide sequence of SEQ ID NO: 40, 42, or 44; or
(c) RNA comprising a nucleotide sequence having an identity of 90% or higher to the nucleotide sequence of the RNA described in (a) or (b), wherein the RNA has a function as a tRNA corresponding to the noncanonical amino acid.

9. The method according to claim 7, wherein the tRNA(Pyl) is an RNA in (a), (b), or (c) described below:
(a) RNA comprising the nucleotide sequence of SEQ ID NO: 46, 119, or 121;
(b) RNA comprising a nucleotide sequence with a modified anticodon in the nucleotide sequence of SEQ ID NO: 46, 119, or 121; or
(c) RNA comprising a nucleotide sequence having an identity of 90% or higher to the nucleotide sequence of the RNA in (a) or (b), wherein the RNA has a function as a tRNA corresponding to the noncanonical amino acid.

10. The method according to any one of claims 1 to 9, wherein the aminoacyl tRNA synthetase is tyrosyl tRNA synthetase or pyrrolidyl tRNA synthetase.

11. The method according to claim 10, wherein the tyrosyl tRNA synthetase is a protein in (a), (b), (c), or (d) described below:
(a) a protein comprising the amino acid sequence of SEQ ID NO: 50 or 52;
(b) a protein comprising an amino acid sequence having a mutation that modifies the substrate specificity of the amino acid sequence of SEQ ID NO: 48, 50, or 52, wherein the protein has an aminoacyl tRNA synthetase activity for the noncanonical amino acid;
(c) a protein comprising the amino acid sequence described in (a) or (b), but which includes substitution, deletion, insertion, and/or addition of 1 to 10 amino acid residues; or
(d) a protein comprising an amino acid sequence having an identity of 90% or higher to the amino acid sequence of the protein described in (a) or (b).

12. The method according to claim 11, wherein the mutation that modifies the substrate specificity includes a mutation in one or more amino acid residues selected from the group consisting of: Y32, H70, E107, D158, I159, L162, and D286.

13. The method according to claim 10, wherein the pyrrolidyl tRNA synthetase is a protein in (a), (b), (c), or (d) described below:
(a) a protein comprising the amino acid sequence of SEQ ID NO: 54 or 115;
(b) a protein comprising an amino acid sequence having a mutation that modifies the substrate specificity of the amino acid sequence of SEQ ID NO: 54 or 115, wherein the protein has an aminoacyl tRNA synthetase activity for the noncanonical amino acid;
(c) a protein comprising the amino acid sequence described in (a) or (b), but which includes substitution, deletion, insertion, and/or addition of 1 to 10 amino acid residues, wherein the protein has an aminoacyl tRNA synthetase activity for the noncanonical amino acid; or
(d) a protein comprising an amino acid sequence having an identity of 90% or higher to the amino acid sequence of the protein described in (a) or (b), wherein the protein has an aminoacyl tRNA synthetase activity for the noncanonical amino acid.

14. The method according to claim 13, wherein the mutation that modifies the substrate specificity includes a mutation in one or more amino acid residues selected from the group consisting of: M241, L266, A267, L270, Y271, L274, N311, C313, M315, Y349, V367, and W383.

15. The method according to any one of claims 1 to 14, wherein the coryneform bacterium has been further modified so as to harbor a phoS gene encoding a mutant PhoS protein.

16. The method according to claim 15, wherein the mutation is replacing an amino acid residue corresponding to the tryptophan residue at position 302 in SEQ ID NO: 2 with an amino acid residue other than an aromatic amino acid and a histidine residue in a wildtype PhoS protein.

17. The method according to claim 16, wherein the amino acid residue other than aromatic an amino acid and a histidine residue is selected from the group consisting of a lysine residue, alanine residue, valine residue, serine residue, cysteine residue, methionine residue, aspartic acid residue, and asparagine residue.

18. The method according to claim 16 or 17, wherein the wildtype PhoS protein is a protein in (a), (b), or (c) described below:
(a) a protein comprising any of the amino acid sequences of SEQ ID NOS: 2 to 7;
(b) a protein comprising any of the amino acid sequences of SEQ ID NOS: 2 to 7, but which includes substitution, deletion, insertion, and/or addition of 1 to 10 amino acid residues, wherein the protein has a function as a sensor kinase of a PhoRS system; or
(c) a protein comprising an amino acid sequence having an identity of 90% or higher to any of the amino acid sequences of SEQ ID NOS: 2 to 7, wherein the protein has a function as a sensor kinase of a PhoRS system.

19. The method according to any one of claims 2 to 18, wherein the signal peptide is a Tat-dependent signal peptide.

20. The method according to claim 19, wherein the Tat-dependent signal peptide is selected from the group consisting of TorA signal peptide, Sufl signal peptide, PhoD signal peptide, LipA signal peptide, and IMD signal peptide.

21. The method according to claim 19 or 20, wherein the coryneform bacterium has been further modified so that the expression of one or more genes encoding a Tat secretion system is increased as compared with a non-modified strain.

22. The method according to claim 21, wherein the genes encoding a Tat secretion system consist of a tatA gene, tatB gene, tatC gene, and tatE gene.

23. The method according to any one of claims 2 to 18, wherein the signal peptide is a Sec-dependent signal peptide.

24. The method according to claim 23, wherein the Sec-dependent signal peptide is selected from the group consisting of PS1 signal peptide, PS2 signal peptide, and SlpA signal peptide.

25. The method according to any one of claims 2 to 24, wherein the genetic construct further comprises a nucleic acid sequence encoding an amino acid sequence including Gln-Glu-Thr between the nucleic acid sequence encoding a signal peptide that functions in a coryneform bacterium and the nucleic acid sequence encoding the protein containing a noncanonical amino acid.

26. The method according to claim 25, wherein the genetic construct further comprises a nucleic acid sequence encoding an amino acid sequence used for enzymatic cleavage between the nucleic acid sequence encoding an amino acid sequence including Gln-Glu-Thr and the nucleic acid sequence encoding the protein containing a noncanonical amino acid.

27. The method according to any one of claims 1 to 26, wherein the coryneform bacterium belongs to the genus *Corynebacterium.*

28. The method according to claim 27, wherein the coryneform bacterium is *Corynebacterium glutamicum.*

29. The method according to claim 28, wherein the coryneform bacterium is derived from *Corynebacterium glutamicum* AJ12036 (FERM BP-734) or *Corynebacterium glutamicum* ATCC 13869.

30. The method according to any one of claims 1 to 29, wherein the coryneform bacterium is a coryneform bacterium with the number of molecules of a cell surface layer protein per cell reduced as compared with a non-modified coryneform bacterium.

31. The method according to any one of claims 1 to 30, wherein the coryneform bacterium has a first expression vector carrying the genetic construct and a second expression vector carrying a gene encoding the tRNA and a gene encoding the aminoacyl tRNA synthetase.

32. The method according to claim 31, wherein the first expression vector further carries a gene encoding the tRNA and/or a gene encoding the aminoacyl tRNA synthetase.

33. The method according to claim 31 or 32, wherein the first expression vector is a pPK vector and the second expression vector is a pVC vector.

34. The method according to any one of claims 31 to 33, wherein the first expression vector is pPK4 or pPK5 and the second expression vector is pVC7 or pVC7N.

35. The method according to any one of claims 1 to 30, wherein the coryneform bacterium has a single expression vector carrying the genetic construct, a gene encoding the tRNA, and a gene encoding the aminoacyl tRNA synthetase.

36. The method according to claim 35, wherein the expression vector is a pPK vector.

37. The method according to claim 35 or 36, wherein the expression vector is pPK4 or pPK5.

38. The method according to any one of claims 1 to 37, wherein the noncanonical amino acid-containing protein is an antibody-related molecule, an antibody mimetic, or a physiologically active protein.

39. The method according to any one of claims 1 to 38, wherein the noncanonical amino acid-containing protein is a VHH fragment, a Z domain of protein A, a fluorescent protein, or a growth factor.
